(19) Europäisches Patentamt
European Patent Office
Office européen des brevets

(11) Veröffentlichungsnummer: **0 254 245 B1**

# (12) EUROPÄISCHE PATENTSCHRIFT

(45) Veröffentlichungstag der Patentschrift: **28.09.94**

(21) Anmeldenummer: **87110443.6**

(22) Anmeldetag: **18.07.87**

Die Akte enthält technische Angaben, die nach dem Eingang der Anmeldung eingereicht wurden und die nicht in dieser Patentschrift enthalten sind.

(51) Int. Cl.5: **C07D 495/14**, C07D 495/22, C07D 333/72, C07D 333/78, C07D 333/80, C07D 495/04, A61K 31/55, //(C07D495/14, 333:00,249:00,243:00), (C07D495/14,333:00,243:00, 235:00)

## (54) Neue Hetrazepine und Verfahren zu ihrer Herstellung.

(30) Priorität: **22.07.86 DE 3624647**

(43) Veröffentlichungstag der Anmeldung:
**27.01.88 Patentblatt 88/04**

(45) Bekanntmachung des Hinweises auf die Patenterteilung:
**28.09.94 Patentblatt 94/39**

(84) Benannte Vertragsstaaten:
**AT BE CH DE ES FR GB GR IT LI LU NL SE**

(56) Entgegenhaltungen:
**EP-A- 230 942**
**EP-A- 240 899**
**EP-A- 268 242**
**EP-A- 312 913**
**EP-A- 315 698**
**EP-A- 316 456**
**EP-A- 320 992**
**EP-A- 328 924**
**EP-A- 338 993**
**EP-A- 342 587**
**EP-A- 345 931**
**EP-A- 367 110**
**EP-A- 0 176 927**
**DE-A- 2 233 457**
**DE-A- 4 010 316**

(73) Patentinhaber: **BOEHRINGER INGELHEIM KG**
**Postfach 200**
**D-55216 Ingelheim (DE)**
(84) Benannte Vertragsstaaten:
**BE CH DE ES FR GR IT LI LU NL SE AT**

(73) Patentinhaber: **BOEHRINGER INGELHEIM INTERNATIONAL GmbH**
**Postfach 200**
**D-55216 Ingelheim (DE)**
(84) Benannte Vertragsstaaten:
**GB**

(72) Erfinder: **Weber, Karl-Heinz, Dr., Dipl.-Chem.**
**Kaiser-Karl-Strasse 11**
**D-6535 Gau-Algesheim (DE)**

EP 0 254 245 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**MEDICINAL RESEARCH REVIEWS (1989) 9, 181-218**

**INT. ARCH. ALLERGY APPL. IMMUNOL. (1989) 88, 82-87**

**CHEMICAL ABSTRACTS, Band 88, Nr. 3, 16. Januar 1978, Seite 632, Zusammenfassungsnr. 22853z, Columbus, Ohio, US; A.S. NORAVYAN et al.: "Synthesis and anticonvulsive activity of derivatives of 1,4-diazepine condensed systems" & KHIM.-FARM.ZH 1977, 11(10), 62-64**

**CHEMICAL ABSTRACTS, Band 88, Nr. 7, 13. Februar 1978, Seite 556, Zusammenfassungsnr. 50934v, Columbus, Ohio, US; & JP-A-77 100 496**

**CHEMICAL ABSTRACTS, Band 83, Nr. 23, 8. Dezember 1975, Seite 464, Zusammenfassungsnr. 193276g, Columbus, Ohio, US; & JP-A-7511397**

**CHEMICAL ABSTRACTS, Band 79, Nr. 11, 11. September 1973, Seite 449, Zusammenfassungsnr. 66340y, Columbus, Ohio, US; & JP-A-7315957**

**CHEMICAL ABSTRACTS, Band 88, Nr. 1, 2. Januar 1978, Seite 576, Zusammenfassungsnr. 6776w, Columbus, Ohio, US; A.S. NORAVYAN et al.: "Synthesis and anticonvulsant activity of 2,3-substituted 5,5- dimethyltetrahydropyrano- and 5,5-dimethyltetrahydrothiopyrano (3,4-b) thiophenes" & KHIM. FARM. ZH 1977, 11(8), 20-24**

Erfinder: **Harreus, Albrecht, Dr., Dipl.-Chem.**
**Sandstrasse 1**
**D-6507 Ingelheim am Rhein (DE)**
Erfinder: **Stransky, Werner, Dr., Dipl.-Chem.**
**Im Hippel 24**
**D-6535 Gau-Algesheim (DE)**
Erfinder: **Walther, Gerhard, Dr., Dipl.-Chem.**
**Pfarrer-Heberer-Strasse 37**
**D-6530 Bingen (DE)**
Erfinder: **Casals-Stenzel, Jorge, Dr.**
**Hirsch-Gereuth-Strasse 76**
**D-8000 München 70 (DE)**
Erfinder: **Muacevic, Gojko, Dr.**
**In der Dörrwiese 13**
**D-6507 Ingelheim am Rhein (DE)**
Erfinder: **Heuer, Hubert, Dr.**
**Im Münchfeld 23**
**D-6500 Mainz 23 (DE)**
Erfinder: **Bechtel, Wolf-Dietrich, Dr.**
**Mühlstrasse 3**
**D-6531 Appenheim (DE)**

## Beschreibung

Die Erfindung betrifft neue Hetrazepine, ihre Herstellung nach bekannten Methoden und ihre Verwendung als Zwischenprodukte.

Die neuen Hetrazepine entsprechen der allgemeinen Formel

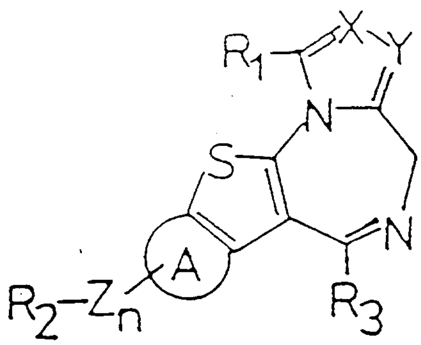

Ia

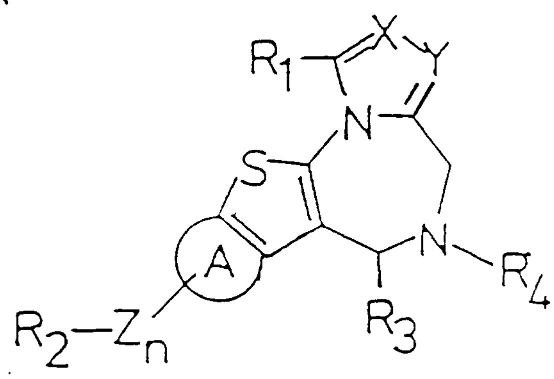

Ib

worin

A ein ankondensierter einfach ungesättigter 5-, 6- oder 7- gliedriger Ring, wobei im Fall von n = 0 und $R_2$ = Wasserstoff ein Kohlenstoffatom durch C=0 ersetzt werden kann, oder A einen ankondensierten Ring der Formel

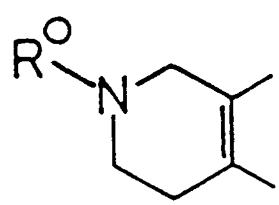

bedeutet, wobei $R^o$ eine verzweigte oder unverzweigte Alkylgruppe mit 1 bis 18, bevorzugt mit 1 bis 4, Kohlenstoffatomen, eine Alkylcarbonyl- oder Alkylthiocarbonylgruppe mit 1 bis 18 bevorzugt 1 bis 4, Kohlenstoffatomen in der Alkylkette oder eine gegebenenfalls substituierte Phenylcarbonyl- oder Phenylthiocarbonylgruppe eine Alkoxycarbonylalkylgruppe mit bis zu 18, bevorzugt bis zu 8 Kohlenstoffatomen, eine Aminocarbonylgruppe, Alkylcarbonylalkylgruppe mit bis zu 18, bevorzugt bis zu 8 Kohlenstoffatomen, eine Alkylcarbonylaminoalkylcarbonylgruppe mit bis zu 18, bevorzugt bis 10 Kohlenstoffatomen oder eine Aminocarbonylalkylgruppe mit bis zu 18, bevorzugt bis 4 Kohlenstoffatomen in der Alkylkette oder Wasserstoff, mit der Maßgabe, daß wenn $R^o$ Wasserstoff $R_1$ nicht gleichzeitig Methyl, $R_3$ nicht o-Chlorphenyl und X und Y nicht beide Stickstoff bedeuten;

Z eine verzweigte oder unverzweigte Alkylen- oder Alkenylengruppe mit n Kohlenstoffatomen;

n 0, 1, 2, 3, 4, 5 oder 6; im Fall einer Alkenylengruppe 0, 2, 3, 4, 5 oder 6;

X/Y unabhängig voneinander C-$R_1$, oder N, aber nicht beide C-$R_1$,

oder Y die Gruppe C-COOR', wobei R' Alkyl oder Wasserstoff bedeutet und X Stickstoff bedeutet;

$R_1$ Wasserstoff, eine verzweigte oder unverzweigte Alkylgruppe mit 1 bis 4 Kohlenstoffatomen, bevorzugt Methyl, die gegebenenfalls durch Hydroxy oder Halogen substituiert sein kann, eine Cyclopropyl- oder Cyclopentylgruppe, eine verzweigte oder unverzweigte Alkoxygruppe mit 1 bis 4 Kohlenstoffatomen, bevorzugt Methoxy, Halogen, bevorzugt Chlor oder Brom;

$R_2$ Hydroxy, Formyl, Carboxy, Cyano, verzweigtes oder unverzweigtes Alkyloxycarbonyl mit 1 bis 18, bevorzugt 8, Kohlenstoffatomen, wobei die Alkylkette gegebenenfalls durch Hydroxy, Amino, Nitro oder Halogen substituiert sein kann, eine gegebenenfalls substituierte Phenyloxycarbonylgruppe;

einen Rest der allgemeinen Formel

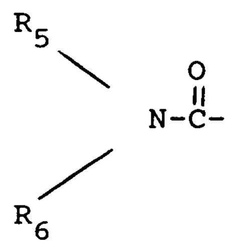

worin $R_5$ und $R_6$, die gleich oder verschieden sein können, Wasserstoff, Phenyl, substituiertes Phenyl, eine verzweigte oder unverzweigte Alkyl-, Alkenyl- oder Alkinylgruppe mit bis zu 18 Kohlenstoffatomen, die gegebenenfalls durch Halogen, Hydroxy, Nitro, Amino,

Alkoxy, bevorzugt Methoxy, oder im Fall von $R_6$ = Wasserstoff oder Alkyl, oder durch ein Säureamid der allgemeinen Formel

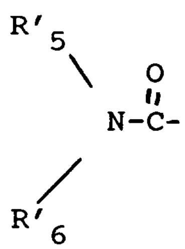

worin $R'_5$ und $R'_6$, dieselbe Bedeutung wie $R_5$ und $R_6$, jedoch mit Ausnahme eines Säureamids haben können, substituiert sein kann; $R_5$ oder $R_6$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoff verknüpfter 5- oder 6-gliedriger heterocyclischer Ring;

oder

$R_5$ und $R_6$ zusammen mit dem Stickstoffatom einen gesättigten oder ungesättigten gegebenenfalls ein- oder mehrfach durch verzweigte oder unverzweigte Alkylgruppen mit 1 bis 4 Kohlenstoffatomen substituierten 5- oder 6-Ring, der als weitere Heteroatome Stickstoff, Sauerstoff oder Schwefel enthalten kann, wobei jedes weitere Stickstoffatom durch eine verzweigte oder unverzweigte Alkylgruppe mit 1 bis 4 Kohlenstoffatomen, bevorzugt Methyl, substituiert sein kann;

$R_2$ einen Rest der allgemeinen Formel

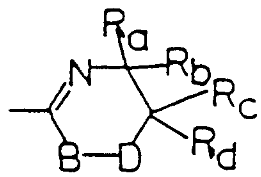

oder

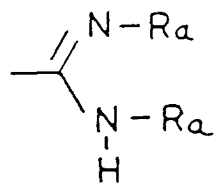

worin
B Sauerstoff, Schwefel, NH oder N-$C_1$-$C_6$-Alkyl D den Rest $(CReRf)_n$, wobei n 0 bis 3 sein kann,
Ra Wasserstoff, Alkyl mit 1 bis 6 Kohlenstoffatomen, $C_1$ bis $C_4$ Alkoxycarbonyl, Dialkylaminocarbonyl,
Rb, Rc, Rd, Re, Rf Wasserstoff, Alkyl mit 1 bis 6 Kohlenstoffatomen, oder Phenyl;

$R_2$ Wasserstoff, wenn A eine Carbonylfunktion oder Stickstoff als Ringglied enthält;

$R_2$

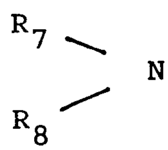

$R_7$ = Wasserstoff, $R_8$ = Wasserstoff, Alkylcarbonyl oder Alkoxycarbonyl mit 1 bis 18, bevorzugt 1 bis 6 Kohlenstoffatomen, Aminocarbonyl, Alkylaminocarbonyl, Dialkylaminocarbonyl mit 1 bis 18 bevorzugt 1 bis 6 Kohlenstoffatomen in der Alkylkette;

$R_2$ Wasserstoff (für n > O);

$R_2$ Halogen,

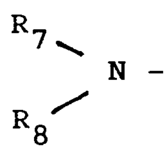

,

wobei $R_7$ und $R_8$, die gleich oder verschieden sein können, Wasserstoff, eine verzweigte oder unverzweigte Alkyl-, Alkenyl- oder Alkinylgruppe mit bis zu 18 Kohlenstoffatomen, die gegebenenfalls durch Halogen, Hydroxy oder einen C-verknüpften Heterocyclus substituiert sein kann, wobei die Kohlenstoffkette durch Stickstoff, Sauerstoff oder Schwefel unterbrochen sein kann,
eine verzweigte oder unverzweigte Alkylcarbonylgruppe mit 1-6 Kohlenstoffatomen, gegebenenfalls substituiert durch Hydroxy oder Halogen, bevorzugt Chlor, oder substituiert durch eine gegebenenfalls ein- oder zweifach durch eine verzweigte oder unverzweigte Alkylgruppe mit 1 bis 6 Kohlenstoffatomen substituierte Aminogruppe, wobei die Alkylgruppe durch Halogen oder Hydroxy substituiert sein kann,
eine gegebenenfalls substituierte Phenylcarbonylgruppe, eine gegebenenfalls substituierte Phenylsulfonylgruppe, eine Alkylsulfonylgruppe mit 1 bis 4 Kohlenstoffatomen,
oder
$R_7$ und $R_8$ bilden zusammen mit dem Stickstoffatom einen gesättigten oder ungesättigten gegebenenfalls ein- oder mehrfach durch verzweigte oder unverzweigte Alkylgruppen mit 1 bis 4 Kohlenstoffatomen substituierten 5-, oder 6-Ring, der als weitere Heteroatome Stickstoff, Sauerstoff oder Schwefel enthalten kann, wobei jedes weitere Stickstoffatom gegebenenfalls durch eine verzweigte oder unverzweigte Alkylgruppe mit 1 bis 4 Kohlenstoffatomen, bevorzugt Methyl, substituiert sein kann;

$R_2$ eine Phenylsulfonyloxygruppe, gegebenenfalls einoder mehrfach durch verzweigte oder unverzweigte Alkyl- und/oder Alkoxygruppen mit 1 bis 4 Kohlenstoffatomen substituiert;

$R_2$ eine verzweigte oder unverzweigte Alkylsulfonyloxygruppe mit 1 bis 4 Kohlenstoffatomen;

$R_2$ eine Phenylcarbonyloxygruppe, gegebenenfalls ein oder mehrfach durch verzweigte oder unverzweigte Alkyl- und/oder Alkoxygruppen mit 1 bis 4 Kohlenstoffatomen substituiert;

$R_2$ eine verzweigte oder unverzweigte Alkylcarbonyloxygruppe mit 1 bis 18, bevorzugt 1 bis 8, Kohlenstoffatomen, wobei die Alkylkette durch Stickstoff, Sauerstoff oder Schwefel unterbrochen sein kann;

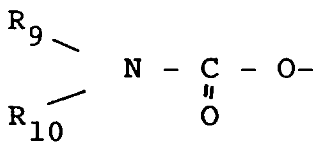 , 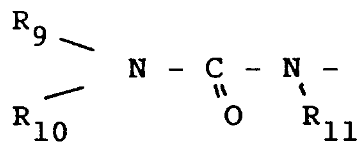

wobei $R_9$ Wasserstoff und $R_{10}$ eine Alkyl-, Alkenyl- oder Alkinylgruppe mit bis zu 4 Kohlenstoffatomen, gegebenenfalls durch Halogen substituiert, eine gegebenenfalls ein- oder mehrfach durch verzweigte oder unverzweigte Alkyl- und/oder Alkoxygruppen mit 1 bis 4 Kohlenstoffatomen substituierte Phenylgruppe,

$R_{11}$ Wasserstoff oder eine verzweigte oder unverzweigte Alkylgruppe mit 1 bis 4 Kohlenstoffatomen;

$R_2$ einen Imidorest; einen Benzimidazolylrest;

$R_2$ einen verzweigten oder unverzweigten Alkyloxy- bzw. Alkylthiorest mit 1 bis 18, bevorzugt 1 bis 4 Kohlenstoffatomen, einen gegebenenfalls substituierten Phenyloxy- oder Phenylthiorest, einen über Sauerstoff oder Schwefel verknüpften, gesättigten oder ungestättigten 5- oder 6-gliedrigen heterocyclischen Ring,

wobei als Substituenten für einen substituierten Phenylrest - sofern nicht gesondert definiert - $C_1$-$C_4$-Alkyl, $C_3$-$C_7$-Cycloalkyl,

$C_1$-$C_4$-Alkoxy, Hydroxy oder Halogen angegeben wird,

$R_3$ Phenyl, wobei der Phenylring ein oder mehrfach, bevorzugt in 2-Stellung, durch Methyl, bevorzugt Halogen, bevorzugt Brom, besonders bevorzugt Chlor, Nitro und/oder Trifluormethyl substituiert sein kann, oder Pyridyl,

und

$R_4$ Wasserstoff, verzweigtes oder unverzweigtes Alkyl mit 1 bis 4 Kohlenstoffatomen oder Alkylcarbonyl mit 1 bis 4 Kohlenstoffatomen, bevorzugt Acetyl, bedeuten können, gegebenenfalls in Form ihrer Racemate, ihrer Enantiomere, ihrer Diastereomere und ihrer Gemische sowie gegebenenfalls ihre physiologisch unbedenklichen Säureadditionssalze.

Soweit nichts anderes angegeben, bedeutet Halogen eines der Atome Fluor, Chlor, Brom oder Jod; Alkyl eine verzweigte oder unverzweigte Alkylgruppe mit 1 bis 18 Kohlenstoffatomen; niederes Alkyl eine verzweigte oder unverzweigte Alkylgruppe mit 1 bis 4 Kohlenstoffatomen wie z.B. Methyl, Ethyl, Propyl, iso-Propyl, n-Butyl, sec.- Butyl, tert.-Butyl; Cycloalkyl ein 3 bis 7-gliedriger carbocyclischer Ring, wie z.B. Cyclopropyl, Cyclopentyl oder Cyclohexyl, der gegebenenfalls durch niederes Alkyl substituiert ist; Alkylgruppen können weitere Substituenten und funktionelle Gruppen enthalten und gegebenenfalls durch Heteroatome unterbrochen sein; unter Aryl werden gegebenenfalls substituierte aromatische Reste mit bis zu 10 Kohlenstoffatomen im Ringsystem verstanden, wobei der Phenylring bevorzugt ist, wobei als Substituenten niederes Alkyl, Cycloalkyl, niederes Alkoxy, Hydroxy oder Halogen in Betracht kommen können. Eine Aminogruppe kann sowohl $-NH_2$, wie auch mono- oder disubstituierte Amine wie auch cyclische Amine bedeuten, die gegebenenfalls weitere Substituenten, wie auch weitere funktionelle Gruppen und Heteroatome enthalten können.

Bevorzugt sind Verbindungen der allgemeinen Formel Ia, worin A ein ankondensierter einfach ungesättigter 5- oder 6-gliedriger Ring, oder A einen ankondensierten Ring der Formel

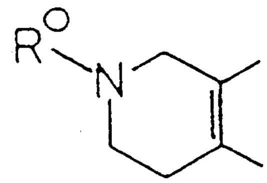

worin R° Acetylaminoacetyl, Acetyl, Thioacetyl, Ethoxycarbonylmethyl, Methoxycarbonylmethyl, Morpholinylcarbonylmethyl, Diethylaminocarbonylmethyl;

Z eine unverzweigte Alkylengruppe mit n Kohlenstoffatomen

n O, 1, 2, 3 oder 4;

X/Y beide N oder X C-$R_1$ und Y N,

oder

$R_1$ Wasserstoff, Hydroxymethyl, Chlormethyl, Cyclopropyl, Ethyl, Methoxy, Ethoxy, Chlor oder Brom, bevorzugt Methyl;

$R_2$ Hydroxy, Carboxy, Cyano, Brom, Alkyloxycarbonyl mit 1 bis 6 Kohlenstoffatomen, bevorzugt 1 bis 2 Kohlenstoffatomen,

einen Rest der allgemeinen Formel

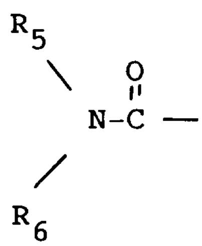

worin $R_5$ und $R_6$, die gleich oder verschieden sein können, Wasserstoff, eine verzweigte oder unverzweigte Alkylgruppe oder Alkenylgruppe mit bis zu 6, 8 oder 16 Kohlenstoffatomen, die gegebenenfalls durch Halogen, Hydroxy, Methoxy, Nitro, Amino, Alkylamino oder Dialkylamino mit 1 bis 4 Kohlenstoffatomen in der Alkylkette, oder im Fall von $R_6$ = Wasserstoff oder Alkyl durch Morpholinylcarbonyl oder Diethylaminocarbonyl substituiert sein kann,

im Fall von $R_5$ = Wasserstoff oder Methyl, $R_6$ ein Thiazolin- oder Thiazolrest, der gegebenenfalls durch eine verzweigte oder unverzweigte Alkylgruppe mit 1 bis 4 C-Atomen substituiert sein kann,

oder

$R_5$ und $R_6$ zusammen mit dem Stickstoffatomen einen Morpholino- oder Piperazinorest bilden, der gegebenenfalls ein- oder mehrfach durch Methyl substituiert sein kann;

$R_2$ ein C-verknüpfter $\Delta^2$-Imidazolin-, -Thiazolin-, -Oxazolin-, oder Tetrahydropyrimidin-Rest, der gegebenenfalls ein- oder mehrfach durch Alkyl mit 1 bis 4 Kohlenstoffatomen substituiert sein kann;

$R_2$ im Fall von n = O Wasserstoff, wenn A eine Carbonylfunktion oder Stickstoff als Ringglied enthält,

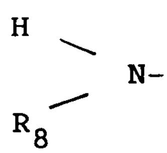

$R_8$ eine Alkyloxycarbonylgruppe mit 1 bis 4 Kohlenstoffatomen;

im Fall von n > O

$R_2$ eine Alkylcarbonyloxygruppe mit 1 bis 3 Kohlenstoffatomen;

$R_2$ eine Alkylsulfonyloxygruppe mit 1 bis 2 Kohlenstoffatomen;

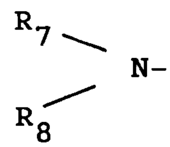

wobei $R_7$ und $R_8$, die gleich oder verschieden sein können, Wasserstoff, eine verzweigte oder unverzweigte Alkylgruppe mit 1 bis 6 Kohlenstoffatomen, gegebenenfalls durch Dialkylamino mit 1 bis 4 Kohlenstoffatomen, bevorzugt Methyl oder Ethyl, Morpholino oder N-Alkylpiperazino oder ein Indolrest substituiert, eine Alkylcarbonylgruppe mit 1 bis 4 Kohlenstoffatomen,
oder
$R_7$ und $R_8$ zusammen mit dem Stickstoffatom einen Morpholino- oder Piperazinorest bilden, der gegebenenfalls ein- oder mehrfach durch Methyl substituiert sein kann, ein Triazolorest, ein Imidazolorest, ein Pyrazolorest, Pyrrolorest, ein Imidorest,

$R_2$ eine verzweigte oder unverzweigte Alkylsulfonyloxygruppe mit 1 bis 4 Kohlenstoffatomen, bevorzugt Methylsulfonyloxy, eine verzweigte oder unverzweigte Alkylcarbonyloxygruppe mit 1 bis 8 Kohlenstoffatomen, bevorzugt 1 bis 4 Kohlenstoffatomen;

$R_2$ Phenyloxy, 3,4-Methylendioxyphenoxy einen Pyridinyloxyrest, einen Alkyloxy- oder Alkylthiorest mit 1 bis 4 Kohlenstoffatomen;

$R_3$ Phenyl oder o-Chlorphenyl

bedeuten können, sowie gegebenenfalls ihre physiologisch unbedenklichen Säureadditionssalze und ihre optisch aktiven Verbindungen.

Besonders bevorzugt sind Verbindungen der allgemeinen Formel Ia, worin A ein ankondensierter, bevorzugt in 3- oder 4-Stellung des Hetrazepins substituierter, einfach ungesättigter 5- oder 6-gliedriger Ring,

Z eine unverzweigte Alkylengruppe mit n Kohlenstoffatomen

n 0, 1 oder 2;

X/Y beide N, oder X C-H oder C-$CH_3$ und Y N,

$R_1$ Wasserstoff, Cyclopropyl, Methoxy, Brom, bevorzugt Methyl;

$R_2$ Hydroxy, Carboxy, Cyano, Methoxycarbonyl, Ethoxycarbonyl,
einen Rest der allgemeinen Formel

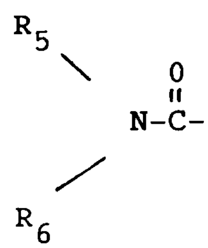

worin $R_5$ und $R_6$, die gleich oder verschieden sein können, Wasserstoff, eine verzweigte oder unverzweigte Alkylgruppe mit 1 bis 6, 8 oder 16 Kohlenstoffatomen, die gegebenenfalls durch Halogen, Hydroxy, Nitro, Amino, Ethylamino oder Diethylamino, Methoxy, oder im Fall von $R_6$ = Wasserstoff oder Alkyl durch Morpholinylcarbonyl oder Diethylaminocarbonyl substituiert sein kann,
Propenyl, Phenyl,
im Fall von $R_5$ = Wasserstoff oder Methyl,
$R_6$ ein Thiazolin- oder Thiazolrest, der gegebenenfalls durch Methyl substituiert sein kann, oder
$R_5$ und $R_6$ zusammen mit dem Stickstoffatom einen Morpholino- oder Piperazinorest bilden, der gegebenenfalls ein- oder mehrfach durch Methyl substituiert sein kann;

$R_2$ ein C-verknüpfter $\Delta^2$-Imidazolin-, -Thiazolin-Oxazolin-Rest, der gegebenenfalls ein- oder mehrfach durch Methyl, Ethyl und/oder iso-Propyl substituiert sein kann, ein Tetrahydro-Pyrimidinring, gegebenenfalls ein- oder mehrfach durch Methyl substituiert, ein Benzimidazolrest, ein Indolrest,

$R_2$ Methoxycarbonylamino;

im Fall von n > 0

$R_2$ eine Acetoxygruppe, eine Methansulfonyloxygruppe,

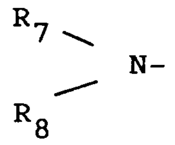

wobei $R_7$ und $R_8$, die gleich oder verschieden sein können, Wasserstoff, eine verzweigte oder unverzweigte Alkylgruppe mit 1 bis 4 Kohlenstoffatomen, die durch Diethylamino oder Morpholino, substituiert sein kann, eine Acetylgruppe oder

$R_7$ und $R_8$ zusammen mit dem Stickstoffatom einen Morpholino- oder Piperazinorest bilden, der gegebenenfalls ein- oder mehrfach durch Methyl substituiert sein kann, ein Triazolorest, ein Imidazolorest, ein Phthalimid,

$R_2$ eine verzweigte oder unverzweigte Alkylsulfonyloxygruppe mit 1 bis 4 Kohlenstoffatomen, bevorzugt Methylsulfonyloxy, eine verzweigte oder unverzweigte Alkylcarbonyloxygruppe mit 1 bis 8 Kohlenstoffatomen, bevorzugt 1 bis 4 Kohlenstoffatomen;

$R_2$ ein Phenyloxyrest, ein Pyridyloxyrest, 3,4-Methylendioxyphenoxy, eine 1,2,4-Triazol-3-yl-thiogruppe, Methoxy,

$R_3$ Phenyl, bevorzugt o-Chlorphenyl, bedeuten können, sowie gegebenenfalls ihre physiologisch unbedenklichen Säureaddtionssalze, gegebenenfalls ihre optisch aktiven Verbindungen.

Bevorzugte Imidoreste sind:

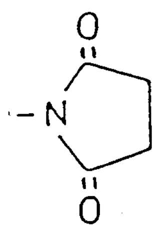

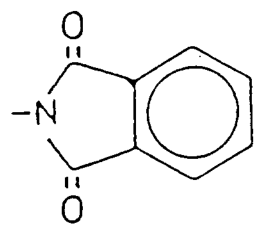

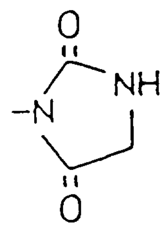

Als Alkylgruppen (auch soweit sie Bestandteile anderer Reste sind) werden bevorzugt, soweit nichts anderes angegeben ist, Methyl, Ethyl, Propyl, iso-Propyl, n-Propyl, n-Butyl, iso-Butyl und tert.-Butyl. Von den langkettigen Alkylresten sind n-Hexadecanyl und n-Octadecanyl bevorzugt

Die Angabe der Zahl der Kohlenstoffatome bezieht sich, sofern nichts anderes angegeben, auf die Länge der Alkyl-, Alkenyl- oder Alkinylkette ohne die Carbonylfunktion.

Besonders bevorzugt sind anellierte 6-gliedrige Ringe A, worin die Seitenkette $Z_n$-$R_2$ in der 3- oder 4-Position des Hetrazepins oder anellierte 5-gliedrige Ringe A, worin die Seitenkette $Z_n$-$R_2$ in der 3-Position des Hetrazepins substituiert ist.

Die neuen Verbindungen der allgemeinen Formel Ia können nach bekannten Verfahren aus den entsprechenden Thienodiazepinthionen der allgemeinen Formel II oder aber durch Variation funktioneller Gruppen der Seitenkette des bereits fertiggestellten Hetrazepingerüstes erhalten werden.

Die neuen Verbindungen der allgemeinen Formel Ib erhält man durch Reduktion von Verbindungen der allgemeinen Formel Ia. Die Umsetzung erfolgt mit bekannten Reduktionsmitteln in organischen Lösungsmitteln, so z.B. mit Zink in einer Mischung aus Eisessig und einem inerten organischen Lösungsmitteln, wie z.B. halogenierten Kohlenwasserstoffen, wie z.B. Dichlormethan, bei Temperaturen zwischen Raumtemperatur und dem Siedepunkt des Reaktionsgemisches oder z.B. mittels Lithiumaluminiumhydrid (soweit $R_2$ unter den angewandten Reaktionsbedingungen nicht reduziert wird).

Verbindungen der allgemeinen Formel Ib, in denen $R_4$ eine Alkyl- oder Acylgruppe bedeutet, lassen sich aus den zuvor genannten Verbindungen durch Alkylierung oder Acylierung nach bekannten Verfahren herstellen.

Verbindungen der allgemeinen Formel Ia1

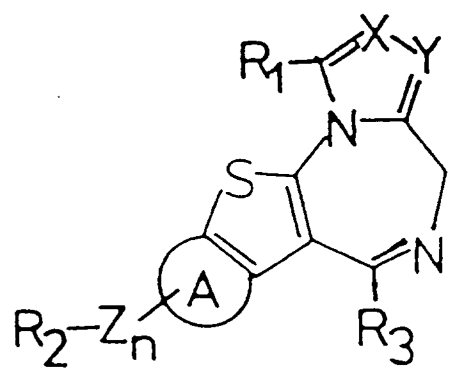

worin $R_2$ = -COOR' eine Estergruppierung wie zuvor definiert, bevorzugt R' = niederes Alkyl mit 1 bis 4 Kohlenstoffatomen, besonders bevorzugt Methyl oder Ethyl bedeutet, sind pharmakologisch wirksam und zugleich wichtige Zwischenverbindungen zur Herstellung $R_2$-funktionalisierter Hetrazepine der allgemeinen Formel Ia bzw. Ib.

Verbindungen der allgemeinen Formel I mit einem ankondensierten Triazolring können in üblicher Weise aus den entsprechenden Thieno-1,4-diazepin-thionen der allgemeinen Formel

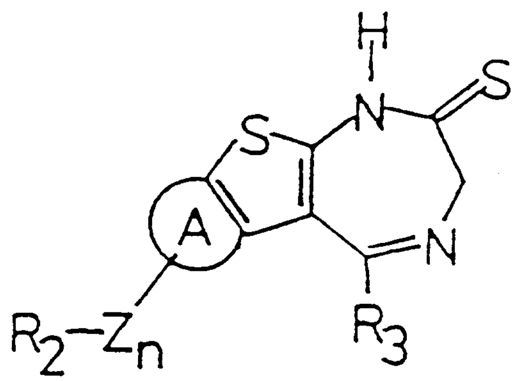

II

(bevorzugt $R_2$ = -COOR' (bevorzugt R' = niederes Alkyl oder Wasserstoff) oder $R_2$, Wasserstoff, Alkylcarbonyloxy, einen Ether- oder Thioether, ein Säureamid oder ein Amin)
hergestellt werden.

Hierzu kann eine Verbindung der Formel II entweder

a) mit einem Säurehydrazid der allgemeinen Formel

$R_1$-CONHNH$_2$ III

umgesetzt,
oder aber

b) mit Hydrazin in eine Verbindung der allgemeinen Formel

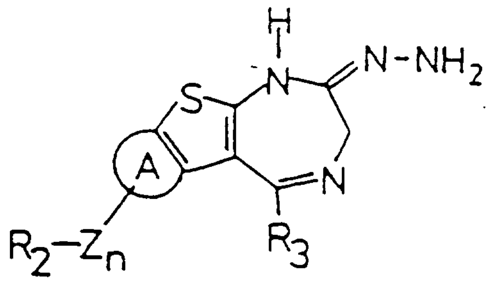

IV

überführt
und anschließend mit einem reaktiven Säurederivat, insbesondere mit einem Säurehalogenid, bevorzugt einem Säurechlorid, der allgemeinen Formel

$R_1$-COHal V

oder mit einem Orthoester der allgemeinen Formel

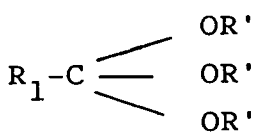

VI

worin R' eine niedere Alkylgruppe, bevorzugt Methyl oder Ethyl bedeutet,
umgesetzt werden.

Die Umsetzung des Thions II mit einem Säurehydrazid III nach dem Verfahren a) erfolgt in einem inerten organischen Lösungsmittel, wie z.B. Dioxan, Dimethylformamid, Tetrahydrofuran oder einem geeigneten Kohlenwasserstoff, wie z.B. Benzol oder Toluol bei Temperaturen zwischen der Raumtemperatur und dem Siedepunkt des Reaktionsgemisches. Die Isolierung der Endprodukte geschieht nach bekannten Methoden, wie z.B. durch Kristallisation oder Säulenchromatographie.

Die Umsetzung des Thions II mit Hydrazin nach dem Verfahren b) erfolgt in inerten organischen Lösungsmitteln, wie beispielsweise Tetrahydrofuran, Dioxan, halogenierten Kohlenwasserstoffen, wie z.B. Methylenchlorid, geeigneten Kohlenwasserstoffen, bei Temperaturen zwischen Raumtemperaturen und dem Siedepunkt des Reaktionsgemisches.

Die dabei entstehenden Hydrazino-1,4-diazepine können nach herkömmlichen Verfahren isoliert oder aber auch direkt weiterverarbeitet werden.

Die weitere Umsetzung mit einem Säurehalogenid V oder einem Orthoester VI erfolgt in einem inerten organischen Lösungsmittel, wie z.B. halogenierten Kohlenwasserstoffen, cyclischen oder aliphatischen Ethern,
kann aber auch direkt in Substanz, zweckmäßigerweise mit einem Überschuß des Orthoester, erfolgen. Die Isolierung des Endprodukts I erfolgt nach bekannten Methoden, beispielsweise durch Kristallisation.

Der Aufbau der Hetrazepine der allgemeinen Formel Ia, in der X eine CH-Gruppe und Y Stickstoff bedeutet, erfolgt in an sich bekannter Weise aus dem Thion der allgemeinen Formel II, durch Umsetzung mit einem Aminoalkin der allgemeinen Formel VII, worin $R_{11}$ Wasserstoff oder eine Alkylgruppe, bevorzugt Wasserstoff, bedeutet, wobei durch den Einsatz von Hydrochloriden für den Hetrazepinringschluß hydrolyseempfindliche $R_2$-Gruppen zugänglich werden.

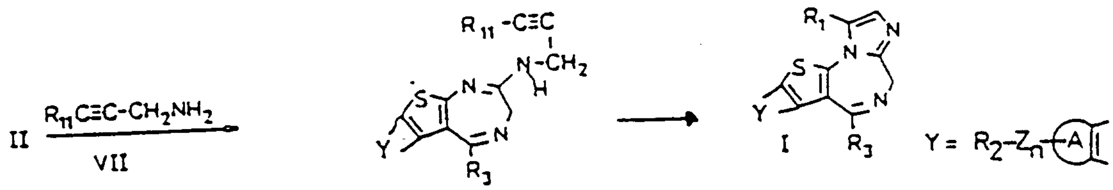


Nach diesem Verfahren können Verbindungen der allgemeinen Formel I hergestellt werden, worin $R_1$ eine Alkyl, bevorzugt die Methylgruppe, bedeutet.

Ein weiteres Verfahren besteht in der Umsetzung des Thions der allgemeinen Formel II mit einem $\alpha$-Aminoaldehyd-alkylacetal oder $\alpha$-Aminoketon-alkylketal der allgemeinen Formel VIII nach folgendem Syntheseschema,

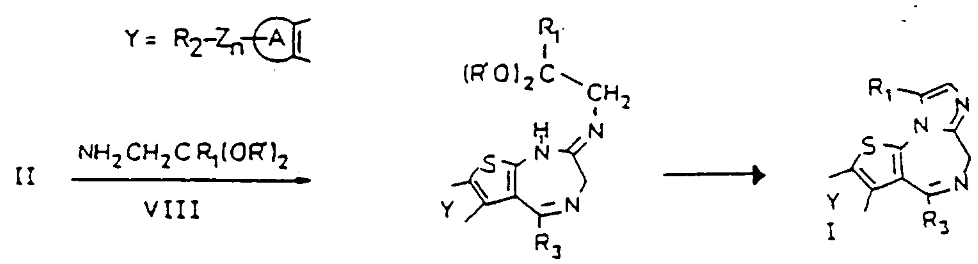


VIII

wobei $R_1$ Wasserstoff oder eine Alkylgruppe mit 1 bis 4 Kohlenstoffatomen und R' eine niedere Alkylgruppe bedeutet.

Analogieverfahren zur Synthese eines Acetals oder Ketals der allgemeinen Formel VIII sowie ein Analogieverfahren zum Ringschluß sind in der schweizerischen Patentschrift CH 580 099 beschrieben.

Verbindungen der allgemeinen Formel Ia, in denen X Stickstoff und Y CH bedeutet, können durch Decarboxylierung von Verbindungen der allgemeinen Formel

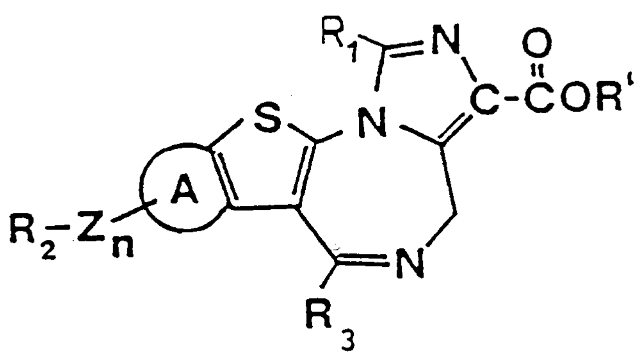

Ic

R' = niederes Alkyl

erhalten werden. Analogieverfahren zur Herstellung geeigneter Verbindungen der allgemeinen Formel Ic sind beispielsweise in der italienischen Patentschrift It 78 03 585 beschrieben.

Verbindungen der allgemeinen Formel I, die einen [1,5-a] verknüpften Imidazolring enthalten, können beispielsweise auch in Analogieverfahren zu den in der DE-OS 25 40 522 beschriebenen hergestellt werden.

Verbindungen der allgemeinen Formel I, in denen $R_1$ Chlor oder Brom bedeuten, werden aus Verbindungen mit $R_1$ = Wasserstoff durch Umsetzung mit Chlor oder Brom in Pyridin hergestellt.

Die entsprechenden Alkoxyverbindungen erhält man beispielsweise aus einer der zuvor erwähnten Chlor- bzw. Bromverbindung durch Umsetzung mit dem entsprechenden Alkoholat.

Bevorzugt werden die Reste $R_1$ für Halogen und Alkoxy, jedoch erst nach dem Aufbau des vollständig funktionalisierten Hetrazepins der allgemeinen Formel Ia nach der beschriebenen Methode eingefügt.

Die oben beschriebenen Verfahren zur Herstellung von Verbindungen der allgemeinen Formel I bzw. Ia erfolgen aus dem Thion der allgemeinen Formel II, bei denen der funktionelle Rest $R_2$ unter den angewandten Reaktionsbedingungen nicht angegriffen wird oder aber durch entsprechende Schutzgruppen geschützt werden kann. Dies trifft insbesondere für $R_2$ = Wasserstoff, eine geschützte Carbonylfunktion oder eine Esterfunktion zu.

So sind Diazepinthione der allgemeinen Formel II wie folgt herstellbar.

In Analogie zu dem von Gewald et al. Chem. Ber. 98, 3571 (1965), ibid 99, 94 (1966) beschriebenen Verfahren, erhält man ausgehend von den entsprechend funktionalisierten Cyclohexanon-, Cyclopentanon- oder Cycloheptanonderivaten a durch Reaktion mit dem entspechenden Cyanoacetophenon b die funktionalisierten Thiene c. Nach bekannten Verfahren werden diese durch Bromacetylierung und anschließende Umsetzung mit Ammoniak in die ringgeschlossenen 1,4- Diazepinone überführt, die anschließend mit Phosphorpentasulfid oder Lawesson Reagenz [R] in das Thion der allgemeinen Formel II überführt werden.

Syntheseschema a

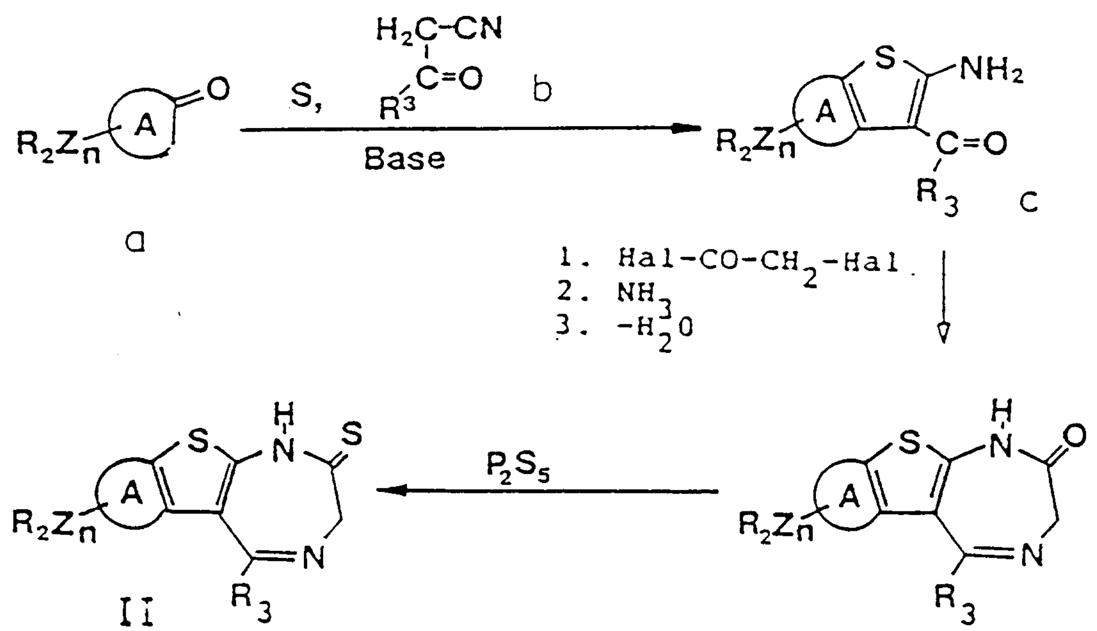


Bevorzugt hat $R_2$ die Bedeutung eines Carbonsäureesters, wie z.B.eines Carbonsäuremethyl- oder ethylesters oder Wasserstoff.

Die Verbindungen der allgemeinen Formel c (Syntheseschema a) sind neu und werden als Zwischenprodukte beansprucht. Verbindungen dieser Struktur sind wertvolle Synthesebausteine zum Aufbau der Hetrazepine der allgemeinen Formel 1a und 1b. Aus dem Stand der Technik waren bislang keine Zwischenprodukte bekannt, die eine sinnvolle Synthese der Endverbindungen ermöglicht hätte. Die DE-OS 22 33 457 erwähnt lediglich Zwischenverbindungen, die keine funktionelle Gruppe tragen. Verbindungen der allgemeinen Formel Ia, in denen A ein unsubstituiertes, an das Thienodiazepin ankondensiertes Cyclopentanon, Cyclohexanon oder Cycloheptanon ist, können beispielsweise aus den eine Carbonylfunktion enthaltenen 2-Amino-3-benzoyl-thienoderivaten nach einer der zuvor beschriebenen Methode hergestellt werden. Hierzu ist es jedoch notwendig, die Carbonylfunktion des carboxcyclischen Ringes mit einer Schutzgruppe zu versehen. Das Reaktionsschema b verdeutlicht das Prinzip der Reaktion am Beispiel des Cyclohexanonderivates.

Reaktionsschema b:

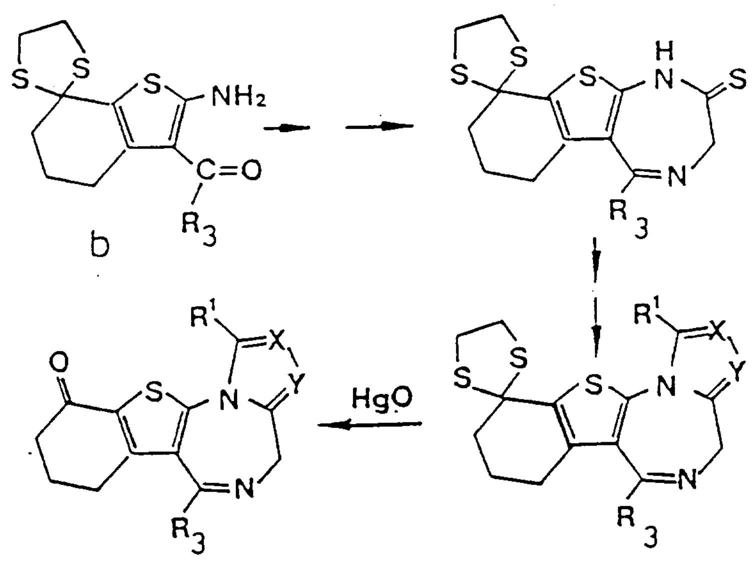


Analog dem Reaktionsschema b lassen sich die entsprechenden Cyclopentanon- Cyclohexanon- oder Cycloheptanonderivate herstellen. Entsprechend geschützte bifunktionalisierte Cycloalkanone, wie z.B. das 1,4-Dioxaspiro[4,5]decan-8-on, zur Herstellung der Thiophenderivate des Typs b, nach der von Gewald beschriebenen Methode, sind literaturbekannt. Die Einführung und Abspaltung der Schutzgruppen erfolgt ebenfalls nach literaturbekannten Verfahren.

Verbindungen der allgemeinen Formel Ia, worin A ein ankondensierter Tetrahydropyridorest ist, können ausgehend von N-Acetylpiperidon-4 in Analogie zu der zuvor beschriebenen Methode nach Gewald in N-Acetyl-hetrazepine der allgemeinen Formel Ia umgesetzt werden. Die weitere Funktionalisierung der N-Acetylgruppe erfolgt nach bekannten Methoden. Verbindungen mit $R^o$ = Wasserstoff erhält man aus den entsprechenden N-Thioacetylderivaten durch Verseifen. Aus diesen lassen sich nach Analogieverfahren Verbindungen der allgemeinen Formel Ia mit den genannten Resten $R^o$ herstellen.

Verbindungen der allgemeinen Formel

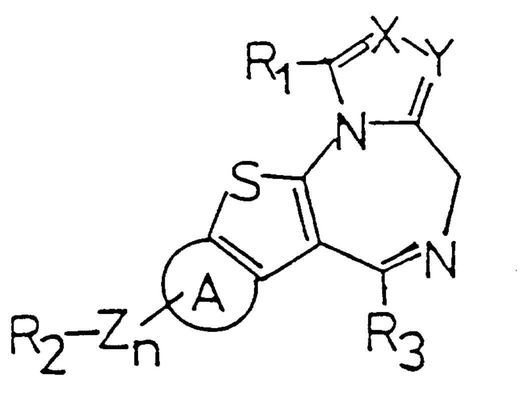

I

worin A, Z, n, $R_3$ und $R_2$ wie zuvor definiert, $R_2$ bevorzugt eine Esterfunktion aufweist, sind wertvolle Ausgangsverbindungen zur Synthese pharmakologisch wirksamer Verbindungen und werden als Zwischenverbindungen beansprucht.

Die Carbonsäureester der allgemeinen Formel Ia sind wertvolle Ausgangsverbindungen zur Einführung weiterer funktioneller Gruppen.

Ausgehend von den Estern können die entsprechenden Carbonsäuren der allgemeinen Formel Ia durch Verseifen, z.B. mit KOH in Ethanol, erhalten werden.

Carbonsäureamide der allgemeinen Formel Ia können nach bekannten Verfahren hergestellt werden, wie z.B. aus den entsprechenden Carbonsäuren oder deren Carbonsäureäquivalenten durch Umsetzung mit einem primären oder sekundären Amin oder Ammoniak der allgemeinen Formel

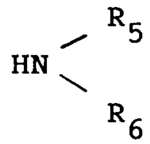

Bevorzugt ist die Überführung in ein Carbonsäurechlorid oder Säureanhydrid oder die Umsetzung der Säure in Gegenwart von Carbonyldiimidazol, Sulfonyldiimidazol, Cyclohexylcarbodiimid.

Die Umsetzung der freien Säure mit dem Amin erfolgt in Gegenwart eines Carbodiimids, beispielsweise von Cyclohexylcarbodiimid, Carbonyldiimidazols oder Sulfonyldiimidazols in einem inerten Lösungsmittel wie Dimethylformamid, Tetrahydrofuran, Dioxan oder halogenierten Kohlenwasserstoff bei Temperaturen zwischen O ° C und dem Siedepunkt des Reaktionsgemisches.

Bei der Reaktion des Amins mit einem Säurehalogenid oder Säureanhydrid wird das Amin in einem inerten Lösungsmittel, beispielsweise Dimethylformamid, Tetrahydrofuran, Dioxan oder einem geeigneten Kohlenwasserstoff wie Toluol bei Temperaturen zwischen Raumtemperatur und dem Siedepunkt des Reaktionsgemisches mit dem Säurehalogenid oder dem Säureanhydrid umgesetzt, wobei gegebenenfalls ein säurebindendes Mittel wie Natriumcarbonat, Natriumbicarbonat oder eine tertiäre organische Base, beispielsweise Pyridin oder Triethylamin, zugegeben wird.

Falls es sich bei dem Amin um eine Flüssigkeit handelt, kann die Umsetzung auch in einem Überschuß des Amins ohne zusätzliches Lösungsmittel erfolgen.

Das Säurehalogenid bzw. Säureanhydrid erhält man aus der freien Säure in üblicher Weise, z.B. durch Reaktion der Säure mit einem Thionylhalogenid bzw. durch Umsetzung eines Alkalisalzes der Säure mit Acetylchlorid oder Chlorameisensäurechlorid.

Anstelle der Reaktion mit einem Amin kann auch mit einem Aminosäurederivat umgesetzt werden.

Ester der allgemeinen Formel Ia, insbesondere die Methyl- oder Ethylester, können durch selektive Reduktion der Esterfunktion in den entsprechenden Alkohol überführt werden. Die Reaktion wird unter inverser Zugabe des Reduktionsmittels, wie z.B. Lithiumalanat oder Natriumborhydrid (inverse Aktivierung), unter allgemein üblichen Reaktionsbedingungen durchgeführt, wie z.B. in inerten organischen Lösungsmitteln, z.B. Ethern, Tetrahydrofuran bei Temperaturen zwischen Raumtemperatur und dem Siedepunkt des Reaktionsgemisches.

Carbamate oder Harnstoffe der allgemeinen Formel Ia mit $R_2$ = $R_{10}NHCOO$ - oder $R_{10}NHCONR_{11}$ - erhält man aus der Reaktion der entsprechenden Alkohole oder Amine mit dem gewünschten Isocyanat in organischen Lösungsmitteln, wie z.B. Tetrahydrofuran, Methylenchlorid, bei Temperaturen zwischen Raumtemperatur und dem Siedepunkt, bevorzugt bei erhöhten Temperaturen, des Reaktionsgemisches unter Zusatz von Base, bevorzugt DABCO (1,4-Diazabicyclo(2,2,2)octan). Verbindungen, in denen $R_2$ Alkyl- oder Phenylcarbonyloxy ist, werden aus den entsprechenden Alkoholen der allgemeinen Formel Ia durch Umsetzung mit einem Säureäquivalent abgeleitet von einer Carbonsäure der allgemeinen Formel

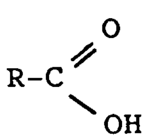

worin R ein Arylrest oder bevorzugt ein verzweigter oder unverzweigter Alkylrest mit 1 bis 8 Kohlenstoffatomen, wobei die Kohlenstoffkette durch Stickstoff, Sauerstoff oder Schwefel unterbrochen sein kann, hergestellt. Hierbei können die gleichen Reaktionsbedingungen wie bei der Herstellung der Säureamide angewandt werden.

Aus den Carbonsäuren der allgemeinen Formel Ia mit $R_2$ = COOH lassen sich nach bekannten Methoden die Carboxylazide herstellen, diese können dann in einem inerten organischen Lösungsmittel, wie z.B. Dioxan, durch die Curtiusumlagerung in die Isocyanate überführt werden. Diese Isocyanate lassen sich nach allgemein bekannten Verfahren wie zuvor beschrieben, in Urethane und Harnstoffe oder durch Hydrolyse in die primären Amine umwandeln.

Ausgehend von Verbindungen der allgemeinen Formel Ia, in denen $R_2$ = OH ist, erhält man durch Reaktion mit Alkyl- bzw. Arylsulfonsäurehalogenide Verbindungen der allgemeinen Formel Ia, worin $R_2$ eine Alkyl- oder Arylsulfonyloxygruppe darstellt. Die Umsetzung erfolgt in inerten organischen Lösungsmitteln, wie z.B. Methylenchlorid, mit Sulfonsäurehalogeniden unter Zusatz von Säurebindern, wie z.B. Triethylamin. Die so erhaltenen Mesylate enthalten den Mesylrest als gute Abgangsgruppe, der nucleophil ausgetauscht werden kann. Entsprechend funktionalisierte Verbindungen der allgemeinen Formel Ia, z.B. $R_2$ = $CH_3SO_3$-, können mit primären oder sekundären Aminen der Formel

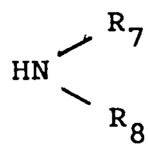

oder einen Imidorest, z.B. Phthalimid umgesetzt werden. Man erhält Verbindungen der allgemeinen Formel I, worin $R_2$ die Gruppe $NR_7R_8$ oder einen Imidorest aufweist.

Die Umsetzung erfolgt in inerten organischen Lösungsmitteln, wie z.B. Tetrahydrofuran, Dioxan, Dimethylformamid, zwischen Raumtemperatur und dem Siedepunkt des Reaktionsgemisches, bevorzugt bei erhöhten Temperaturen.

Ausgehend von den Mesylaten der allgemeinen Formel I erhält man durch Umsetzung der Mesylate mit den entsprechenden Alkoholen oder Mercaptanen als Solvens oder bevorzugt in Form ihrer Alkalimetallsalze in Dioxan oder Dimethylformamid bei einer Reaktionstemperatur zwischen der Raumtemperatur und dem Siedepunkt des Reaktionsemisches, bevorzugt bei 60 - 80 °C, die entsprechenden Ether- bzw. Thioether der allgemeinen Formel I

Verbindungen der allgemeinen Formel Ia mit $R_2$ = $NH_2$ erhält man auch in Analogie nach bekannten Verfahren durch Spaltung des entsprechenden Phthalimids.

Die so erhaltenen primären oder sekundären Amine können nach bekannten Verfahren mit Carbonsäureäquivalenten abgeleitet von Carbonsäuren der allgemeinen Formel

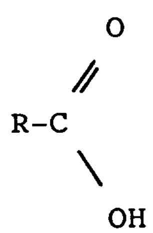

worin R die Bedeutung von $R'_5$ hat, zu Verbindungen der allgemeinen Formel Ia, worin $R_7$ oder $R_8$ eine Alkyl- oder Arylcarbonylgruppe bedeutet, umgesetzt werden.

Die Oxidation der Alkohole ergibt die um ein Kettenglied verkürzten Aldehyde.

Verbindungen der allgemeinen Formel Ia, in denen $R_2$ ein Heterocyclus, wie z.B. ein Oxazolin, Thiazolin ein Tetrahydropyrimidin oder ein Imidazolin bedeutet, erhält man beispielsweise aus den entsprechenden Carbonsäuren der allgemeinen Formel Ia durch Reaktion mit einem bisfunktionalisierten Amin, wie z.B. einem Aminoalkohol, einem Aminomercaptan oder einem Diamin, in Gegenwart von Triphenylphosphin, Tetrachlorkohlenstoff und einer tertiären organische Base in Acetonitril. In Analogie hierzu lassen sich auch die entsprechenden 6- und 7-gliedrigen Heterocyclen herstellen. Die Reaktion erfolgt in einem Temperaturbereich zwischen O °C und dem Siedepunkt der Reaktionsmischung, bevorzugt zwischen 0 °C und Raumtemperatur. Verbindungen der allgemeinen Formel Ia, worin $R_2$ ein Oxazolinrest bedeutet, erhält man auch aus den entsprechend hydroxyfunktionalisierten Carbonsäureamiden durch Ringschlußreaktion mit Thionylchlorid in einem inerten organischen Lösungsmittel, wie z.B. Methylenchlorid.

Diese lassen sich gewünschtenfalls durch Schwefelung, z.B. mit Phosphorpentasulfid oder Lawesson Reagenz [R] in das entsprechende Thiazolin überführen.

Verbindungen der allgemeinen Formel I, in der $R_2$ ein Amidin der Struktur

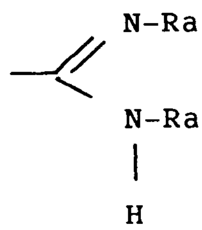

erhält man analog wie oben beschrieben aus der entsprechenden Carbonsäure und einem primären Amin.

Verbindungen der allgemeinen Formel Ia, in denen $R_2$ eine Cyanogruppe ist, erhält man aus den entsprechenden primären Carbonsäureamiden durch Reaktion mit Phosphoroxychlorid in einem inerten organischen Lösungsmittel, z.B. Dichlorethan, unter Rückflußbedingungen oder durch Umsetzung des entsprechenden Bromids mit z.B. Triethylammoniumcyanid bevorzugt bei RT (Raumtemperatur).

Verbindungen der allgemeinen Formel Ia, worin $R_2$ ein gegebenenfalls durch verzweigte oder unverzweigte Alkylgruppen substituiertes Imidazolin ist, können ausgehend von Verbindungen Ia mit $R_2$ = CN über das imidoethylesterhydrochlorid durch Reaktion mit einem Diamin erhalten werden (Pinner-Reaktion). Die Bildung des Imidoethylesterhydrochlorids erfolgt durch Behandlung des Nitrils mit einem Überschuß an ethanolischer Salzsäure. Das entstandene kristalline Rohprodukt wird in Ethanol mit dem Diamin (z.B. Ethylendiamin) zuerst unter Eiskühlung, dann unter Rückflußbedingungen umgesetzt. Man erhält so Verbindungen der Formel Ia, in der $R_2$ ein Imidazolin-2-rest ist. Dessen Aminofunktion kann im Fall von N-H nach bekannten Methoden alkyliert werden.

Verbindungen der allgemeinen Formel Ia, worin $R_2$ ein Halogenatom, z.B. Jod, erhält man aus einer Verbindung der allgemeinen Formel Ia, worin $R_2$ Toluolsulfonsäurerest ist durch Reaktion mit einem entsprechenden Halogenierungsmittel, z.B. NaJ, in wasserfreien Lösungsmitteln, wie z.B. Aceton.

Verbindungen der allgemeinen Formel Ia mit A gleich

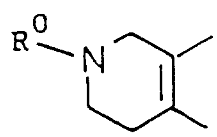

und $R^0$ gleich Alkyl, substituiertes Metyl, Alkylcarbonyl, substituiertes Alkylcarbonyl oder Arylcarbonyl erhält man durch Alkylierung bzw. Acylierung von Verbindungen der allgemeinen Formel I mit $R^0$ = Wasserstoff,

die entsprechenden Thiocarbonylverbindungen durch Umsetzung der Carbonylverbindung mit einem Schwefelreagenz, Z.B. Phosphorpentasulfid oder Lawesson Reagenz$^R$.

Soweit die erfindungsgemäßen Verbindungen ein asymmetrisch substituiertes Kohlenstoffatom aufweisen, können sie nach bekannten Methoden in ihre optisch aktiven Antipoden aufgetrennt werden, so z.B. durch Chromatographie an optisch aktivem Säulenmaterial.

Nach bekannten Analogieverfahren oder in Analogie zu den beschriebenen Methoden werden beispielsweise die folgenden Verbindungen hergestellt.

4-(Morpholin-4-yl-carbonyl)-6-(2-chlorphenyl)-11-methyl-2,3,4,5-tetrahydro-8H-[1]benzothieno[3,2-f]-[1,2,4]triazolo[4,3-a][1,4]diazepin

3-(Morpholin-4-yl-carbonyl)-6-(2-chlorphenyl)-11-methyl-2,3,4,5-tetrahydro-8H-[1]benzothieno[3,2-f][1,2,4]-triazolo[4,3-a][1,4]diazepin

3-(N,N-Diethylaminocarbonyl)-6-(2-chlorphenyl)-11-methyl-2,3,4,5-tetrahydro-8H-[1]benzothieno[3,2-f][1,2,4]-triazolo[4,3-a][1,4]diazepin

4-(N,N-Diethylaminocarbonyl)-6-(2-chlorphenyl)-11-methyl-2,3,4,5-tetrahydro-8H-[1]benzothieno[3,2-f][1,2,4]-triazolo[4,3-a][1,4]diazepin

4-(4-Methylpiperazinylcarbonyl)-6-(2-chlorphenyl)-11-methyl-2,3,4,5-tetrahydro-8H-[1]benzothieno[3,2-f]-[1,2,4]triazolo[4,3-a][1,4]diazepin

4-(N-Methyl-N-2-hydroxyethylaminocarbonyl)-6-(2-chlorphenyl)-11-methyl-2,3,4,5-tetrahydro-8H-[1]-benzothieno[3,2-f][1,2,4]triazolo[4,3-a][1,4]diazepin

4-(N,N-Dimethylaminocarbonyl)-5-(2-chlorphenyl)-10-methyl-3,4-dihydro-2H,7H-cyclopenta[4,5]thieno[3,2-f]-[1,2,4]triazolo[4,3-a][1,4]diazepin
3-(N,N-Diethylaminocarbonyl-5-(2-chlorphenyl)-10-methyl-3,4-dihydro-2H,7H-cyclopenta[4,5]thieno[3,2-f]-[1,2,4]triazolo[4,3-a][1,4]diazepin
3-(N,N-Diethylaminocarbonyl-5-(2-chlorphenyl)-10-brom-3,4-dihydro-2H,7H-cyclopenta[4,5]thieno[3,2-f]-[1,2,4]triazolo[4,3-a][1,4]diazepin
4-(Morpholin-4-yl-carbonylmethylaminocarbonyl)-5-(2-chlorphenyl)-10-brom-3,4-dihydro-2H,7H-cyclopenta-[4,5]thieno[3,2-f]-[1,2,4]triazolo[4,3-a][1,4]diazepin
4-(Morpholin-4-yl-carbonyl)-6-(2-chlorphenyl)-11-methyl-2,3,4,5-tetrahydro-8H-[1]benzothieno[3,2-f]imidazo-[1,2-a][1,4]diazepin
4-(N,N-Diethylamino)-6-(2-chlorphenyl)-11-methyl-2,3,4,5-tetrahydro-8H-[1]benzothieno[3,2-f]imidazo[1,2-a]-[1,4]diazepin
3-(N-Morpholinomethyl)-5-(2-chlorphenyl)-10-methyl-3,4-dihydro-2H,7H-cyclopenta[4,5]thieno[3,2-f][1,2,4]-triazolo[4,3-a][1,4]diazepin
3-(Acetoxymethyl)-5-(2-chlorphenyl)-10-methyl-3,4-dihydro-2H,7H-cyclopenta[4,5]thieno[3,2-f][1,2,4]triazolo-[4,3-a][1,4]diazepin
3-(1.2.4-Triazol-1-yl-methyl)-5-(2-chlorphenyl)-10-methyl-3,4-dihydro-2H,7H-cyclopenta[4,5]thieno[3,2-f]-[1,2,4]triazolo-[4,3-a][1,4]diazepin
3-(N-2,6-Dimethylmorpholino-methyl)-5-(2-chlorphenyl)-10-methyl-3,4-dihydro-2H,7H-cyclopenta[4,5]thieno-[3,2-f][1,2,4]triazolo-[4,3-a][1,4]diazepin
3-Acetoxymethyl-5-(2-chlorphenyl)-3,4-dihydro-2H,7H-cyclopenta[4,5]thieno[3,2-f][1,2,4]triazolo[4,3-a][1,4]-diazepin
4-Acetoxymethyl-6-(2-chlorphenyl)-11-methyl-2,3,4,5-tetrahydro-8H-[1]benzothieno[3,2-f]imidazo[1,2-a][1,4]-diazepin
3-Acetoxymethyl-5-(2-chlorphenyl)-10-methyl-3,4-dihydro-2H,7H-cyclopenta[4,5]thieno[3,2-f]imidazo[1,2-a]-[1,4]diazepin
3-Diethylaminomethyl-5-(2-chlorphenyl)-10-methyl-3,4-dihydro-2H,7H-cyclopenta[4,5]thieno[3,2-f][1,2,4]-triazolo[4,3-a][1,4]diazepin
4-Hydroxymethyl-6-(2-chlorphenyl)-11-methyl-2,3,4,5-tetrahydro-8H-[1]benzothieno[3,2-f][1,2,4]triazolo[4,3-a][1,4]diazepin
4-(N-Morpholinomethyl)-6-(2-chlorphenyl)-11-methyl-2,3,4,5-tetrahydro-8H-[1]benzothieno[3,2-f][1,2,4]-triazolo[4,3-a][1,4]diazepin
4-(Pyrazol-1-yl-methyl)-6-(2-chlorphenyl)-2,3,4,5-tetrahydro -8H-[1]benzothieno[3,2-f][1,2,4]triazolo[4,3-a]-[1,4]diazepin
4-[4,4-Dimethyl-2-oxazolin-2-yl]-6-(2-chlorphenyl)-11-methyl-2,3,4,5-tetrahydro-8H-[1]benzothieno[3,2-f]-[1,2,4]triazolo[4,3-a][1,4]diazepin
4-[4,4-Dimethyl-2-imidazolin-2-yl]-6-(2-chlorphenyl)-11-methyl-2,3,4,5-tetrahydro-8H-[1]benzothieno[3,2-f]-[1,2,4]triazolo[4,3-a][1,4]diazepin
4-[1,4,4-Trimethyl-2-imidazolin-2-yl]-6-(2-chlorphenyl)-11-methyl-2,3,4,5-tetrahydro-8H-[1]benzothieno[3,2-f]-[1,2,4 ]triazolo[4,3-a][1,4]diazepin
4-Aminocarbonyl-6-(2-chlorphenyl)-11-methyl-2,3,4,5-tetrahydro-8H-[1]benzothieno[3,2-f][1,2,4]triazolo[4,3-a][1,4]diazepin
4-[Morpholin-4-yl-carbonyl]-6-(2-chlorphenyl)-2,3,4,5-tetrahydro-8H-[1]benzothieno[3,2-f]imidazo[1,5-a][1,4]-diazepin
4-[4,4-Dimethyl-2-thiazolin-2-yl]-6-(2-chlorphenyl)-11-methyl-2,3,4,5-tetrahydro-8H-[1]benzothieno[3,2-f]-[1,2,4]triazolo[4,3-a][1,4]diazepin
4-Amino-6-(2-chlorphenyl)-11-methyl-2,3,4,5-tetrahydro-8H-benzothieno[3,2-f][1,2,4]triazolo[4,3-a][1,4]-diazepin
3-(N-Morpholinyl-methyl)-5-(2-chlorphenyl)-10-methyl-3,4-dihydro-2H,7H-cyclopenta[4,5]thieno[3,2-f]imidazo-[1,2-a][1,4]diazepin
4-(1,2,4-Triazol-1-yl-methyl)-6-(2-chlorphenyl)-11-methyl-2,3,4,5-tetrahydro-8H-[1]benzothieno[3,2-f][1,2,4]-triazolo[4,3-a][1,4]diazepin
4-(1,2,4-Triazol-1-yl-methyl)-6-(2-chlorphenyl)-11-methyl-2,3,4,5-tetrahydro-8H-[1]benzothieno[3,2-f]imidazo-[1,2-a][1,4]diazepin.

Die erfindungsgemäßen Verbindungen besitzen PAF-antagonistische Wirkung.
Bekanntlich handelt es sich bei PAF (Plättchen Aktivierender Faktor) um das Phospholipid Acetyl-glyceryl-ether-phosphoryl-cholin (AGEPC), das als potenter Lipidmediator bekannt ist, der von tierischen und menschlichen proinflammatorischen Zellen freigesetzt wird. Unter solchen Zellen finden sich hauptsächlich

basophile und neutrophile Granulozyten, Makrophagen (aus Blut und Gewebe) sowie Thrombozyten, die an Entzündungsreaktionen beteiligt sind.

PAF zeigt im pharmakologischen Experiment Bronchokonstriktion, Blutdrucksenkung, Auslösung einer Thrombozytenaggregation sowie eine proinflammatorische Wirkung.
Diese experimentell nachweisbaren Wirkungen des PAF weisen direkt oder indirekt auf mögliche Funktionen dieses Mediators in der Anaphylaxie, in der Pathophysiologie des Asthma bronchiale und allgemein in der Entzündung hin.

PAF-Antagonisten werden benötigt, um einerseits weitere pathophysiologische Funktionen dieses Mediators an Tier und Mensch aufzuklären und andererseits pathologische Zustände und Krankheiten, an denen PAF beteiligt ist, zu behandeln. Beispiele für die Indikationen eines PAF-Antagonisten sind Entzündungsprozesse des Tracheobronchialbaumes (akute und chronische Bronchitis, Asthma bronchiale) oder der Niere (Glomerulonephritis), der Gelenke (rheumatische Erkrankungen), anaphylaktische Zustände, Allergien und Entzündungen im Bereich der Schleimhäute und der Haut (z.B. Psoriasis) sowie durch Sepsis, Endotoxine oder Verbrennungen bedingte Schockzustande. Weitere wichtige Indikationen für einen PAF-Antagonisten sind Läsionen und Entzündungen im Bereich der Magen- und Darmschleimhaut, wie z.B. Gastritis, im allgemeinen Ulcus pepticum, jedoch insbesondere Ulcus ventriculi und Ulcus duodeni.

Weiterhin eignen sich die erfindungsgemäßen Verbindungen zur Behandlung bei den folgenden Indikationsstellungen: Obstruktive Lungenerkrankungen, wie z.B. bronchiale Hyperreaktivität, entzündliche Lungenwegserkrankungen, wie z.B. chronische Bronchitis;
Herz- Kreislauferkrankungen, wie z.B. Polytrauma, Anaphylaxe, Arteriosklerose, entzündliche Darmerkrankungen, EPH-Gestose (ederma-proteinuria Hypertension), Erkrankungen des extrakorporalen Kreislauf, ischämische Erkrankungen, entzündliche und immunologische Erkrankungen, Immunmodulation bei Transplantationen von Fremdgeweben, Immunmodulation bei Leukämie. Metastasenausbreitung z.B. bei bronchialer Neoplasie, Erkrankungen des ZNS, wie z.B. Migräne, Agarophobie (panic disorder), weiterhin erweisen sich die erfindungsgemäßen Verbindungen als Cyto- und Organoprotektiv, z.B. zur Neuroprotektion, z.B. bei Leberzirrhose, DIC (disiminierte intravasale Gerinnung);
Nebenwirkungen einer Arzneimitteltherapie, z.B. anaphylaktoide Kreislaufreaktionen, Kontrastmittelzwischenfälle, Nebenwirkungen bei der Tumortherapie;
Unverträglichkeiten bei Bluttransfusionen; fulminantes Leberversagen ($CCl_4$-Intoxikation) Amanitaphalloides-Intoxikation (Knollenblätterpilzvergiftung);
Symptome von parasitären Erkrankungen (z.B. Wurmerkrankungen)
Neue zusätzliche Indikation: Immunfunktion bei Aids, Diabetes, juvenile Diabetes, diabetische Retinopathie, polytraumatischer Schock, hämorrhagischer Schock, CNS: Ishämie, Multiple Sklerose.
PAF assoziierte Interaktion mit Gewebshormon (autocoid hormones), Lymphokine und anderen Mediatoren.

Die PAF-antagonistische Wirkung einzelner Benzodiazepine ist bekannt, vgl. E. Kornecki et al, Science 226, 1454 - 1456 (1984). Für Alprazolam wurde nach der unten beschriebenen Methode ein $IK_{50}$ - (Konzentration bei einer 50%igen Aggregationshemmung) von 14 $\mu$M, für Triazolam ein $IK_{50}$ von 9 $\mu$M gemessen. Diese, als Tranquilizer beziehungsweise Hypnotika ausgewiesenen und im Handel befindlichen Verbindungen sind jedoch wegen ihrer ausgeprägten sedierenden Wirkung trotz ihrer relativ guten PAF-antagonistischen Wirkung zum Einsatz als PAF-Antagonisten in der Therapie in vielen Fällen ungeeignet.

Bei den erfindungsgemäßen Verbindungen hingegen fehlt die sedierenden Wirksamkeit, während die PAF-antagonistische Wirkung im Vergleich zu der der bekannten Benzodiazepine zumindest gleichwertig ist.

Die PAF-antagonistische Wirksamkeit einiger Verbindungen der Formeln Ia und Ib wurde anhand der Hemmung der Blutplättchen-Aggregation in vitro geprüft.

1. In vitro Untersuchungen: Hemmung der Blutplättchen-Aggregation

Zur Bestimmung der PAF-antagonistischen Wirkung von Substanzen wurde die durch PAF induzierte Aggregation von Humanthrombozyten in vitro verwendet. Zur Gewinnung von thrombozytenreichem Plasma (TRP) erfolgt die Blutentnahme aus einer nicht gestauten Vene mit Hilfe einer Plastikspritze, in der sich 3,8 %ige Natriumcitratlösung befindet. Das Verhältnis zwischen Natriumcitratlösung und Blut beträgt 1:9. Nach vorsichtiger Durchmischung wird das Citratblut bei 150 x g (1200 U/min) 20 Minuten lang zentrifugiert. Die Messung der Thrombozytenaggregation erfolgt nach dem von Born und Cross ausgearbeiteten Verfahren (G. V. R. Born und M.J. Cross, J. Physiol. 168, 178 (1963)), wobei dem TRP unter ständigem Rühren PAF als Auslöser der Aggregation zugesetzt wird.

Die Prüfsubstanz wird jeweils 2 - 3 Minuten vor Auslösung der Aggregation in einem Volumen von 10 $\mu$l zugesetzt. Als Lösungsmittel dienen entweder Aqua.dest., Ethanol und/oder Dimethylsulfoxyd. Kontrollansätze erhalten entsprechende Volumina dieser Lösungsmittel. Nach Registrierung der Ausgangsabsorption (2-3 Minuten) wird die Aggregation mit PAF ($5 \times 10^{-8}$ M) induziert.

Zur Beurteilung von Substanzeffekten wird das Maximum der ersten Aggregationswelle verwendet. Die durch PAF induzierte maximale Absorptionsrate (= maximale Aggregation x 100 %) wird jeweils gleichzeitig in einem Parallelansatz (= Kontrollansatz in einem der Kanäle des 2 Kanal-Aggregometers) zu jedem Testansatz (zweiter Kanal) mitgeprüft und als 100 %-Wert verwendet. Der unter dem Einfluß der Testsubstanz erreichte Aggregationswert wird als 100 % angegeben.

Jede Prüfsubstanz wird bei Konzentrationen von $10^{-3}$ bis $10^{-8}$ M mit einem Stichprobenumfang von jeweils n = 4 hinsichtlich einer hemmenden Wirkung auf die durch PAF induzierte Thrombozytenaggregation untersucht. Danach wird eine Konzentrations-Wirkungskurve anhand von 3 Konzentrationen erstellt und die $IK_{50}$ (Konzentration bei einer 50%igen Aggregationshemmung) ermittelt. Bei einigen Verbindungen werden lediglich orientierende Versuche durchgeführt.

Aus dem Stand der Technik sind zahlreiche Thieno- und Benzodiazepine bekannt, die sich jedoch strukturell von den erfindungsgemäßen Verbindungen unterscheiden, so z.B. die im Europäischen Recherchenbericht zitierte DE-A-25 08 329, die ein ankondensiertes [1,2,4]Triazolo[2,3-a]-Ringsystem enthält, die als Tranquillizer bekannt sind.

Aus der europäischen Patentanmeldung Nr. 176 927 ist die PAF-antagonistische Wirkung von 6-(2-Chlorphenyl)-11-methyl-2,3,4,5-tetrahydro-8H-[1]benzothieno[3,2-f][1,2,4]triazolo[4,3-a][1,4]diazepin (A) und von 6-(2-Chlorphenyl)-11-methyl-2,3,4,5-tetrahydro-8-H-pyrido[4',3':4,5]thieno-[3,2-f][1,2,4]triazolo-[4,3-a][1,4]diazepin (B) bekannt.

Für diese Verbindungen werden $IK_{50}$-Werte (die Konzentration, bei der eine 50%ige Aggregationshemmung bestimmt wurde) von < 2,5 (A) bzw. < 10 (B) angegeben.

Beide Verbindungen zeigen im Benzodiazepin-Rezeptor-Bindungstest mit [$^3$H] Flunitrazepam als Radioligand (Bechtel et al in Arzneimittelforschung 3a, 1986, 534) Werte von 21 (A) bzw. 35 (B) ($10^{-9}$ Mol), was auf eine zentrale Wirkung dieser Verbindungen hindeutet. Überraschenderweise wurde nun gefunden, daß einige der erfindungsgemäßen Verbindungen der allgemeinen Formel Ia im Flunitrazepam-Bindungstest wesentlich größere Werte aufweisen und nicht mehr ZNS-aktiv sind.

In der nachfolgenden Tabelle sind die "PAF-Werte" sowie Werte für den Flunitrazepam-Bindungstest einiger bekannter wie auch einiger erfindungsgemäßer Verbindungen aufgeführt: Wirkungsstarke PAF-Antagonisten, die ebenfalls keine ZNS-Aktivität aufweisen, sind aus der EP-A-194 416 bekannt, diese unterscheiden sich jedoch strukturell ebenfalls von den beanspruchten erfindungsgemäßen Verbindungen durch das Fehlen des zusätzlich ankondensierten Ringes.

Tabelle A

| Verbindung | $IK_{50}$-Wert PAF $10^{-6}$ | $IK_{50}$-Wert FNB $10^{-9}$ | Mol |
|---|---|---|---|
| Alprazolam | 14 | 5.5 | |
| Triazolam | 9 | 1.4 | |
| Verbindung (A) | < 2.5 | 21 | |
| Verbindung (B) | < 7 | 35 | |
| Beispiel 1 | 0.5 | > 5 000 | |
| Beispiel 2 | 2.5 | > 5 000 | |
| Beispiel 7 | 0.3 | 3600 | |
| Beispiel 10 | 0.3 | - | |
| Beispiel 11 | 0.5 | 5 000 | |
| Beispiel 17 | 1.8 | 1 070 | |
| Beispiel 18 | 1.3 | - | |
| Beispiel 19 | 0.6 | - | |
| Beispiel 21 | 0.3 | >5 000 | |
| Beispiel 25 | 1 | 2 300 | |
| Beispiel 33 | 2,3 | - | |
| Beispiel 35 | 3.3 | >5 000 | |
| Beispiel 36 | 0.4 | >5 000 | |
| Beispiel 37 | 1.8 | >5 000 | |
| Beispiel 38 | 2.7 | - | |
| Beispiel 46 | 4.8 | 1 460 | |
| Beispiel 58 | 0.4 | 380 | |
| Beispiel 62 | 0.4 | 4 600 | |
| Beispiel 63 | 0.4 | >5 000 | |
| Beispiel 64 | 0.8 | - | |
| Beispiel 68 | 0.4 | - | |
| Beispiel 79 | 2,2 | 4 800 | |
| Beispiel 83 | 0.9 | - | |
| Beispiel 86 | 1 | >5 000 | |
| Beispiel 66 | <0.2 | 920 | |
| Beispiel 113 | <0.2 | 3000 | |

Die neuen Verbindungen der allgemeinen Formeln Ia oder Ib können topisch, oral, transdermal, parenteral oder durch Inhalation verabreicht werden. Die Verbindungen liegen hierbei als aktive Bestandteile in üblichen Darreichungsformen vor, z.B. in Zusammensetzungen, die im wesentlichen aus einem inerten pharmazeutischen Träger und einer effektiven Dosis des Wirkstoffes bestehen, wie z.B. Tabletten, Dragees, Kapseln, Oblaten, Pulver, Lösungen, Suspensionen, Inhalationsaerosole, Salben, Emulsionen, Sirupe, Suppositorien usw.. Eine wirksame Dosis der erfindungsgemäßen Verbindungen liegt bei

oraler Anwendung zwischen 1 und 50, vorzugsweise zwischen 3 und 20 mg/Dosis, bei intravenöser oder intramuskulärer Anwendung zwischen 0,001 und 50, vorzugsweise zwischen 0,1 und 10 mg/Dosis. Für die Inhalation sollen Lösungen, die 0,01 bis 1,0, vorzugsweise 0,1 bis 0,5 % Wirkstoff enthalten, eingesetzt werden.

2. In vivo Untersuchungen

2.1. Antagonisierung der durch PAF bewirkten Bronchokonstriktion an narkotisierten Meerschweinchen

Spontan atmende männliche, 300 - 450 g schwere Meerschweinchen werden 1 Stunde vor einer i.v. Infusion von PAF (30 ng/(kg x min) mit der Testsubstanz oder einem Kontrollvehikel oral vorbehandelt. Die Versuchstiere werden dann mit 2 mg/kg Urethan intraperitoneal anästhesiert, worauf die Vena jugularis, die Arteria carotis und die Trachea kanüliert werden. Die PAF-Infusion induziert bei den Kontrolltieren eine starke, anhaltende Bronchokonstriktion, die anhand des Atemzugvolumens, der Compliance und der Resistance gemessen wird und ebenso eine Senkung des Blutdruckes. Nach ca. 7 - 10 Minuten tritt der Tod ein. Mit den beschriebenen PAF-Antagonisten können diese Effekte auf Atmung und Blutdruck sowie der Eintritt des Todes verhindert werden.

2.2. Antagonisierung der durch PAF bewirkten Blutdrucksenkung an der narkotisierten Ratte

Normotone, männliche, 200 - 250 g schwere Wistar-Ratten werden mit 2 mg/kg Urethan intraperitoneal anästhesiert. Die Arteria carotis und Vena jugularis werden kanüliert. Eine intravenöse PAF-Infusion (30 ng/(kg x min)) induziert bei den Kontrolltieren eine starke, anhaltende Blutdrucksenkung. Diese kann dosisabhängig durch intravenöse Injektionen (kumulative Verabreichung) der beschriebenen Verbindungen aufgehoben werden. Auch eine orale oder intravenöse Verabreichung der Verbindung vor Beginn der PAF-Infusion kann die blutdrucksenkende Wirkung der oben genannten PAF-Infusion dosisabhängig verhindern.

2.3. Antagonisierung der durch PAF induzierten Hautquaddel an der Ratte (Modifiziert nach P.P. Koelzer und K.H. Wehr, Arzneim.-Forsch. 8, 181 (1958)

Eine intrakutane Injektion von PAF induziert eine Hautquaddel, die Ausdruck einer durch PAF bedingten Erhöhung der Gefäßpermeabilität ist.

Männlichen Wistar-Ratten mit einem Körpergewicht von 250 ± 20 g wird die Bauchdecke (kurz) geschoren. Danach werden 1 ml/kg einer 1%igen Trypanblau-Lösung durch eine Schwanzvene den Tieren injiziert. Symmetrisch zur Mittellinie (linea alba) werden an drei Stellen in Abständen von ca. 1,5 cm intrakutane Injektionen von physiologischer Kochsalzlösung oder PAF-Lösung (12,5 bis 15,0 ng/Stelle in 0,1 ml) verabreicht. Während an der Injektionsstelle der Kochsalzlösung keine Reaktion auftrat, bewirkte PAF eine Hautreaktion (Quaddel), die durch eine Blaufärbung unterschiedlicher Intensität - abhängig von der PAF-Dosis - sichtbar wurde. Durch gleichzeitige intrakutane Verabreichung der beschriebenen Verbindungen oder durch eine intravenöse Vorbehandlung konnte diese durch PAF induzierte Hautreaktion verhindert werden.

3. Wirkungen auf das zentrale Nervensystem

Es ist allgemein bekannt, daß Substanzen dieses Strukturtyps zentralnervöse Effekte verursachen, die für eine Verbindung mit PAF-antagonistischer Wirkung nicht erwünscht sind. Daher wurden die beschriebenen Verbindungen hinsichtlich ihrer hypnogenen und antikonvulsiven Wirkung sowie ihres Einflusses auf die Lokomotion geprüft. Eine mögliche hypnotische Wirkung wurde an 400 bis 450 g schweren Meerschweinchen untersucht. Dosen bis zu 200 mg/kg p.o. dieser Substanzen waren nicht in der Lage, eine hypnotische oder sedative Wirkung an diesen Tieren zu erzeugen. Zur Prüfung einer antikonvulsiven Wirkung kann der Pentetrazol-Antagonismus bei Mäusen (20 bis 25 g Körpergewicht) verwendet werden (M. I. Gluckmann, Current Therapeutic Research, 7:721, 1965). Dosen bis zu 100 mg/kg p.o. dieser Verbindungen (1 Stunde vor Pentetrazol) zeigten in diesem Test keinen Einfluß auf die durch Pentetrazol (125 mg/kg i.p., LD 100) hervorgerufene Mortalität.

Die Wirkung auf die Nachtmotilität (Lokomotion) von Mäusen (20 - 25 g Körpergewicht) kann in einem Lichtschrankenkäfig untersucht werden. Dabei wird die Anzahl der Lichtstrahlunterbrechungen gemessen. Dosen bis zu 300 mg/kg p.o. der oben genannten Verbindungen zeigten keine Aktivität.

In der Tabelle A sind die in vitro Untersuchungen bezüglich der Hemmung der Blutplättchen aufgeführt, wie sie unten beschrieben sind.

Beispiel 1

4-(Morpholin-4-yl-carbonyl)-6-(2-chlorphenyl)-11-methyl-2,3,4,5-tetrahydro-8H-[1]benzothieno[3,2-f][1,2,4]-triazolo[4,3-a][1,4]diazepin

10 g (0.024 Mol) 4-Carboxyl-6-(2-chlorphenyl)-11-methyl-2,3,4,5-tetrahydro-8H-[1]benzothieno[3,2-f]-[1,2,4] triazolo[4,3-a]diazepin werden in 60 ml Methylenchlorid mit 4.2 g (0.026 Mol) Carbonyldiimidazol versetzt und 45 Minuten bei Raumtemperatur gerührt. Nach Zusatz von 2,2 g (0.025 Mol) Morpholin wird das Rühren 4 Stunden fortgesetzt. Anschließend wäscht man das Reaktionsgemisch mit gesättigter Natriumhydrogencarbonatlösung und mit Wasser, trocknet die organische Phase mit Natriumsulfat, dampft das Lösungsmittel ab und bringt den Rückstand mit Ether zur Kristallisation. Man erhält 9.6 g (83 %) farblose Kristalle, die sich aus Isopropanol umkristallisieren lassen.
Fp. 242 - 243 °C.

| $C_{24}H_{24}ClN_5O_2S$(482.0) | | | | | | |
|---|---|---|---|---|---|---|
| Ber.<br>Gef. | C = | 59.80,<br>59.63 | H | 5.02,<br>4.95 | N | 14.53<br>14.73 |

Das Ausgangsmaterial wird wie folgt erhalten:

a) 50 g (0.29 Mol) 3-Hydroxycyclohexancarbonsäureethylester, $Kp._{18}$: 135-140 °C (hergestellt durch Hydrierung von 3-Hydroxybenzoesäureester) werden in 600 ml Aceton bei 25-30 °C mit 92,5 ml Chromschwefelsäure (133 g Chromtrioxid, 115 ml konzentrierter Schwefelsäure, mit Wasser auf 500 ml verdünnt) versetzt und 30 Minuten bei 35 °C gerührt.

Man dekantiert die Lösung von dem dunkelgrünen Öl ab und extrahiert das Öl nochmals mit Aceton. Die Auszüge werden im Vakuum eingedampft, der Rückstand mit Methylenchlorid aufgenommen, die Lösung mit Wasser gewaschen, getrocknet und eingedampft. Man erhält 50 g 3-Cyclohexanoncarbonsäureethylester als hellgelbes Öl.

b) 50 g (0.29 Mol) der oben genannten Verbindung, 51 g (0.29 Mol) o-Chlorcyanoacetophenon, 9.3 g Schwefel und 130 ml Dimethylformamid werden gerührt und die Mischung bei 30-35 °C mit 25.9 ml Triethylamin versetzt. Man rührt 30 Minuten nach, destilliert das Dimethylformamid ab, nimmt den Rückstand in Essigester auf und wäscht mit Wasser. Nach dem Trocknen wird eingedampft und über $SiO_2$ chromatographiert, wobei ein Gemisch aus Methylenchlorid-Methanol (95: 5) zum Eluieren benutzt wird. Aus dem Eluat erhält man als Hauptfraktion 35-40 g gelbe Kristalle des 2-Amino-5-ethoxycarbonyl-3-(2-chlorbenzoyl)-4,5,6,7-tetrahydro-benzo[b]thiophens.
Fp. 136-137 °C.

c) 34 g (0.02 Mol) des Aminoketons, hergestellt nach Beispiel b), ergeben mit 8.5 ml Bromacetylbromid in 300 ml Dioxan in Gegenwart von 7,5 ml Pyridin 38 g der N-Bromacetylverbindung vom Fp. 105-107 °C; die in 650 ml wasserfreiem Essigester gelöst werden. Bei Raumtemperatur leitet man unter Rühren 2 Stunden Ammoniak ein, läßt über Nacht stehen. Man fügt 100 ml Eiswasser hinzu, trennt die organische Phase ab, trocknet und engt ein. Es hinterbleiben 30-32 g eines rötlichen Öls, das man zum Diazepinringschluß in 600 -700 ml Toluol aufnimmt. Man fügt 150 g $SiO_2$ hinzu und rührt das Gemisch 2 Stunden bei Rückflußtemperatur unter Verwendung eines Wasserabscheiders. Das Lösungsmittel wird abdekantiert und der Rückstand mehrmals mit Methanol in der Hitze extrahiert. Die methanolischen Auszüge werden eingedampft und der Rückstand aus Essigester umkristallisiert. Ausbeute: 23-25 g 7-Carbethoxy-5-(o-chlorphenyl)-6,7,8,9-tetrahydro-1H,3H-[1]benzothieno-[2,3-e]diazepin-2-on vom Fp.: 209-211 °C.

d) 36,2 g (0.97 Mol) des oben beschriebenen Diazepinons werden in 350 ml Diglyme aufgenommen, 15 g Natriumhydrogencarbonat und 30 g Phosphorpentasulfid hinzugefügt und 4 - 5 Stunden bei 75 - 8O °C gerührt. Nach dem Abkühlen auf Raumtemperatur fügt man allmählich 350 ml Wasser hinzu, wobei das Diazepinthion auskristallisiert. Die Kristalle werden abgesaugt, mit Wasser gewaschen, in Methylenchlorid gelöst, die Lösung getrocknet und eingedampft. Das Thion kristallisiert nach Etherzusatz. Ausbeute 30 - 32 g gelbe Kristalle vom Fp. 197 - 198 °C.

e) 14 g (0.03 Mol) des zuvor genannten Diazepinthions werden in 17O ml Tetrahydrofuran gelöst und mit 2.7 g Hydrazinhydrat versetzt. Man rührt 30 Minuten bei Raumtemperatur, destilliert das Lösungsmittel ab und vermischt den Rückstand mit Ether. Ausbeute: 13 g Kristalle vom Fp. 199 - 2OO °C; sie werden

in 100 ml Ethanol aufgenommen und nach Zusatz von 50 ml Orthoessigsäuretriethylester 1 Stunde unter Rückfluß gekocht. Man destilliert das Lösungsmittel ab und chromatographiert den Rückstand über $SiO_2$ (Laufmittel: Methylenchlorid/Methanol 98: 2). Der Rückstand der Hauptfraktion wird aus Essigester umkristallisiert.
Ausbeute: 11.8 g, (81 % d.Th.) farblose Kristalle des 4-(Ethoxycarbonyl)-6-(2-chlorphenyl)-11-methyl-2,3,4,5-tetrahydro-8H-[1]benzothieno[3,2-f][1,2,4]triazolo[4,3-a][1,4]-diazepin vom Fp.: 183 - 184 °C.
f) 15 g (0.036 Mol) des oben beschriebenen Esters werden mit 150 ml 2N ethanolischer Kalilauge 30 Minuten bei Raumtemperatur gerührt. Die Lösung wird anschließend im Vakuum eingedampft, der Rückstand in Wasser aufgenommen und unter Eiskühlung mit 2N Salzsäure auf pH 5-6 eingestellt. Die Carbonsäure wird mit Methylenchlorid extrahiert. Man erhält nach der Aufarbeitung Kristalle vom Fp. 312-315 °C.

Beispiel 2

3-(Morpholin-4-yl-carbonyl)-6-(2-chlorphenyl)-11-methyl-2,3,4,5-tetrahydro-8H-[1]benzothieno[3,2-f][1,2,4]-triazolo[4,3-a][1,4]diazepin

16 g (0.04 Mol) 3-Carboxy-6-(2-chlorphenyl)-11-methyl-2,3,4,5-tetrahydro-8H-[1]benzothieno[3,2-f]-[1,2,4]triazolo[4,3-a][1,4]diazepin und 5.3 g N-Hydroxybenztriazol werden in 15O ml Dimethylformamid suspendiert bzw. gelöst und mit 3.4 g (0.04 Mol) Morpholin versetzt. Unter Rühren und Eiskühlung fügt man 9.7 g (0.04 g Mol) Dicyclohexylcarbodiimid hinzu und rührt 20 - 24 Stunden bei 0-5 °C weiter. Der ausgefallene Dicyclohexylharnstoff wird abgesaugt und das Filtrat i.Vakuum eingedampft. Man löst den Rückstand in 100 ml 0.5N Salzsäure und saugt die ungelösten Bestandteile ab. Das Filtrat wird neutralisiert und mit Methylenchlorid extrahiert. Der Rückstand der Methylenchloridphase wird aus Essigester-Ether umkristallisiert und ergibt 15 -16 g der Titelverbindung vom Fp.253-255 °C.

| $C_{24}H_{24}ClN_5 0_2$ (482.0) | | | | | | |
|---|---|---|---|---|---|---|
| Ber. | C = | 59.80, 59.61, | H | 5.02, 5.17, | N | 14.53 14.07 |

Ausgehend von 4-Hydroxy-cyclohexancarbonsäureethylester ($Kp._{18}$: 142-145 °C) erhält man die als Ausgangsverbindung eingesetzte Carbonsäure nach dem in Beispiel 1 beschriebenen Herstellungsverfahren über die folgenden Zwischenprodukte:

| | |
|---|---|
| 2-Amino-3-(2-chlorbenzoyl)-6-ethoxycarbonyl-4,5,6,7-tetrahydrobenzo[b]thiophen | Fp.: 167-168 °C |
| Bromacetylderivat | Fp.: 133-135 °C |
| Aminoacetylderivat | Fp.: 145-146 °C |
| Diazepin-2-on | Fp.: 223-225 °C |
| 8-Carbethoxy-5-(2-chlorphenyl)-6,7,8,9-tetrahydro-3H-[1]benzothieno[2,3-e]diazepin-2-thion | Fp.: 212-214 °C |

Beispiel 3

4-(Morpholin-4-yl-carbonyl)-11-cyclopropyl-6-(2-chlorphenyl)-2,3,4,5-tetrahydro-8H-[1]benzothieno[3,2f]-[1,2,4]triazolo[4,3-a][1,4]diazepin

Ausgehend von 15 g (0.037 Mol) 4-Carboxy-11-cyclopropyl-6-(2-chlorphenyl)-2,3,4,5-tetrahydro-8H-[1]-benzothieno[3,2-f][1,2,4]triazolo[4,3-a][1,4]diazepin erhält man durch Umsetzung mit 32 g Morpholin in Dimethylformamid nach der in Beispiel 2 beschriebenen Methode 13.7 g der Titelverbindung als hellgelbes Pulver, das bei 175 °C zu sintern beginnt.

| $C_{26}H_{26}ClN_5O_2S$ (508.1) | | | | | | |
|---|---|---|---|---|---|---|
| Ber.<br>Gef. | C | 61.47,<br>61.17 | H | 5.53<br>5.18 | N | 13.78<br>13.65 |

Das Ausgangsmaterial wird wie folgt hergestellt:
9.14 g (0.022 Mol) 7-Carbethoxy-5-(2-chlorphenyl)-6,7,8,9-1H,3H-[1]benzothieno[2,3-e]diazepin-2-thion werden in 100 ml Dioxan gelöst oder suspendiert und nach Zugabe von 2,14 g Cyclopropancarbonsäurehydrazid 3 Stunden unter Rückfluß gekocht. Nach Abdampfen des Lösungsmittels chromatographiert man den Rückstand über eine mit $SiO_2$ gefüllte Säule und eluiert mit Methylenchlorid-Methanol (98:2). Man erhält 8.6 g eines zähen rötlichen Öls, das ohne weitere Reinigung mit 85 ml 2N ethanolischer Kalilauge zur Säure verseift wird.

Beispiel 3a

4-tert.-Butylaminocarbonyl-6-(2-chlorphenyl)-11-methyl-2,3,4,5-tetrahydro-8H-[1]benzothieno[3,2-f][1,2,4]-triazolo[4,3-a][1,4]diazepin

2,9 g (7mmol) der Carbonsäure aus Beispiel 1 f) werden in 40 ml wasserfreiem Dichlormethan mit 1,3 ml (18 mmol) Thionylchlorid versetzt und 30 Minuten bei Raumtemperatur gerührt. Die Suspension wird eingeengt und nach Zugabe von 25 ml (240 mmol) tert.-Butylamin 3 h unter Rückfluß erhitzt. Anschließend dampft man zur Trockne ein, nimmt mit Dichlormethan auf, extrahiert mit Wasser. Der Rückstand der organischen Phase wird an Kieselgel mit Dichlormethan/Methanol (95 : 5) chromatographiert und anschließend aus Ethanol/Ether umkristallisiert.
Ausbeute 1,2 g der Titelverbindung vom Fp.: 273 ° C

| | | | | | | | | | | |
|---|---|---|---|---|---|---|---|---|---|---|
| Ber.: | C | 61,59 | H | 5,60 | N | 14,96 | Cl | 7,58 | S | 6,85 |
| Gef.: | C | 61,49 | H | 5,83 | N | 14,74 | Cl | 7,49 | S | 6,74 |

Beispiel 4

3-(Morpholin-4-yl-carbonyl)-6-(2-chlorphenyl)-2,3,4,5-tetrahydro-8H-[1]benzothieno[3,2-f][1,2,4]triazolo[4,3-a]-[1,4]diazepin

Bei der Umsetzung von 8-Carbethoxy-5-(2-chlorphenyl)-6,7,8,9-tetrahydro-1H,3H-[1]benzothieno[2,3-e]-diazepin-2-thion mit Hydrazinhydrat und anschließend mit Orthoameisensäure-triethylester erhält man den entsprechenden ringgeschlossenen Ester (worin $R_1$ = Wasserstoff ist) und hieraus durch Verseifung die Carbonsäure und hieraus die Titelverbindung vom Fp.: 254-256 ° C.

Beispiel 5

11-Brom-3-(morpholin-4-yl-carbonyl)-6-(2-chlorphenyl)-2,3,4,5-tetrahydro-8H-[1]benzothieno[3,2-f][1,2,4]-triazolo[4,3-a][1,4]diazepin

6.1g (0.013 Mol) des Morpholids, hergestellt nach Beispiel 4, werden in 50 ml Chloroform gelöst bzw. suspendiert und mit 2,5 ml Pyridin versetzt. Man läßt 0.9 ml Brom, gelöst in 10 ml Chloroform, zutropfen und rührt über Nacht bei Raumtemperatur. Die Reaktionslösung wird mit Wasser gewaschen, getrocknet, eingeengt und über $SiO_2$ chromatographiert. Aus dem Rückstand des Eluats erhält man die gewünschte Bromverbindung vom Fp. 253 ° C.

Beispiel 6

11-Methoxy-3-(morpholin-4-yl-carbonyl)-6-(2-chlorphenyl)-2,3,4,5-tetrahydro-8H-[1]benzothieno[3,2-f][1,2,4]-triazolo[4,3-a][1,4]diazepin

2,4 g Bromverbindung, hergestellt nach Beispiel 5, werden mit 2,5 g Ätzkali (KOH) in 250 ml Methanol gelöst und 1 Stunde unter Rückfluß erhitzt. Das Methanol wird abdestilliert, der Rückstand mit Methylenchlorid aufgenommen und die Lösung mit Wasser gewaschen. Man trocknet, dampft ein und verreibt den Rückstand mit Ether.
Ausbeute: 1.4 - 1.6 g, Fp. 207 °C

| $C_{24}H_{24}ClN_5O_3S$ (498.O) | | | | | | |
|---|---|---|---|---|---|---|
| Ber.<br>Gef. | C<br>C | 57.88,<br>57,92 | H | 4.86,<br>4.81 | N | 14.06<br>14.01 |

Beispiel 7

3-(Morpholin-4-yl-carbonyl)-5-(2-chlorphenyl)-10-methyl-3,4-dihydro-2H,7H-cyclopenta[4,5]thieno[3,2-f]-[1,2,4]triazolo[4,3-a][1,4]diazepin

15 g (0.038 Mol) 3-Carboxy-5-(2-chlorphenyl)-10-methyl-3,4-dihydro-2H,7H-cyclopenta[4,5]thieno[3,2-f]-[1,2,4]triazolo[4,3-a][1,4]diazepin wurden mit 3,5 g Morpholin mittels Dicyclohexylcarbodiimid gemäß Beispiel 2 in das Amid überführt. Man erhält 15-16 g eines Pulvers vom Fp. 150 °C. Aus Ethanol umkristallisiert schmilzt die Verbindung bei 167-168 °C. Die Verbindung enthält 1-2% Kristallalkohol.
$^1$H-NMR ($CDCl_3$): δ 7.22 - 7.59 (m, 4H, Aryl-H); 4.94 (s, breit, 2H, $CH_2$-7-Ring); 2.06 - 4.00 (m, 13H, Cyclopentenyl-H, Morpholin-H); 2.67 (s, 3H, $CH_3$-Triazol-Ring).

Die eingesetzte Säure wird wie folgt hergestellt:

a) 41,6 g (0,27 Mol) Cyclopentanon-3-carbonsäureethylester (H.Stetzer und H. Kuhlmann, Liebigs Ann.Chem. 1979, 944; Kp. 70 - 74 °C), 47.8 g o-Chlorcyanoacetophenon und 9 g Schwefel werden in 120 ml Dimethylformamid suspendiert bzw. gelöst und mit 26.5 ml Triethylamin in 120 ml Ethanol versetzt , man erhitzt 30 - 45 Min. auf 30 - 60 °C und arbeitet analog Beispiel 1b auf. Nach dem Chromatographieren erhält man 29-3O g 2-Amino-3-(2-chlorbenzoyl)-5-ethoxycarbonyl-5,6-dihydro-4H-cyclopenta[b]-thiophen in Form gelber Kristalle vom Fp.: 121 - 122 °C.

b) Bromacetylierung, Umsetzung mit Ammoniak und Cyclisierung in Toluol in Gegenwart von $SiO_2$ gemäß Beispiel 1c ergeben das 7-Carbethoxy-5-(2-chlorphenyl)-7,8-dihydro-3H,6H-cyclopenta[4,5]thieno-[2,3-e]diazepin-2-on als farblose Kristalle vom Fp. 158-160 °C.

Das Diazepinon wird analog Beispiel 1d weiter umgesetzt. Man erhält den Ester der Triazoloverbindung vom Fp.: 175-176 °C und hieraus die entsprechende Carbonsäure durch Verseifung.

Isomerentrennung

Das obige Racemat (Beispiel 7, Fp. 167-168 °C) wird auf einer chiralen L-Polyamid-Kieselgel-(HPLC) Säule in seine Enantiomeren getrennt (Fließmittel: Dioxan/n-Hexan 7:3).

Man erhält das (-)-Enantiomere

$$[\alpha]_{436}^{20} - 26,5°$$

(c = 8.85, Dioxan) und das entsprechende (+)-Enantiomere

$$[\alpha]_{436}^{20} + 26.4$$

(c = 11,4, Dioxan)

Beispiel 7a

3-(n-Hexadecylamino-carbonyl)-5-(2-chlorphenyl)-10-methyl-3,4-dihydro-2H,7H-cyclopenta[4,5]thieno[3,2-f]-[1,2,4]triazolo[4,3-a][1,4]diazepin

6g(15mmol) der entsprechenden Carbonsäure (s. Bsp. 7) werden mit 4,5g (18mmol) Hexadecylamin, 4,4g Dicyclohexylcarbodiimid und 2,4g Hydroxybenztriazol nach der in Beispiel 2 beschriebenen Methode umgesetzt.

Man erhält 7g eines hellgelben Öls (75% d.Th.)
$^1$H-NMR ($CDCl_3$) $\delta$ 7.26 - 7.53 (4H, m, Aryl-H); 5.86 ($^1$H, t, J = 7Hz, NH); 4.86 (2H, s, breit, $CH_2$-7-Ring); 2.71 (3H, s, $CH_3$ C = N); 1.39 - 3.43 (7H, m, Cyclopentenyl-H, $NCH_2$); 1.26 (28H, s, Hexadecanyl); 0.88 (3H, t, J = 6Hz, 3H, $CH_3$-Hexadecanyl).

Beispiel 7b

3-(Dioctylamino-carbonyl)-5-(2-chlorphenyl)-10-methyl 3,4-dihydro-2H,7H-cyclopenta[4,5]thieno[3,2-f][1,2,4]-triazolo[4,3-a][1,4]diazepin

2g (5mmol) obige Carbonsäure (Beispiel 7) ergeben mit 1,5g (6mmol) Dioctylamin nach obiger Methode die Titelverbindung.
2g (65 d.Th.) zähes Öl,
$^1$H-NMR ($CDCl_3$): $\delta$ 7.33 - 7.55 (m, 4H, Aryl-H); 4.92 (s, breit, 2H, $CH_2$-7-Ring); 2.74 (s, 3H, $CH_3C=N$); 1.04 - 3.81 (m, 33H, Cyclopentyl H, Octylamino $CH_2$); 0.89 (t, J = 6Hz, $CH_3$ Octylamin).

Beispiel 7c

3-(Dipropylaminocarbonyl)-5-(2-chlorphenyl)-10-methyl-3,4-dihydro-2H,7H-cyclopenta[4,5]thieno[3,2-f][1,2,4]-triazolo[4,3-a][1,4]-diazepin

4.8 g (10 mmol) des Diallylamids (Beispiel 138) werden in 250 ml Methanol gelöst und nach Zugabe von 1 g Palladium-Kohle (5 %) unter leichtem Überdruck hydriert. Die Wasserstoffaufnahme ist quantitativ. Nach Filtrieren und Entfernung des Lösungsmittels hinterbleibt die Titelverbindung als weißer Schaum.
$^1$H NMR ($CDCl_3$): $\delta$ 7.22 - 7.58 (m, 4H, Aryl-H); 4.94 (s, breit, 2H, $CH_2$ 7-Ring; 2.69 (s, 3H, $CH_3$-C=N); 1.23 - 3.41 (m, 13H, cyclopentenyl-H, N-$CH_2CH_2$-$CH_3$; 0.82 (t, J = 7Hz, 6H, N-$CH_2$-$CH_2$-$CH_3$).

Beispiel 8

4-(Morpholin-4-yl-carbonyl)-5-(2-chlorphenyl)-10-methyl-3,4-dihydro-2H,7H-cyclopenta[4,5]thieno[3,2-f]-[1,2,4]triazolo-[4,3-a][1,4]-diazepin

20 g (0.05 Mol) 4-Carboxy-5-(2-chlorphenyl)-10-methyl-3,4-dihydro-2H,7H-cyclopenta[4,5]thieno[3,2-f]-[1,2,4]triazolo[4,3-a][1,4]diazepin vom Fp. 285 - 286 °C werden mit 4.6 g Morpholin nach der Dicylohexylcarbodiimid-Methode zum entsprechenden Amid umgesetzt. Man erhält nach der Aufarbeitung 13 g (55% d.Th.) der kristallinen Titelverbindung vom Fp.: 298-300 °C.
(Methylenchlorid/Ether)

| $C_{23}H_{22}ClN_5O_2$ (467,9) | | | | | | |
|---|---|---|---|---|---|---|
| Ber. | C | 59.03,<br>58,84 | H | 4.74,<br>4.73 | N | 14.97<br>14.69 |

Die als Ausgangsverbindung benutzte Carbonsäure wird analog Beispiel 7 aus dem käuflichen Cyclopentanon-2-carbonsäureethylester aufgebaut.

| | |
|---|---|
| 2-Amino-4-carbethoxy-3-(2-chlorbenzoyl)-5,6-dihydro-4H-cyclopenta[b]thiophen | Fp.: 122-124°C |
| Bromacetylverbindung | 148-150°C |
| Aminoacetylverbindung | 198-200°C |
| Diazepinon | 178-179°C |
| Ethyl-Ester des Triazolodiazepins | 285-286°C |

Beispiel 9

5-(Morpholin-4-yl-carbonyl)-7-(2-chlorphenyl)-12-methyl-3,4,5,6-tetrahydro-2H,9H-cyclohepta[4,5]thieno[3,2-f][1,2,4]triazolo[4,3-a][1,4]diazepin

0.75 g der entsprechenden Carbonsäure ergeben nach der im Beispiel 2 beschriebenen Dicyclohexylcarbodiimid-Methode mit 0.18 g Morpholin das gewünschte Amid vom Schmp. 290-291°C,

| $C_{25}H_{26}ClN_5O_2$ (496.0) | | | | | | |
|---|---|---|---|---|---|---|
| Ber. | C = | 60.53,<br>60.12, | H | 5.28<br>5.36 | N | 14.12<br>14.93 |

Die Carbonsäure kann ausgehend von 3-Ethoxycarbonylcycloheptanon (A. Mondau und G.Humman, Chem. Ber. 105, 1459 (1972)) analog der zuvor beschriebenen Methode in einer mehrstufigen Synthese hergestellt werden.
Fp. des 5-Carboethoxy-cyclohepta-thieno-triazolo-diazepins 187 - 188°C

Beispiel 10

4-(Morpholin-4-yl-carbonyl)-6-(2-chlorphenyl)-11-methyl-2,3,4,5-tetrahydro-8H-[1]benzothieno[3,2-f]imidazo-[1,2-a][1,4]diazepin

5 g (0.012 Mol) der entsprechenden Carbonsäure wurden, wie zuvor beschrieben, mit 1,2 g Morpholin nach der Dicyclohexylcarbodiimid-Methode umgesetzt und ergeben nach der Aufarbeitung 4.0 g des Morpholids vom Fp: 248-250°C.

| $C_{25}H_{25}ClN_4O_2S$(481.0) | | | | | |
|---|---|---|---|---|---|
| C | 62,42,<br>62,31 | H | 5.24,<br>5.36 | N. | 11.65<br>1O.99 |

Die Säure erhält man nach folgendem Verfahren:
25 g (o.O6 Mol) des Diazepinthions (hergestellt nach Beispiel 1 d), Fp. 197 - 198°C, wurden in 300 ml Dioxan bei Raumtemperatur mit 10 g Propargylamin versetzt und 2-3 Stunden unter Rückfluß gekocht. Man dampft ein, nimmt den Rückstand mit Methylenchlorid auf, schüttelt mit Wasser; trocknet die organische Phase und erhält nach Eindampfen und Verreiben mit Ether 18.7 g Kristalle vom Fp.: 179-181°C.

8 g (0.018 Mol) des oben hergestellten Aminoalkins werden zusammen mit 35 ml konz. Schwefelsäure 15 Minuten bei 100°C gerührt. Man gießt auf Eis, stellt mit NaOH auf pH 5 und extrahiert die entstandene Carbonsäure mit Methylenchlorid/Methanol (95 : 5). Aus der organischen Phase werden nach üblicher Aufarbeitung 5 - 6 g Carbonsäure erhalten, Fp.: 275 - 278°C.

Beispiel 10a

3-Carboxy-5-(2-chlorphenyl)-10-methyl-3,4-dihydro-2H,7H-cyclopenta[4,5]thieno[3.2-f]imidazo[1.2-a][1.4]-diazepin

0.4 g (0.84 mmol) 7-Carbomethoxy-5-(2-chlorphenyl)-2-(2-methyl-1,3-dioxolan-2-yl-methylamino)-7,8-dihydro-3H,6H-cyclopenta[4,5]thieno[3,2-f][1,4]diazepin werden in 4 ml konzentrierter Schwefelsäure 5 Minuten bei 50 °C erwärmt. Das Reaktionsgemisch wird nach dem Verdünnen mit Wasser unter Eiskühlung mit konzentrierter Natronlauge neutralisiert, die Lösung mit Methylenchlorid extrahiert und die organische Phase nach dem Waschen und Trocknen eingedampft. Man erhält als Rückstand 0.22 g (65 % Ausbeute) Säure.

$^1$H-NMR ($CD_3OD$): δ 7.40 - 7.61 (m, 4H, Aryl-H; 6.93 (qu, J <1Hz, 1H, CH=); 4.22 (s, breit, 2H, $CH_2$-7-Ring); 3.18 - 3.67 (m, 5H, $CH_2$, CH, 5-Ring); 2.48 (d, J < 1Hz, 3H, $CH_3$).

Synthese der zum Teil bekannten Vorstufen:

Aus Chloraceton und Phthalimidkalium erhält man in 93 % Ausbeute N-Acetonylphthalimid vom Fp. 103-105 °, das mit Ethylenglycol und konzentrierter Schwefelsäure in Toluol unter Verwendung eines Wasserabscheiders in 69 %iger Ausbeute zum N-Acetonylphthalimid-ethylenketal vom Fp. 91 - 93 °C ketalisiert wird. Die Phthalimidspaltung muß mit Hydrazinhydrat im molaren Verhältnis in Ethanol durchgeführt werden, wobei das erhaltene 2-Aminomethyl-2-methyl-dioxolan in ethanolischer Lösung anfällt und nach dem Abziehen des Solvens ohne weitere Reinigung mit dem 7-Carbomethoxy-5-(2-chlorphenyl)-7,8-dihydro-3H,6H-cyclopenta[4,5]-thieno[2,3-e][1,4]diazepin-2-thion, hergestellt analog Beispiel 7b und 1d), in Dioxan mit 45 % Ausbeute zum 7-Carbmethoxy-5-(2-chlorphenyl)-2-(2-methyl-1,3-dioxolan-2-yl-methylamino)-7,8-dihydro-3H,6H-cyclopenta[4,5]thieno[3,2-f][1,4]diazepin vom Fp. 166-168 °C umgesetzt wird.

Beispiel 10b

3-(n-Hexadecylamino-carbonyl)-5-(2-chlorphenyl)-10-methyl-3,4-dihydro-2H,7H-cyclopenta[4,5]thieno[3,2-f]-imidazo[1,2-a][1,4]diazepin

1,9g (4,8mmol) der entsprechenden Carbonsäure (Fp: 175 °C) (erhalten aus dem Diazepin-2-on, Beispiel 7, analog dem im Beispiel 10 beschriebenen Weg oder wie in Beispiel 10a angegeben), ergeben mit 1,5g (6mmol) n-Hexadecylamin 2g (69% d.Th.) der Titelverbindung als zähes Öl.

$^1$H-NMR ($CDCl_3$): δ 7.25 - 7.49 (m, 4H, Aryl-H); 6.89 (qu, J < 1Hz, 1H, CH=); 5.69 (t, J = 6Hz, 1H, NH); 4.17 (s, breit, 2H, $CH_2$-7-Ring). 3.05 - 3.37 (m, 7H, $CH_2$, CH, 5-Ring; $NCH_2$); 2.44 (d, J < 1Hz, 3H, $CH_3$-C=); 0.99 - 1.53 (m, 28H, N-$CH_2$-$(CH_2)_{14}$);
0.88 (t, J = 8Hz, 3H, $CH_3$-$(CH_2)_n$).

Beispiel 11

4-(Diethylaminocarbonyl)-6-(2-chlorphenyl)-11-methyl-2,3,4,5-tetrahydro-8H-[1]benzothieno][3,2-f]imidazo-[1,2-a][1,4]diazepin

Aus der entsprechenden Säure (Beispiel 10) erhält man analog durch Umsetzung mit Diethylamin das Amid vom Fp. 201-203 °C.

| $C_{25}H_{27}ClN_4OS$(467.2) | | | | | |
|---|---|---|---|---|---|
| C | 64.29<br>64.08 | H | 5.83<br>5.90 | N | 12.00<br>11.87 |

Beispiel 12

4-(Diethylaminocarbonyl)-6-(2-chlorphenyl)-10-methyl-2,3,4,5-tetrahydro-8H-[1]benzothieno[3,2-f]imidazo[1,2-a][1,4]diazepin

4.2 g (0.009 Mol) Diazepinthion (siehe Beispiel 1 d) werden mit 3 g 2-Aminopropionaldehyddiethylacetal und 0.75 g p-Toluolsulfonsäure in 80 ml Dioxan 3 Stunden unter Rückfluß gekocht. Man engt das Reaktionsgemisch ein, nimmt mit Methylenchlorid auf, wäscht mit Wasser, trocknet, engt erneut ein und gibt Isopropyläther hinzu, wobei die Zwischenverbindung kristallisiert. Ausbeute 2.7 g.

Dieses Derivat ergibt bei 15-minütigem Erhitzen in konzentrierter Schwefelsäure (100 °C) nach der Aufarbeitung die Carbonsäure. Hieraus wird, wie zuvor beschrieben, das Amid hergestellt.

Beispiel 12a

3-(Morpholin-4-yl-carbonyl)-5-(2-chlorphenyl)-9-methyl-10-brom-3,4-dihydro-2H,7H-cyclopenta[4,5]thieno-[3,2-f]imidazo[1,2-a][1,4]diazepin

1 g (2,1 mmol) 3-(Morpholin-4-yl-carbonyl)-5-(2-chlorphenyl)-9-methyl-3,4- dihydro-2H,7H-cyclopenta-[4,5]thieno[3,2-f]imidazo[1,2-a][1,4]diazepin werden in 30 ml wasserfreiem Trichlormethan und 0.22 g Pyridin vorgelegt und bei Raumtemperatur 0.4 g (2,5 mmol) Brom zugetropft. Nach 16 h wird zweimal mit Wasser gewaschen, die organische Phase mit $Na_2SO_4$ getrocknet und eingeeengt, der Rückstand über eine mit Kieselgel gefüllte Säule filtriert ($CH_2Cl_2$ mit ca. 4 % $CH_3OH$). Aus dem eingeengten Eluat erhält man durch Verreiben mit Ether 0,5 g Titelverbindung, die ab 135 °C sintert.

Beispiel 12 b

3-Carboxy-5-(2-chlorphenyl)-9-methyl-3,4-dihydro-2H,7H-cyclopenta[4,5]thieno[3,2-f]imidazo[1,2-a][1,4]-diazepin

7,3 g (14,5 mmol) 2-(1,1-diethoxy-prop-2-yl-amino)-5-(2-chlorphenyl)-7-methoxycarbonyl-7,8-dihydro-3H,6H-cyclopenta[4,5]thieno[2,3-e]diazepin werden in 35 ml konz. Schwefelsäure suspendiert und 15 Min. bei 100 °C gerührt. Anschließend wird das Reaktionsgemisch auf Eis gegeben und mit Ammoniumhydroxid alkalisch gestellt (pH 9) und dann mit 2N HCl auf pH 6 eingestellt. Es wird mit Dichlormethan (3 x 150 ml) extrahiert, die getrocknete organische Phase über Kieselgur/Kohle filtriert, dann eingeengt und der Rückstand mit wenig Ether verrieben. Man erhält 4,3 g der Titelverbindung vom Schmelzpunkt 160 - 162 °C.

Herstellung der Ausgangsverbindung:

10 g (25,6 mmol) des entsprechenden Diazepin-2-thions und 9,5 g (65 mmol) 2-Amino-propionaldehyd-diethylacetal werden in 100 ml Dioxan auf Rückflußtemperatur erhitzt. Nach zweistündiger Reaktion wird das Lösungsmittel abdestilliert, der Rückstand mit Dichlormethan/Wasser versetzt. Die abgetrennte, getrocknete Phase wird dann über Kieselgel/Kohle filtriert, eingeengt und der Rückstand über eine mit Kieselgel gefüllte Säule filtriert (Dichlormethan mit ca. 2 % $CH_3OH$). Das eingeengte Eluat wird mit Diisopropylether verrieben. Man erhält 7,4 g 2-(1,1-Diethoxy-prop-2-yl-amino)-5-(2-chlorphenyl)-7-methoxycarbonyl-7,8-dihydro-3H,6H-cyclopenta[4,5]thieno[2,3-e]diazepin als grau-braune Kristalle vom Schmp. 137 - 140 °C.

Beispiel 12 c

3-Carboxy-5-(2-chlorphenyl)-3,4-dihydro-2H,7H-cyclopenta[4,5]thieno[3,2-f]imidazo[1,2-a][1,4]diazepin

Ausgehend von 9,8 g (0.02 Mol) 2-(1,1-Diethoxyethylamino)- 5-(2-chlorphenyl)-7-methoxycarbonyl-7,8-dihydro-3H,6H-cyclopenta[4,5]thieno[2,3-e]diazepin erhält man in Analogie zu vorgeschriebenem Beispiel 7,5 g der Titelverbindung als graue Kristalle vom Schmelzpunkt >315 °C.

Herstellung der Ausgangsverbindung:

In Analogie zu Beispiel 12 aus 10 g (25,6 mmol) des entsprechenden Diazepinthions und 8,7 g (65 mmol) Aminoacetaldehyd-diethylacetal erhält man 9,8 g des erforderlichen Acetals als braune Kristalle vom Schmelzpunkt 124 bis 125 °C.

Beispiel 13

6-(2-Chlorphenyl)-11-methyl-4,5-dihydro-3H,8H-[1]benzothieno[3,2-f][1,2,4]triazolo[4,3-a][1,4]diazepin-2-on

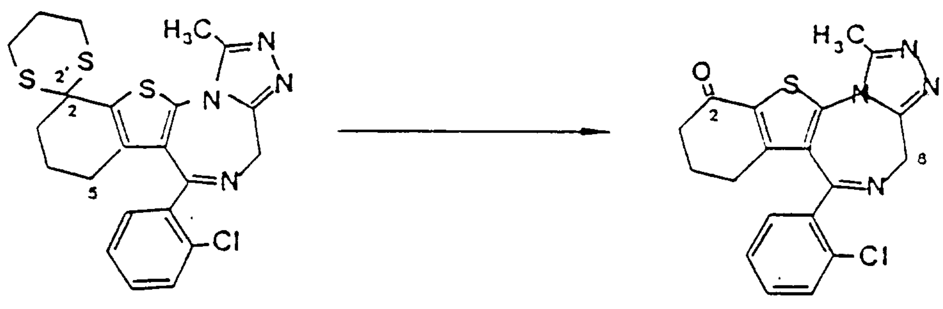

Eine Suspension von 8,5 g (O,O18 Mol) Spiro[6-(2-chlorphenyl)-11-methyl-2,3,4,5-tetrahydro-8H-[1]-benzothieno[3,2-f][1,2,4]triazolo[4,3-a][1,4]diazepin-2,2'(1,3)dithian], 9,77 g (0.036 Mol) Quecksilber(II)chlorid und 3.98 g (O.018 Mol) Quecksilber(II)oxid in 270 ml 90 %igem wäßrigem Methanol wird zwei Stunden unter Rühren zum Rückfluß erhitzt. Anschließend wird die Reaktionsmischung abfiltriert und der Rückstand mehrmals mit Chloroform gewaschen. Die Filtrate werden vereinigt und im Vakuum eingedampft. Man löst den Rückstand in Chloroform und schüttelt die erhaltene Lösung mehrmals mit verdünnter wäßriger Ammoniumchloridlösung aus. Nach dem Trocknen über wasserfreiem Natriumsulfat wird die organische Phase eingeengt. Der Rückstand wird durch chromatographische Aufarbeitung an Kieselgel mit Aceton als Eluierungsmittel gereinigt. Man erhält 4,5 g (65,4 % d.Th.) kristallines Keton vom Schmelzpunkt 212 - 218 ° C, das sich aus Acetonitril umkristallisieren läßt.
Fp. 219-222 ° C

| $C_{19}H_{15}ClN_4OS$ (382.88) | | | | | | | | | | |
|---|---|---|---|---|---|---|---|---|---|---|
| Ber.:<br>Gef.: | C | 59.60<br>59.47 | H | 3.95<br>3.97 | N | 14.63<br>14.66 | Cl | 9.26<br>9.20 | S | 8.37<br>8.23 |

Darstellung des Ausgangsmaterials:

a) 2-Acetylamino-3-(2-chlorbenzoyl)-5,6-dihydro-4H-benzo[b]thiophen-7-on

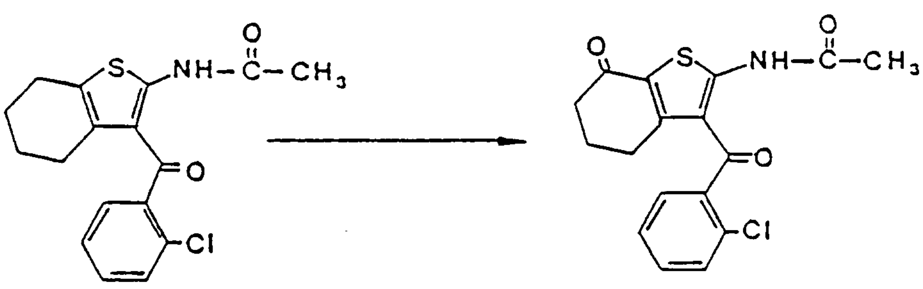

In eine Suspension von 13.35 g (0.04 Mol) 2-Acetylamino-3-(2-chlorbenzoyl)-5,6-dihydro-7H-benzo[b]-thiophen (Fp. 173 - 176 ° C) [hergestellt durch Umsetzung von (2-Amino-3-(2-chlorbenzoyl)-4,5,6,7-tetrahydro-benzo[b]-thiophen[1])mit Essigsäureanhydrid] tropft man unter Rühren in ca. 30 Minuten bei 20 - 30 ° (Eiskühlung) eine Lösung von 20 g (0.2 Mol) Chrom(VI)oxid in 60 ml Wasser. Die Reaktionsmischung wird noch zwei Stunden gerührt, mit 500 ml Wasser verdünnt und anschließend mehrmals mit

1) F.J. Tinney et al., J. Med. Chem. 17/6, 624 ff. (1974)

Essigester ausgeschüttelt. Die vereinigten Essigesterphasen werden nacheinander mit verdünnter wäßriger Ammoniak-Lösung und Wasser gewaschen, über Natriumsulfat getrocknet und eingeengt. Der Rückstand wird mit Ether suspendiert und abgesaugt. Man erhält 3.8 g (27,3 % d.Th.) Kristalle (Fp. 198 - 202 ° C), die aus Methanol umkristallisiert werden können.
Fp. 203 ° - 206 ° C.

| $C_{17}H_{14}ClNO_3S$ (347.83) | | | | | | | | |
|---|---|---|---|---|---|---|---|---|
| Ber.:<br>Gef.: | C | 58.70<br>58.71 | H | 4.06<br>4.13 | N | 4.03<br>4.12 | S | 9.22<br>9.13 |

b) 2-Amino-3-(2-chlorbenzoyl)-5,6-dihydro-4H-benzo[b]thiophen-7-on
26,5 g (0.076 Mol) 2-Acetylamino-3-(2-chlorbenzoyl)-5,6-dihydro-4H-benzo[b]thiophen-7-on werden unter Rühren in eine Lösung von 5.6 g (0.1 Mol) Kaliumhydroxyd in 15o ml Methanol eingetragen. Es entsteht hierbei eine Lösung, aus der nach kurzer Zeit das Aminoketon ausfällt. Nach zwei Stunden werden die Kristalle abgesaugt, mit Methanol gewaschen und getrocknet.
Ausbeute: 18,6 (79.8 % d.Th.); Fp.: 224-226 ° C
c) Spiro[2-amino-3-(2-chlorbenzoyl)-4,5,6,7-tetrahydrobenzo[b]thiophen-7,2'(1,3)dithian]

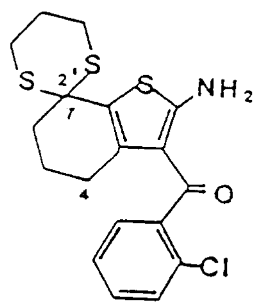

In eine Suspension von 18.5 g (0.06 Mol) des nach b erhaltenen Aminoketons und 6,55 g (0.06 Mol) 1,3-Propandithiol in 200 ml Chloroform wird sieben Stunden unter Eiskühlung Chlorwasserstoff eingeleitet. Die Reaktionsmischung wird über Nacht bei Raumtemperatur gerührt.Durch Zusatz von Methanol erhält man eine klare Lösung, die im Vakuum bis zur Trockne eingedampft wird. Der Rückstand wird mit Acetonitril suspendiert. Die anfallenden Kristalle werden säulenchromatographisch über Kieselgel mit Chloroform als Eluens gereinigt.
Ausbeute: 12,6 g (52,6 % d.Th.); Fp. 211 - 212 ° C. (Acetonitril).
d) Spiro[2-bromacetylamino-3-(2-chlorbenzoyl)-4,5,6,7-tetrahydro-benzo[b]thiophen-7,2'(1,3)dithian] erhält man analog Beispiel 14 b).
Fp. 162 - 164 ° C (Zersetzung).
e) Spiro[5-(2-chlorphenyl)-6,7,8,9-tetrahydro-1H,3H-[1H]-benzothieno[2,3-e]-[1,4]diazepin-2-on-9,2'(1,3)-dithian]

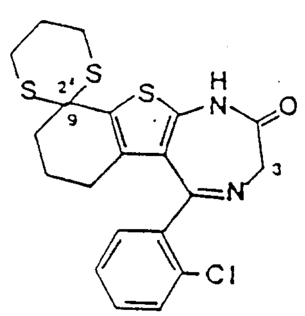

43.8 g (O.085 Mol) der nach d hergestellten N-Bromacetyl-Verbindung werden anlog Beispiel 14

umgesetzt. Man erhält 34,8 g (95,4 % d.Th.) des Diazepinons [Fp. 263 - 265 °C (Zersetzung)].

f) Spiro[6-(2-chlorphenyl)-11-methyl-2,3,4,5-tetrahydro-8H-[1]benzothieno[3,2-f][1,2,4]triazolo[4,3-a][1,4]-diazepin-2,2'(1,3)-dithian]

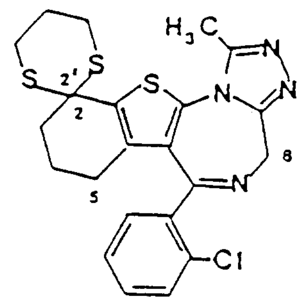

Fp. 267 - 270 °C. Die Verbindung wird ausgehend von dem unter e) beschriebenen Diazepinon analog Beispiel 14d und 14e erhalten.

Beispiel 14

6-(2-Chlorphenyl)-11-methyl-4,5-dihydro-2H,8H-[1]benzothieno[3,2-f][1,2,4]triazolo[4,3-a][1,4]diazepin-3-on

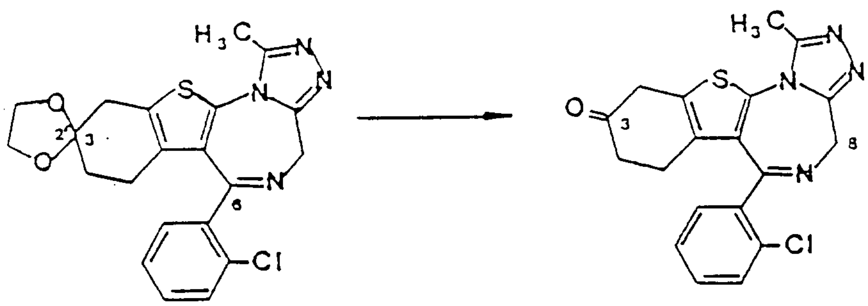

Man trägt 8.5 g (0.02 Mol) Spiro[6-(2-chlorphenyl)-11-methyl-2,3,4,5-tetrahydro-8H-[1]benzothieno[3,2-f]-[1,2,4]triazolo-[4,3-a][1,4]diazepin-3,2'(1,3)dioxolan] in 85 ml 4 normale Salzsäure ein und rührt die Reaktionsmischung 3O Minuten bei Raumtemperatur. Anschließend wird die Reaktionslösung mit Chloroform ausgeschüttelt. Die wäßrige Phase wird mit konzentrierter Ammoniaklösung alkalisch gestellt und mit Chloroform extrahiert. Die Chloroformlösung wird mit Wasser gewaschen, über Natriumsulfat getrocknet und im Vakuum eingeengt. Der Rückstand wird zur Reinigung an einer Kieselgelsäule chromatographiert (Elutionsmittel: Chloroform/Methanol 95 : 5). Man erhält 4.4 g (57.7 % d.Th.) des Ketons vom Schmelzpunkt 200 - 204 °C.

Die analysenreine Titelverbindung hat einen Schmelzpunkt von 202 °C - 204 °C (DMF/Ether).

| $C_{19}H_{15}ClN_4OS$ (382.82) | | | | | | | | | | |
|---|---|---|---|---|---|---|---|---|---|---|
| Ber.: | C | 59.60 | H | 3.95 | N | 14.63 | Cl | 9.26 | S | 8.37 |
| Gef.: | | 59.34 | | 3.71 | | 14.46 | | 9.11 | | 8.05 |

Das Ausgangsmaterial kann wie folgt erhalten werden:

a) Spiro[2-amino-3-(2-chlorbenzoyl)-4,5,6,7-tetrahydrobenzo[b]thiophen-6,2'(1,3)dioxolan]

Eine Lösung von 81.7 g (0.52 Mol) 1,4-Dioxaspiro[4,5]decan-8-on und 93,5 g (0.52 Mol) o-Chlor-2-cyano-acetophenon in 240 ml Dimethylformamid wird unter Rühren nacheinander mit 16.7 g Schwefel und 48 ml Triethylamin versetzt. Bei leicht exothermer Reaktion entsteht allmählich eine klare Lösung. Man rührt 30 Minuten nach, fügt 270 ml Alkohol zu und erhitzt die Reaktionslösung vier Stunden auf 60 °. Anschließend werden die Lösungsmittel im Vakuum abdstilliert. Das so erhaltene Rohprodukt wird mit

Essigester und verdünnter Kochsalzlösung verrührt. Die hierbei anfallenden Kristalle werden abgesaugt, mit Ether gewaschen und getrocknet.
Ausbeute 108 g (59 % d.Th.); Fp. 176 - 178 ° C.

Das analysenreine Aminoketon hat einen Schmelzpunkt von 179 - 182 ° C. (Essigester).

| $C_{17}H_{16}ClNO_3S$ (349.85) | | | | | | | | | | |
|---|---|---|---|---|---|---|---|---|---|---|
| Ber.:<br>Gef.: | C | 58.36<br>58.27 | H | 4.61<br>4.58 | N | 4.00<br>3.92 | Cl | 10.14<br>10.03 | S | 9.17<br>9.15 |

b) Spiro[2-(bromacetylamino)-3-(2-chlorbenzoyl)-4,5,6,7-tetrahydro-benzo[b]thiophen-6,2'(1,3)dioxolan]
171 g (0.49 Mol) des nach a erhaltenen Aminoketons werden in Dioxan unter Zusatz von 44 g Pyridin mit 110,4 g Bromacetylbromid umgesetzt. Man erhält 215.8 g (94 % d.Th.) der Bromacetyl-Verbindung vom Schmelzpunkt 155-157 ° C.

c) Spiro[5-(2-chlorphenyl)-6,7,8,9-tetrahydro-1H,3H-[1]benzothieno[2,3-e][1,4]diazepin-2-on-8,2'(1,3)-dioxolan]
Bei Raumtemperatur werden unter Rühren 70 g (0.15 Mol) der nach b erhaltenen N-Bromacetyl-Verbindung in 1200 ml mit Ammoniakgas gesättigtem Essigester eingetragen. Nach beendeter Zugabe leitet man noch zwei Stunden Ammoniakgas ein und läßt zwölf Stunden unter Rühren nachreagieren. Der anorganische Niederschlag wird abgesaugt und das Filtrat im Vakuum eingeengt. Zum Ringschluß nimmt man die rohe Aminoacetylverbindung mit 800 ml Toluol auf, fügt 240 g Kieselgel zu und erhitzt das Gemisch unter gutem Rühren vier Stunden am Wasserabscheider unter Rückfluß. Nach dem Abkühlen wird das Kieselgel abfiltriert und das entstandene Diazepinon mit heißem Methanol extrahiert. Der beim Einengen der vereinigten Auszüge erhaltene Rückstand wird in Essigester suspendiert, abgesaugt und gut mit Ether gewaschen.

Man erhält 45,8 g (79.2 % d.Th.) des Diazepinons [Fp. 262 - 263 ° C (Zersetzung)].

d) Spiro[5-(2-chlorphenyl)-6,7,8,9-tetrahydro-1H,3H-[1]benzothieno[2,3-e][1,4]diazepin-2-thion-8,2'-(1,3)-dioxolan]
Eine Suspension von 40.5 g (0.1 Mol) des nach c erhaltenen Diazepin-2-ons und 40.45 g (0.1 Mol) Lawesson-Reagens (Aldrich)$^R$ in 750 ml Tetrahydrofuran wird zwei Stunden unter Rückfluß erhitzt. Man destilliert das Lösungsmittel ab und reinigt das zurückbleibende, dunkle öl säulenchromatographisch über neutrales Aluminiumoxid/Aktivitätsstufe III, wobei zunächst Chloroform und dann ein Gemisch von Chloroform/Methanol (95 : 5) zum Eluieren benutzt wird. Das beim Einengen der Hauptfraktionen kristallin anfallende Diazepin-2-thion wird mit Ether suspendiert und abgesaugt.
Ausbeute: 28,5 g (68.4 % d.Th.); Fp. 203 - 210 ° C Zers. Die reine Titelverbindung schmilzt bei 213 - 214 ° C. Zers.(aus Toluol).

e) Spiro[6-(2-chlorphenyl)-11-methyl-2,3,4,5-tetrahydro-8H-[1]benzothieno[3,2-f][1,2,4]triazolo[4,3-a][1,4]-diazepin-3,2'(1,3)-dioxolan]
Eine Lösung von 11.8 g (0.029 Mol) des nach d erhaltenen Diazepin-2-thions in 120 ml Tetrahydrofuran wird mit 5.45 g (0.11 Mol) Hydrazinhydrat versetzt und eine Stunde bei Raumtemperatur gerührt. Das Lösungsmittel wird im Vakuum abdestilliert. Der zurückbleibende braune Sirup wird mit Chloroform aufgenommen, die Lösung mehrmals mit Wasser gewaschen, getrocknet und eingeengt.

Der Rückstand (10.2 g rohe Hydrazino-Verbindung) wird in 100 ml Ethanol suspendiert und nach Zusatz von 18 ml Orthoessigsäuretriäthylester 45 Minuten unter Rückfluß erhitzt. Die Reaktionslösung wird im Vakuum eingedampft. Der kristalline Rückstand wird mit wenig Essigester suspendiert und abgesaugt.
Ausbeute: 7.6 g (61 % d.Th.); Fp. 218-220 ° C. Die analysenreine Titelverbindung hat nach dem Umkristallisieren aus Essigester einen Schmelzpunkt von 224 - 225 ° C.

| $C_{21}H_{19}ClN_4O_2S$ (426.94) | | | | | | | | | | |
|---|---|---|---|---|---|---|---|---|---|---|
| Ber.:<br>Gef.: | C | 59.08<br>59.26 | H | 4.49<br>4.45 | N | 13.12<br>12.96 | Cl | 8.31<br>8.23 | S | 7.51<br>7.44 |

Beispiel 15

Spiro[2-amino-3-(2-chlorbenzoyl)-4,5,6,7-tetrahydro-benzo[b]thiophen-5,2'(1,3)dioxolan]

Ausgehend von 20.7 g (0.133 Mol) 1,4-Dioxaspiro[4,5]decan-7-on [M.W. Cronyn et al. J.A.C.S. 74, 3331 - 3333 (1952)], 23.69 g (0.133 Mol) o-Chlor-2-cyano-acetophenon, 4.29 g Schwefel und 12.2 ml Triethylamin erhält man analog Beispiel (14a) 34,2 g Rohprodukt, aus dem säulenchromatographisch ($SiO_2$/Chloroform) 2.8 g der Titelverbindung isoliert werden.
Fp. 216 - 218 °C.(Toluol).

| $C_{17}H_{16}ClNO_3S$ (349.85) | | | | | | | | | | |
|---|---|---|---|---|---|---|---|---|---|---|
| Ber.:<br>Gef.: | C | 58.36<br>58.66 | H | 4.61<br>4.60 | N | 4.00<br>3.86 | Cl | 10.14<br>9.96 | S | 9.17<br>9.20 |

Beispiel 16

2-Hydroxy-6-(2-chlorphenyl)-11-methyl-2,3,4,5-tetrahydro-8H-[1]benzothieno[3,2-f][1,2,4]triazolo[4,3-a][1,4]-diazepin

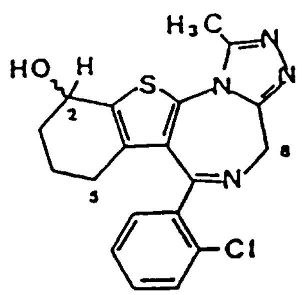

Eine Suspension von 0.38 g (0.001 Mol) 6-(2-Chlorphenyl)-11-methyl-4,5-dihydro-3H,8H-[1]benzothieno-[3,2-f][1,2,4]triazolo[4,3-a][1,4]diazepin-2-on in 3.8 ml Methanol wird unter Rühren mit 0.038 g (0.001 Mol) Natriumborhydrid versetzt. Die Reaktionslösung wird anschließend noch eine Stunde bei Raumtemperatur gerührt, mit wenig Wasser versetzt und im Vakuum eingeengt. Man nimmt den Rückstand mit Chloroform/Wasser auf und extrahiert die Chloroformlösung mehrmals mit Wasser. Die organische Phase wird über wasserfreiem Natriumsulfat getrocknet und das Lösungsmittel abdestilliert. Das zurückbleibende trübe Harz wird in Aceton gelöst und über eine kleine Kieselgelsäule filtriert. Der beim Einengen des Eluates erhaltene Rückstand wird mit Essigester zur Kristallisation gebracht.
Ausbeute: 170 mg (44.3 % d.Th.); Fp. 217 - 219 °C.

| $C_{19}H_{17}ClN_4OS$ (384.9) | | | | | | |
|---|---|---|---|---|---|---|
| Ber.:<br>Gef.: | C | 59.29<br>59.03 | H | 4.45<br>4.42 | N | 14.56<br>14.39 |

Beispiel 17

4-(Hydroxymethyl)-6-(2-chlorphenyl)-11-methyl-2,3,4,5-tetrahydro-8H-[1]benzothieno[3,2-f][1,2,4]triazolo[4,3-a][1,4]diazepin

10 g (0.0227 Mol) des Ethylesters aus Beispiel 1e werden in 100 ml wasserfreiem THF vorgelegt, portionsweise mit 0.52 g (0.0136 Mol) Lithiumaluminiumhydrid versetzt und 1 Stunde zum Rückfluß erhitzt. Man zersetzt das Reaktionsgemisch mit 0.5 ml Wasser, 0.5 ml 15 %iger Natronlauge und 1.5 ml Wasser,

saugt den Niederschlag ab, engt das Filtrat ein, nimmt den Rückstand in Methylenchlorid auf, wäscht die Lösung mit Wasser, trocknet und zieht das Solvens ab. Das Reaktionsgemisch wird an Kieselgel mit Methylenchlorid/Methanol 9 : 1 als Eluens chromatographiert und durch Verreiben mit Essigester oder Aceton zur Kristallisation gebracht. Man erhält 6.6 g (Ausbeute 73 %) der gewünschten Verbindung vom Fp.168-172°C.

Beispiel 18

3-(Hydroxymethyl)-5-(2-chlorphenyl)-3,4-dihydro-2H,7H-cyclopenta[4,5]thieno[3,2-f][1,2,4]triazolo[4,3-a][1,4]-diazepin

Analog Beispiel 1 wird Cyclopentan-3-on-carbonsäuremethylester mit $Kp_9$ = 95 - 98°C in 61 % Ausbeute umgesetzt zu 2-Amino-3-(2-chlorbenzoyl)-5-carbomethoxy-5,6-dihydro-4H-cyclopenta[b]thiophen vom Fp. 120 - 125°C.
Bromacetylierung und anschließende Umsetzung mit Ammoniak ergeben Öle in jeweils quantitativer Ausbeute.

Der Ringschluß zu 7-Carbomethoxy-5-(2-chlorphenyl)-6,7-dihydro-1H,3H,8H-cyclopenta[4,5]thieno[2,3-e][1,4]diazepin-2-on vom Fp.200 -205°C gelingt mit 78 % Ausbeute.

Das daraus auf üblichem Weg in 51 % Ausbeute erhältliche 3-Carbomethoxy-5-(2-chlorphenyl)3.4-dihydro-2H,7H-cyclopenta[4,5]thieno[3,2-f][1,2,4]triazolo[4,3-a][1,4]diazepin vom Fp. 178 - 181°C wird wie folgt reduziert.

4.5 g (0.O11 Mol) des Methylesters und 1 g (0.0275 Mol) Natriumborhydrid werden in 40 ml tert.-Butanol zum Rückfluß erhitzt und 10 ml wasserfreies Methanol zugetropft. Nach 60 Min. wird die Lösung eingedampft, der Rückstand in Methylenchlorid/Wasser verteilt, die organische Phase getrocknet und nach dem Abziehen des Solvens der Rückstand an Kieselgel mit Methylenchlorid/Methanol 98 : 2 bis 96 : 4 als Eluens chromatographiert. Durch Verreiben mit Ether erhält man in 76 % Ausbeute 2.6 g Kristalle vom Fp. 182 - 184°C.

Beispiel 19

3-Acetoxymethyl-5-(2-chlorphenyl)-10-methyl-3,4-dihydro-2H,7H-cyclopenta[4,5]thieno[3,2-f][1,2,4]triazolo-[4,3-a][1,4]diazepin

Zu 1.2 g (0.003 Mol) der Verbindung des Beispiels 18 und 0.42 ml (0.003 Mol) Triethylamin in 20 ml wasserfreiem Methylenchlorid tropft man bei Raumtemperatur 0.21 ml (0.003 Mol) Acetylchlorid zu, läßt 24 Stunden rühren, schüttelt mit Wasser aus, und zieht nach dem Trocknen das Solvens ab. Der Rückstand wird an Kieselgel mit Aceton/Methanol 9 : 1 als Eluens chromatographiert und ergibt 0.55 g eines gelben Öls (Ausbeute 42 %).

Beispiel 20

4-Methansulfonyloxymethyl-6-(2-chlorphenyl)-11-methyl-2,3,4, 5-tetrahydro-8H-[1]benzothieno[3,2-f][1,2,4]-triazolo[4,3-a][1,4]-diazepin

Zu 6.6 g (0.0165 Mol) der Verbindung des Beispiels 18 und 1.55 ml (0.02 Mol) Methansulfonsäurechlorid in 60 ml wasserfreiem Methylenchlorid werden bei 10°C 2.8 ml (0.02 Mol) Triethylamin zugetropft; das Reaktionsgemisch wird noch 2 Stunden bei Raumtemperatur weitergerührt und die organische Phase anschließend mit Wasser gewaschen und getrocknet. Nach dem Abziehen des Solvens wird der ölige Rückstand mit Essigester oder Ether verrieben und ergibt 7 g gelbliche Kristalle vom Fp. 250°C (Ausbeute 89 %).

Beispiel 21

4-(N-Morpholinomethyl)-6-(2-chlorphenyl)-11-methyl-2,3,4,5-tetrahydro-8H-[1]benzothieno[3,2-f][1,2,4]-triazolo[4,3-a][1,4]-diazepin

3.5 g (0.007 Mol) der Verbindung des Beispiels 20 werden in 50 ml Dioxan suspendiert, mit 3 g (0.035 Mol) Morpholin versetzt und 8 Stunden zum Rückfluß erhitzt. Von der entstandenen klaren Lösung wird das

Solvens abgezogen und der Rückstand zwischen 2 N Salzsäure und Methylenchlorid verteilt. Die wäßrige Phase schüttelt man mit Methylenchlorid aus und stellt dann alkalisch. Die ausfallende Base wird mit Methylenchlorid extrahiert, die organische Phase mit Wasser gewaschen und getrocknet. Nach dem Abziehen des Solvens chromatographiert man den Rückstand an Kieselgel mit Methylenchlorid/Methanol 9 : 1 als Eluens, kristallisiert den erhaltenen Feststoff aus Essigester und erhält 1.9 g weiße Kristalle vom Fp.189 190 °C.

Beispiel 21a

3-Morpholin-4-yl-methyl)-5-(2-chlorphenyl)-10-methyl-3.4-dihydro-2H,7H-cyclopenta[4.5]thieno[3.2-f][1,2,4]-triazolo[4.3-a][1.4]diazepin.

Ausgehend von der in Beispiel 7b) beschriebenen Verbindung 3-(Ethoxycarbonyl)-5-(2-chlorphenyl)-10-methyl-3.4-dihydro-2H,7H-cyclopenta[4.5]thieno[3.2-f][1,2,4]triazolo[4.3-a][1.4)diazepin wird mit Natriumborhydrid in tert.-Butanol mit Methanolaktivierung analog Beispiel 18 die 3-Hydroxymethylverbindung des Beispiels 65 mit Fp. 178-180 °C (aus Isopropylether/Essigester) in 81 % Ausbeute erhalten. Sie wird analog Beispiel 20 in quantitativer Ausbeute in die als Öl vorliegende 3-Methansulfonyloxymethylverbindung des Beispiels 67 umgewandelt. Analog Beispiel 21 läßt sich anschließend die 3-(Morpholin-4-yl-methyl)-Verbindung des Beispiels 66 in 79 % Ausbeute und mit einem Fp. von 179 - 181 °C (Acetonitril) erhalten. Diese Verbindung kann vollständig frei von organischen Solventien durch Lösen in Wasser bei pH 4.5 - 5, kurzes Abdestillieren der wäßrigen Lösung im Wasserstrahlvakuum am Rotavapor und anschließendem Ausfällen durch pH-Verschiebung auf pH 6.5 - 7 mit gesättigter Bicarbonatlösung erhalten werden. Fp. 136 - 138 °C (Wasser).

Beispiel 22

3-(N-Phthalimidomethyl)-5-(2-chlorphenyl)-10-methyl-3,4-dihydro-2H,7H-cyclopenta[4,5]thieno[3,2-f][1,2,4]-triazolo[4,3-a][1,4]diazepin

6 g (0.013 Mol) 3-Methansulfonyloxymethyl-5-(2-chlorphenyl)-10-methyl-3,4-dihydro-2H,7H-cyclopenta-[4,5]thieno[3,2-f][1,2,4]triazolo[4,3-a][1,4]-diazepin (hergestellt analog Beispiel 20) und 2.4 g (0.013 Mol) Phthalimid-Kalium werden in 60 ml wasserfreiem DMF (Dimethylformamid) 8 Stunden bei 80° gerührt und nach dem Abkühlen in 250 ml Wasser eingerührt. Nach extraktiver Aufarbeitung mit Methylenchlorid kann der Rückstand an Kieselgel mit Methylenchlorid/Methanol 9 : 1 als Eluens chromatographiert werden.

Beispiel 23

3-Aminomethyl-5-(2-chlorphenyl)-10-methyl-3,4-dihydro-2H,7H-cyclopenta[4,5]thieno[3,2-f][1,2,4]triazolo[4,3-a][1,4]diazepin

Eine Mischung aus 6.5 g (0.012 Mol) der Verbindung des Beispiels 22, 2.9 ml (0.06 Mol) Hydrazinhydrat und 100 ml Ethanol wird 6 Stunden bei Raumtemperatur gerührt, wobei eine Suspension entsteht. Man saugt ab, dampft das Filtrat ein, nimmt den Rückstand in 2 N Salzsäure auf und extrahiert mit Methylenchlorid. Die wäßrige Phase wird anschließend alkalisch gestellt und die Base mit Methylenchlorid extrahiert, die organische Phase getrocknet und das Solvens abgezogen. Der Rückstand wird an Kieselgel mit Methylenchlorid/Methanol/Ammoniak 85 :15 :1 als Eluens chromatographiert und ergibt 1.2 g (24 % Ausbeute) der Titelverbindung als amorphe Substanz.

Beispiel 23a

4-(Aminomethyl)-6-(2-chlorphenyl)-11-methyl-2,3,4,5-tetrahydro-8H-[1]benzothieno-[3,2f][1,2,4]triazolo[4,3-a]-[1,4]diazepin

4,3g (9mmol) Mesylat (Beispiel 20, Fp. 250 °C) werden in 40g Dimethylformamid nach Zugabe von 1 g Natriumazid 6 Stunden bei 80-100 °C gerührt. Das Lösungsmittel wird im Vakuum abdestilliert, der Rückstand in Methylenchlorid aufgenommen, mit Wasser gewaschen, getrocknet und eingedampft. Die Zugabe von Ether ergibt 3,4 g Kristalle des entsprechenden Azids vom Fp: 175-176 °C. 3.2g dieser Verbindungen werden in Gegenwart von Raney-Nickel in 60 ml Methanol hydriert. Man erhält 2.1 g farblose

Kristalle vom Fp: 115-118 °C Zers.

Beispiel 24

3-Acetylaminomethyl-5-(2-chlorphenyl)-10-methyl-3,4-dihydro-2H,7H-cyclopenta[4,5]thieno[3,2-f][1,2,4]-triazolo[4,3-a][1,4]diazepin

0.2 g (0.0005 Mol) der Verbindung des Beispiels 23 und 0.07 ml 0.0005 Mol Triethylamin werden in 10 ml wasserfreiem Methylenchlorid gelöst, mit 0.04 ml Acetylchlorid versetzt und 1 Stunde bei Raumtemperatur gerührt. Anschließend wird das Reaktionsgemisch mit Wasser gewaschen, getrocknet, das Solvens abgezogen und der Rückstand an Kieselgel mit Methylenchlorid/Methanol 9 : 1 als Fließmittel chromatographiert. Man erhält 0.1 g der Acetylaminoverbindung als amorphe Substanz in 45 %iger Ausbeute.

Beispiel 25

3-Thioacetyl-6-(2-chlorphenyl)-11-methyl-2,3,4,5,-tetrahydro-8H-pyrido-[4',3':4,5]thieno[3,2-f][1,2,4]triazolo-[4,3-a][1,4]diazepin

Ausgehend von Acetylpiperidon-4 erhält man analog Beispiel 1 in 62 % Ausbeute 2-Amino-3-(2-chlorbenzoyl)-6-acetyl-4,5,6,7-tetrahydro-thieno[2,3-c]pyridin und daraus in jeweils quantitativer Ausbeute die 2-Bromacetylamino- und 2-Aminoacetylaminoverbindung. Der Ringschluß zum 5-(2-Chlorphenyl)-8-acetyl-6,7,8,9-tetrahydro-1H,3H-pyrido[4',3':4,5]thieno[2,3-e][1,4]diazepin-2-on vom Fp. 248 - 250 °C gelingt mit 53 % Ausbeute.

Die Umsetzung mit Phosphorpentasulfid ergibt in 53 % Ausbeute 5-(2-Chlorphenyl)-8-thioacetyl-6,7,8,9-tetrahydro-1H,3H-pyrido[4',3':4,5]thieno[2,3-e][1,4]diazepin-2-thion (Massenspektrum m/e = 405/407), woraus nach der Hydrazinhydrat-Orthoessigsäuretriethylester-Methode in 30 % Ausbeute die gewünschte Verbindung mit einem Fp. 250 °C
(Massenspektrum m/e = 427/429) erhalten wird.

Beispiel 26

6-(2-Chlorphenyl)-11-methyl-2,3,4,5-tetrahydro-8-H-pyrido[4',3':4,5]thieno[3,2-f][1,2,4]triazolo[4,3-a][1,4]-diazepin

4.8 g (0.011 Mol) der Verbindung des Beispiels 25 und 100 ml 2 N methanolische Kalilauge werden 1 Stunde bei 60 °C gerührt, wobei eine klare Lösung entsteht. Nach dem Abziehen des Solvens wird der Rückstand zwischen Methylenchlorid und Wasser verteilt und die Methylenchlorid-Phase mit 2 N Salzsäure extrahiert. Anschließend wird die Salzsäure-Phase alkalisch gestellt und die ausgefallene Base mit Methylenchlorid extrahiert, die organische Phase mit Wasser gewaschen, getrocknet und das Solvens abgezogen. Der Rückstand wird an Kieselgel mit Methylenchlorid/Methanol 9 : 1 als Eluens chromatographiert. Man erhält 2.6 g (63 % Ausbeute) des Diazepins vom Fp. 84 - 86 °C.

Beispiel 27

3-(Carboethoxymethyl)-6-(2-chlorphenyl)-11-methyl-2,3,4,5,-tetrahydro-8-H-pyrido[4',3':4.5]thieno[3,2-f]-[1,2,4]triazolo[4,3-a][1,4]-diazepin

Zu 2,1 g (0.0057 Mol) der Verbindung des Beispiels 26 und 1 ml (0.007 Mol) Triethylamin in 20 ml wasserfreiem THF werden 1.15 g (0.007 Mol) Bromessigsäureethylester in 10 ml wasserfreiem THF bei Raumtemperatur zugetropft und 48 Stunden gerührt. Nach dem Filtrieren wird das Filtrat eingeengt und der verbleibende Rückstand zwischen Methylenchlorid und Wasser verteilt. Die organische Phase wird mit Wasser gewaschen, getrocknet und das Solvens abgezogen. Der Rückstand wird an Kieselgel mit Methylenchlorid/Methanol 9 : 1 als Eluens chromatographiert und ergibt 1.6 g (62 % Ausbeute) der gewünschten Verbindung als Öl.

Beispiel 28

4-Carboethoxy-6-(2-chlorphenyl)-11-methyl-2,3,4,5-tetrahydro-8H-[1]benzothieno[3,2-f]imidazo[1,2-a][1,4]-diazepin

Aus 2-Propargylamino-5-(2-chlorphenyl)-7-carboethoxy-6,7,8,9-tetrahydro-1H,3H-[1]benzothieno[2,3-e]-[1,4]diazepin, der Zwischenverbindung von Beispiel 10, bereitet man sich durch Lösen in Acetonitril und Zugabe von etherischer Salzsäure das Hydrochlorid (gelbe Kristalle mit Fp 240 °C).

13.7 g (0.029 Mol) dieses Hydrochlorids werden 1 Stunde bei 200 °C gerührt, abgekühlt und in Methylenchlorid aufgenommen, mit verdünntem Ammoniak und Wasser gewaschen und nach dem Trocknen das Solvens abgezogen. Der Rückstand wird an Kieselgel mit Methylenchlorid/Methanol als Eluens chromatographiert und ergibt 3.6 g (29 % Ausbeute) des Imidazodiazepins als amorphes Pulver.

Beispiel 28a

3-Carbomethoxy-5-(2-chlorphenyl)-10-methyl-3,4-dihydro-2H,7H-cyclopenta[4,5]thieno[3.2-f]imidazo[1.2-a]-[1.4]diazepin

0.25 g (0.5 mmol) 7-Carbomethoxy-5-(2-chlorphenyl)-2-(2-methyl-1,3-dioxolan-2-yl-amino)-7,8-dihydro-1H,3H,6H-cyclopenta[4,5]thieno[2,3-e][1,4]diazepin, dessen Herstellung in Beispiel 10a beschrieben ist, werden analog Beispiel 10a zur Imidazoverbindung cyclisiert. Bei Einsatz von konzentriertem Ammoniak anstelle von konzentrierter Natronlauge bei der Neutralisation läßt sich die Carbomethoxyfunktion in 7 Position erhalten und man isoliert in 70 % Ausbeute 0.15 g der Titelverbindung.
$^1$H-NMR ($CDCl_3$) $\delta$ 7.31 - 7.51 (m, 4H, Aryl-H); 6.93 (qu, J <1Hz, 1H, CH=); 4.15 (s, breit, 2H, $CH_2$-7-Ring); 3.69 (s, 3H, $OCH_3$); 3.08 - 3,61 (m, 5H, $CH_2$, CH, 5-Ring); 2.44 (d, J <1Hz, 3H, $CH_3$-C=):

Beispiel 28b

Die Verbindung 3-Carbomethoxy-5-(2-chlorphenyl)-10-methyl-3,4-dihydro-2H,7H-cyclopenta[4.5]thieno-[3.2-f]imidazo[1.2-a]-[1-4]diazepin, erhalten gemäß Beispiel 28a oder alternativ analog Beispiel 28, wird analog Beispiel 21a in 90% Ausbeute zur als helles Öl anfallenden 3-Hydroxymethylverbindung des Beispiels 158 reduziert und anschließend in 87 % Ausbeute zur als gelbes Öl anfallenden 3-Methansulfonyloxymethylverbindung des Beispiels 159 umgewandelt. Die weitere Umsetzung mit Morpholin ergibt die amorphe Verbindung des Beispiels 113 in 51 % Ausbeute.

Beispiel 29

N-{6-(2-Chlorphenyl)2,3,4,5-tetrahydro-8H-[1]benzothieno[3,2-f][1,2,4]triazolo[4,3-a][1,4]diazepin-4(R,S)-yl]-carbonyl}-S-leucinmorpholid

3 g (7.25 mmol) der in Beispiel 1 beschriebenen racemischen Cyclohexancarbonsäure werden in einer Mischung aus 55 ml Tetrahydrofuran und 20 ml Dimethylformamid mit 1.2 g (7.4 mmol) Carbonyldiimidazol 1 h bei Raumtemperatur gerührt. Anschließend fügt man 1.8 g (7.6 mmol) S-Leucinmorpholid-Hydrochlorid und 0,77 g (7.6 mmol) Triethylamin zu. Man rührt die Reaktionsmischung 3 Tage, engt im Vakuum ein, nimmt den Rückstand mit Dichlormethan auf und extrahiert mehrfach mit Wasser. Die organische Phase wird nach Trocknen eingedampft und der verbliebene Rückstand an Kieselgel RP 18 mit dem Laufmittelsystem Acetonitril: 0.01 molare Ammoniumcarbonatlösung : Diethylamin (35 : 65 : 0.1) chromatographisch in die beiden Diastereomeren aufgetrennt.

Die erste Fraktion wird nach Verdampfen der Lösungsmittel mit Ether kristallisiert.
Ausbeute 90 mg (2 %) vom Fp. 158 - 160 °C

| $C_{30}H_{55}ClN_6O_3S$ x $H_2O$ (613.18) | | | | | | |
|---|---|---|---|---|---|---|
| Ber. | C | 58.76 | H | 6.08 | N | 13.71 |
| Gef. | C | 58.27 | H | .582 | N | 13.46 |

Die zweite Fraktion wird ebenfalls aus Ether kristallisiert.
Ausbeute: 80 mg (2 %) vom Fp. 162-165 °C.

| $C_{30}H_{35}ClN_6O_3$ x o.5 $H_20$ (604.17) | | | | | | | | | | |
|---|---|---|---|---|---|---|---|---|---|---|
| Ber.:<br>Gef.: | C<br>C | 59.64<br>59.65 | H<br>H | 6.01<br>6.04 | N<br>N | 13.91<br>13.80 | Cl<br>Cl | 5.87<br>5.73 | S<br>S | 5.31<br>5.36 |

Beispiel 30

4-[4,4-Dimethyl-2-oxazolin-2-yl]-6(2-chlorphenyl)-11-methyl-2,3,4,5-tetrahydro-8H-[1]benzothieno[3,2-f]-[1,2,4]triazolo[4,3-a][1,4]diazepin

2.05 g (5 mmol) der Cyclohexancarbonsaure aus Beispiel 1 werden zusammen mit 0.45 g (5 mmol) 2-Amino-2-methyl-1-propanol, 1.52 g (15 mmol) Triethylamin und 3,1 g Tetrachlorkohlenstoff in einer Mischung aus 10 ml Pyridin : Acetonitril (1:1) vorgelegt. Innerhalb von 3 Stunden tropft man 3.9 g (15 mmol) Triphenylphosphin gelöst in 10 ml Pyridin/Acetonitril (1:1) bei Raumtemperatur zu. Zur Nachreaktion rührt man noch 12 Stunden. Die Suspension wird eingeengt. Der Rückstand wird zunächst mit Ether, dann mit Essigester suspendiert und jeweils abfiltriert. Die Filtrate werden vereinigt und zur Trockne eingedampft. Der Rückstand wird an Kieselgel mit Dichlormethan/Methanol (95/5) chromatographisch gereinigt. Die sauberen Fraktionen werden nach dem Einengen aus Ether kristallisiert.
Ausbeute: 0.9 g (39 %), Fp. 239 °C

| $C_{24}H_{24}ClN_5OS$ (466.01) | | | | | | | | | | |
|---|---|---|---|---|---|---|---|---|---|---|
| Ber.<br>Gef. | C<br>C | 61.85<br>61.76 | H<br>H | 5.19<br>5.31 | N<br>N | 15.03<br>14.75 | Cl<br>Cl | 7.61<br>7.60 | S<br>S | 6.88<br>6.86 |

Die im Titel genannte Verbindung ist auch auf folgendem Wege zugänglich.

1 g (2.1 mmol) des Carbonsäureamids 42 aus Tabelle 1, werden in 50 ml Dichlormethan mit 1 ml Thionylchlorid unter Eiskühlung versetzt. Nach 5 Stunden bei Raumtemperatur engt man die Suspension ein, versetzt den Rückstand mit Eiswasser, stellt alkalisch und extrahiert mit Dichlormethan. Aus der organischen Phase gewinnt man wie oben beschrieben 0.41 g (43 %) der Titelverbindung vom Fp. 239 °C.

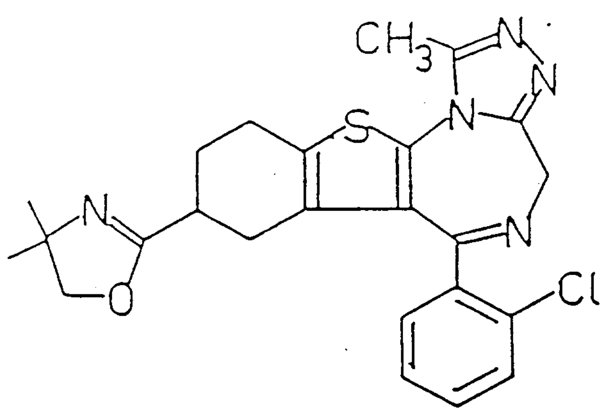

Beispiel 31

4-[4,4-Dimethyl-2-oxazolin-2-yl]6-(2-chlorphenyl)-11-methyl-2,3,4,5,6,7-hexahydro-8H-[1]benzothieno-[3,2-f]-[1,2,4]triazolo[4,3-a][1,4]diazepin

2 g (4.1 mmol) des primären Carbonsäureamids 42 aus Tabelle 1 werden in einer Mischung aus 12 ml Eisessig und 12 ml Dichlormethan durch Zugabe von 1 g Zinkstaub reduziert. Nach 3 Stunden Rühren bei Raumtemperatur wird abfiltriert, mit Dichlormethan gewaschen und die vereinigten Filtrate mit Ammoniaklösung alkalisch gestellt. Die organische Phase wird abgetrennt, getrocknet und eingeengt. Der Rückstand

wird an Kieselgel chromatographisch gereinigt (Laufmittel: Dichlormethan/Methanol (95/5). Die analysenreine Substanz wird aus Ether kristallin erhalten: 0.4 g vom Fp. 190 ° C.

Das so dargestellte Amid wird durch die in Beispiel 30 beschriebene Ringschlußreaktion mit Thionylchlorid in das im Titel genannte Oxazolin überführt.

Man erhält 70 mg vom Fp. 179 ° C.

Beispiel 32

4-Cyano-6-(2-chlorphenyl)-2,3,4,5-tetrahydro-11-methyl-8H-[1]benzothieno[3,2-f][1,2,4]triazolo[4,3-a][1,4]-diazepin

10.3 g (25 mmol) des primären Carbonsäureamids 44 aus Tabelle 1 suspendiert man in 150 ml Dichlorethan und 9 ml Phosphoroxychlorid und erhitzt 5 Stunden Stunden unter Rückfluß. Die Reaktionsmischung wird nach dem Abkühlen auf Eis gegossen, alkalisch gestellt und mit Dichlormethan extrahiert. Aus der organischen Phase gewinnt man nach chromatographischer Reinigung an Kieselgel mit Dichlormethan/Methanol (95/5) und Umkristallisieren aus Essigester/Ether 7.6 g (78 %) des Cyanids vom Fp. 212 ° C.

| $C_{20}H_{16}ClN_5S$ (393.89) | | | | | | | | |
|---|---|---|---|---|---|---|---|---|
| Ber.: | C | 60.98 | H | 4.09 | N | 17.78 | Cl | 9.00 |
| Gef.: | C | 60.93 | H | 4.07 | N | 17.71 | Cl | 9.16 |

Beispiel 33

4-[2-Imidazolin-2-yl]-6-(2-chlorphenyl)-11-methyl-2,3,4,5-tetrahydro- 8H-[1]benzothieno[3,2-f][1,2,4]triazolo-[4,3-a][1,4]diazepin

3.4 g (8.6 mmol) des in Beispiel 32 genannten Cyanids werden mit 8 ml einer 30%igen ethanolischen Salzsäure versetzt und 10 Tage im Kühlschrank stehen gelassen. Man filtriert das ausgefallene Imidoesterhydrochlorid ab. Das Rohprodukt wird sofort weiter umgesetzt. Man tropft unter Eiskühlung 10 ml Ethylendiamin zu und erhitzt 20 Stunden bei 80 ° C. Nach Abdampfen des überschüssigen Amins nimmt man mit Eiswasser auf, bringt auf pH 3, wäscht mit Dichlormethan und verwirft die organische Phase. Dann wird die wäßrige Phase stark alkalisch eingestellt und erneut mit Dichlormethan extrahiert. Letztere organische Phase wird zur Trockne gebracht und der Rückstand an basischem Aluminiumoxid mit Dichlormethan/Methanol (97/3) chromatographiert. Als erste Fraktion isoliert man die Titelverbindung. Durch Umkristallisieren aus Essigester/Ether erhält man 1,2 g (34 %) des Imidazolins vom Fp. 230 ° C

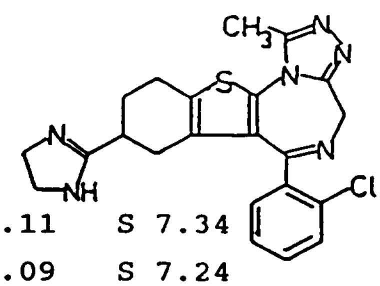

$C_{22}H_{21}ClN_6$ (436.96)

| | | | | | |
|---|---|---|---|---|---|
| Ber.: | C 60.47 | H 4.84 | N 19.23 | Cl 8.11 | S 7.34 |
| Gef.: | C 60.52 | H 4.80 | N 19.23 | Cl 8.09 | S 7.24 |

Als zweite Fraktion fällt das Säureamid 43 aus Tabelle 1 in einer Ausbeute von 1.1 g an.

Beispiel 33a

4-[4,4-Dimethylimidazolin-2-yl]-6-(2-chlorphenyl)-11-methyl-2,3,4,5- tetrahydro-8H-[1]benzothieno[3,2-f]-[1,2,4]triazolo[4,3-a][1,4]diazepin

17,6g (44,7 mmol) des Nitrils aus Beispiel 32 löst man in einer Mischungaus 100 ml Ether und 100 ml Ethanol, leitet durch diese Lösung 2 Stunden bei 0 ° C getrocknetes Salzsäuregas und läßt dann 2 Tage gut verschlossen stehen. Man engt das Lösungsmittel ein und gibt zum Imidoester-Rohprodukt 20g 1,2-Diamino-2-methylpropan und 30 ml Ethanol. Nach 3-stündigem Erhitzen bei 80°C arbeitet wie in Beispiel 33 angegeben auf.

Man gewinnt so 13,3g (64% d.Th.) der Titelverbindung vom Fp. 272°C (Ethanol Ether).

| | | | | | | | | | | |
|---|---|---|---|---|---|---|---|---|---|---|
| Ber.: | C | 61,99 | H | 5.42 | N | 18,07 | Cl | 7,62 | S | 6,90 |
| Gef.: | C | 61,66 | H | 5,46 | N | 18,00 | Cl | 7,56 | S | 6,55 |

Beispiel 34

4-[1-Methyl-2-imidazolin-2-yl]-6-(2-chlorphenyl)-11-methyl-2,3,4,5- tetrahydro-8H-[1]benzothieno[3,2-f][1,2,4]-triazolo[4,3-a][1,4]diazepin

0.65 g (1,5mmol) des im Beispiel 33 gewonnenen Imidazolins löst man in 40 ml Tetrahydrofuran unter Zusatz von 5 ml Dimethylformamid. Man gibt 0.08 g Natriumhydrid (80%ige Disperion in Öl) zu und rührt 30 Min bei Raumtemperatur. Sodann tropft man 0.1 ml (1.6 mmol) Methyljodid gelöst in 10 ml Tetrahydrofuran zu. Nach 24 Stunden wird eingeengt und der Rückstand wie oben beschrieben an basischem Aluminiumoxid chromatographiert.

Man gewinnt so 0.2 g (29 %) der N-Methyl-imidazolinverbindung vom Fp.: 195 - 197°C als Halbhydrat.

| $C_{23}H_{23}ClN_6S$ x 1/2 $H_20$ (460) | | | | | | | | | | |
|---|---|---|---|---|---|---|---|---|---|---|
| Ber.: | C | 60.05 | H | 5.26 | N | 18.27 | Cl | 7.71 | S | 6.97 |
| Gef.: | C | 60.30 | H | 5.11 | N | 18.25 | Cl | 7.78 | S | 7.08 |

Beispiel 34a

4-Amino-6-(2-chlorphenyl)-11-methyl-2,3,4,5-tetrahydro-8H-[1]benzothieno[3,2-f][1,2,4]triazolo[4,3-a][1,4]-diazepin

10,3 g (25 mmol) der Cyclohexancarbonsäure aus Beispiel 1 werden in 50 ml Aceton vorgelegt, bei O°C tropft man nacheinander 4,2 ml Triethylamin und 3,6 g Chlorameisensäureethylester zu. Man rührt 45 Minuten bei dieser Temperatur und fügt 1,75 g (27 mmol) Natriumazid, gelöst in 10 ml Wasser, tropfenweise zu. Anschließend läßt man 2 Stunden bei 10°C reagieren, zieht das Aceton zum großen Teil ab, versetzt mit Wasser und extrahiert mit Dichlormethan. Nach Trocknen der organischen Phase und Einengen im Vakuum erhält man 11,8 g Carbonsäureazid als amorphes Pulver.

1 g dieses Rohprodukts erhitzt man in 15 ml wasserfreiem Dioxan 3 Stunden unter Rückflußbedingungen, dann fügt man 50 ml 0,1 n HCl zu, erhitzt nochmals 3 Stunden, engt ein, bringt auf pH = 12 mit Natronlauge und läßt 2 Stunden stehen.

Dann extrahiert man das Amin mit Dichlormethan und chromatographiert nach Eindampfen der organischen Phase an basischem Aluminiumoxid mit Fließmittel:

Dichlormethan/Methanol (97:3).

Man isoliert 0,1 g des Amins als amorphe Substanz vom Fp. 180 - 185°C.

$^1$H-NMR ($CDCl_3$) $\delta$ 7,10-7,68 (4H, m, Aryl-H); 5,57 und 4,20 (2H, AB-System, $J_{AB}$ = 13 Hz, $CH_2$-7-Ring); 2,67 (3H, s, $CH_3$-Triazol-ring); 1,29 - 3,29 (9H, m, Cyclohexanring, $NH_2$).

Beispiel 34b

4-Methoxycarbonylamino-6-(2-chlorphenyl)-11-methyl-2,3,4,5-tetrahydro-8H-[1]benzothieno[3,2-f][1,2,4]-triazolo[4,3-a][1,4]diazepin

1,1 g (2,7 mmol) des im obigen Beispiel gewonnenen Isocyanats werden zusammen mit 20 ml (0,5 mol) Methanol und 20 ml Dichlormethan sowie 0,2 g (1,8 mmol) DABCO (1,4-Diazabicyclo(2,2,2)octan) 3 Tage bei Raumtemperatur gerührt. Man engt bis zur Trockne ein und reinigt chromatographisch an Kieselgel mit dem Laufmittelsystem Dichlormethan/Methanol 95 : 5. Die sauberen Fraktionen werden aus Ether umkristallisiert.

Ausbeute: 0,3 g (40%) vom Fp. 166-168 °C
$^1$H-NMR ($CDCl_3$) δ 7,3 (4H, m, Aryl-H), 5,57 und 4,2 (2H, AB-System, $J_{AB}$ = 13 Hz, $CH_2$-7-Ring), 3,6 (5,3 H, $CH_3O$-), 2,77 (m, 2H) und 1,77 - 2,22 (m, 5H, Cyclohexan-H), 2,66 (3H, s, $CH_3$-Triazol).
m/e = 441/443 ($M^+$), 409/411 ($M^+$-32($OCH_3$)), 366/368 ($M^+$-75 ($NH_2COOCH_3$)).

In den nachfolgenden Tabellen werden zusätzliche Abkürzungen verwendet
Me = Methyl, Et = Ethyl, i-Pr = iso-Propyl, t-Bu = tert.-Butyl.

Bei der in den Beispielen angegebenen Numerierung wurden die folgenden Strukturen zugrunde gelegt:

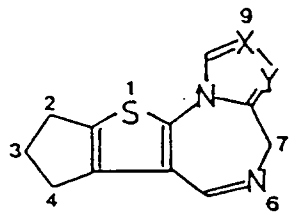

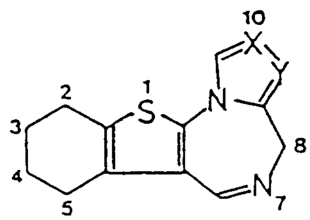

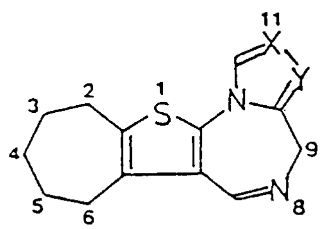

Nach den vorstehend beschriebenen Verfahren werden die folgenden Verbindungen zugänglich:

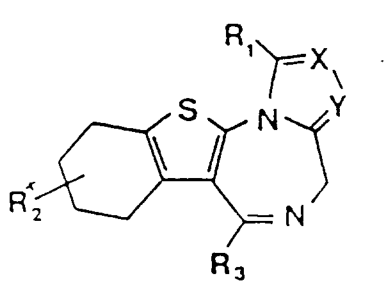

A

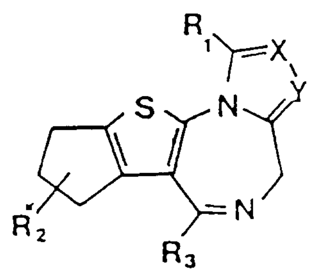

B

soweit nichts anderes angegeben, bedeuten X und Y beide Stickstoff und $R_3$ = o-Chlorphenyl.

| Bsp. | $R_2^*$ | $R_1$ | Type | Pos. $R_2^*$ | nach Bsp | Fp. |
|---|---|---|---|---|---|---|
| 35 | $-CO-N(C_2H_5)_2$ | $CH_3$ | A | 3 | 2 | 226-228°C |
| 36 | $-CO-N(C_2H_5)_2$ | $CH_3$ | A | 4 | 1 | 206-209°C |
| 37 | 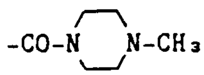 | $CH_3$ | A | 4 | 1 | 253-255°C |
| 38 | 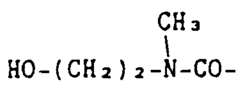 | $CH_3$ | A | 4 | 1 | 250-251°C |
| 39 | 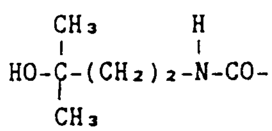 | $CH_3$ | A | 4 | 1 | 237-238°C |
| 40 | 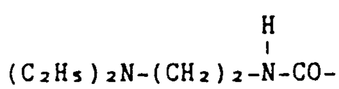 | $CH_3$ | A | 4 | 1 | Pulver |
| 41 | 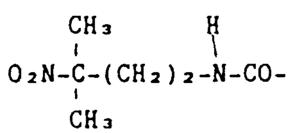 | $CH_3$ | A | 3 | 2 | Pulver |
| 42 | 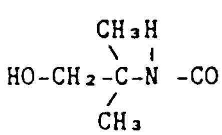 | $CH_3$ | A | 4 | 1 | 198°C |

| Bsp. | $R_2^x$ | $R_1$ | Type | Pos. $R_2^x$ | nach Bsp | Fp. |
|---|---|---|---|---|---|---|
| 43 | 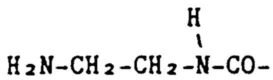 | $CH_3$ | A | 4 | 1 | 223-224°C |
| 44 | $H_2N-CO-$ | $CH_3$ | A | 4 | | 280°C |
| 45 | 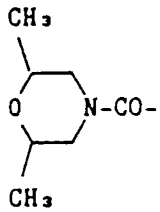 | $CH_3$ | A | 4 | 1 | 230-231°C |
| 46 | 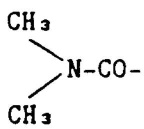 | $CH_3$ | B | 4 | 8 | 317-318°C |
| 47 | 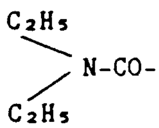 | $CH_3$ | B | 4 | 8 | 251-253°C |
| 48 | 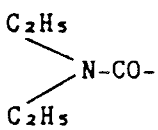 | $CH_3$ | B | 3 | 7 | Pulver |
| 49 | 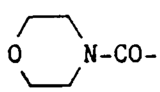 | $-OCH_3$ | B | 3 | 7/6 | Pulver |
| 50 | 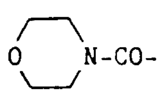 | $-OCH_3$ | B | 4 | 8/6 | 244-246°C |
| 51 | 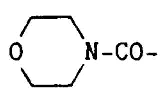 | $-OCH_3$ | A | 4 | 6 | 207-209°C |

| Bsp. | $R_2^x$ | $R_1$ | Type | Pos. $R_2^x$ | nach Bsp | Fp. |
|---|---|---|---|---|---|---|
| 52 | 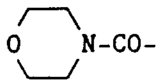 | H | A | 4 | 3 | 147-148°C |
| 53 | 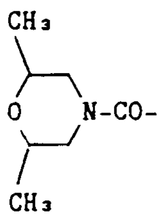 | $-OCH_3$ | B | 3 | 7/6 | Pulver |
| 54 | 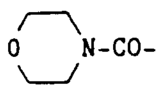 | H | B | 4 | | 270-271°C |
| 55 | 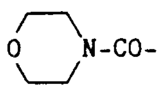 | Br | B | 4 | | 280-282°C |
| 56 | 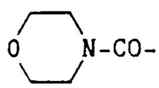 | H | B | 3 | | |
| 57 | 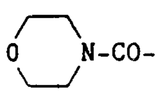 | Br | B | 3 | | 120°C Zers. |
| 58 | 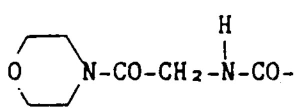 | $CH_3$ | B | 3 | 7 | Pulver |
| 59 | 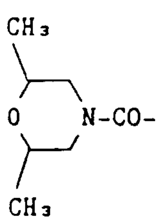 | $CH_3$ | B | 3 | | Pulver |
| 60 | 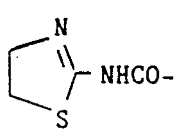 | $CH_3$ | A | 4 | 1 | 267-268°C |

| Bsp. | $R_2^x$ | $R_1$ | Type | Pos. $R_2^x$ | nach Bsp | Fp. |
|---|---|---|---|---|---|---|
| 61 | 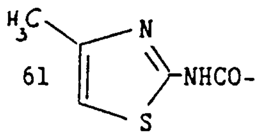 | $CH_3$ | A | 4 | 1 | 318°C |
| 62 | $-CH_2-O-C(=O)-CH_3$ | $CH_3$ | A | 4 | | 183-185°C |
| 63 | 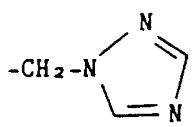 | $CH_3$ | A | 4 | | 82°C(Zers.) |
| 64 | $-CH_2-OH$ | $CH_3$ | X=CH A | 4 | | amorph |
| 65 | $-CH_2-OH$ | $CH_3$ | B | 3 | | 95°C(Zers.) |
| 66 | 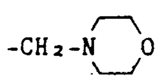 | $CH_3$ | B | 3 | | 177-178°C |
| 67 | $-CH_2-OSO_2-CH_3$ | $CH_3$ | B | 3 | | Öl |
| 68 | $-CH_2-O-C(=O)-CH_3$ | -H | B | 3 | | 161-162°C |
| 69 | $-CH_2-OSO_2CH_3$ | -H | B | 3 | | 163-165°C |

| Bsp. | $R_2^x$ | $R_1$ | Type | Pos. $R_2^x$ | nach Bsp | Fp. |
|---|---|---|---|---|---|---|
| 70 | 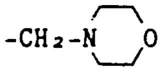 | -H | B | 3 | | 162-163°C |
| 71 | 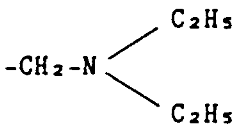 | -H | B | 3 | | 130-133 °C |
| 72 | 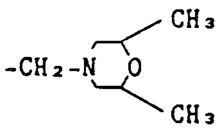 | $-CH_3$ | B | 3 | | 175-175° |
| 73 | 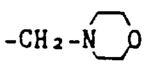 | -Br | B | 3 | | - |
| 74 | 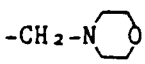 | $-OCH_3$ | B | 3 | | - |
| 75 | 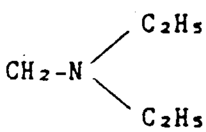 | Br | B | 3 | | - |
| 76 | 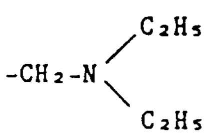 | $OCH_3$ | B | 3 | | - |
| 77 | 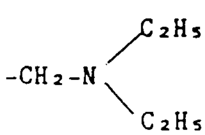 | $CH_3$ | B | 3 | | amorph |

| Bsp. | $R_2^*$ | $R_1$ | Type | Pos. $R_2^*$ | nach Bsp | Fp. |
|---|---|---|---|---|---|---|
| 78 | 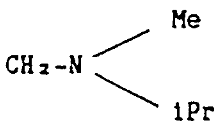 | $CH_3$ | B | 3 | | amorph |
| 79 | 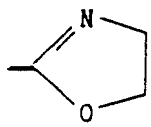 | $CH_3$ | A | 4 | | 213 |
| 80 * | 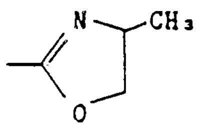 | $CH_3$ | A | 4 | | 212-213°C |
| 81* | 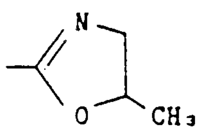 | $CH_3$ | A | 4 | | 225-226 |
| 82 | 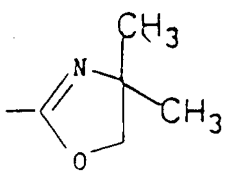 | $CH_3$ | B | 3 | | 228-229°C |
| 83 | 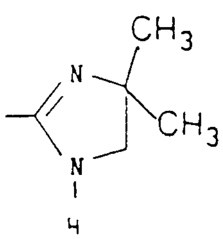 | $CH_3$ | A | 4 | | 272°C |
| 84 | 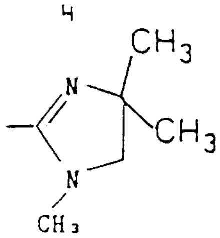 | $CH_3$ | A | 4 | | 215-217°C |

| Bsp. | $R_2$* | $R_1$ | Type | Pos. $R_2$* | nach Bsp | Fp. |
|---|---|---|---|---|---|---|
| 85 * | 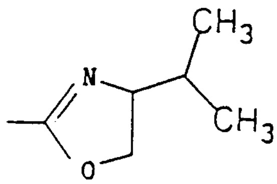 | $CH_3$ | A | 4 | | 197-200°C |

"Azaverbindungen"

86

3-Acetylaminomethylcarbonyl-6-(2-chlorphenyl)-11-methyl-2,3,4,5-tetrahydro-8H-pyrido-[4',3':4,5]-thieno[3,2-f]-[1,2,4]triazolo[4,3-a][1,4]diazepin Öl

87

3-(N-Morpholinocarbonylmethyl)-6-(2-chlorphenyl)-11-methyl-2,3,4,5-tetrahydro-8H-pyrido-[4',3':4,5]-thieno[3,2-f]-[1,2,4]triazolo[4,3-a][1,4]diazepin 125-128°C

* Diastereomerengemisch (50 : 50)

| Bsp. | $R_2$* | $R_1$ | Type | Pos. $R_2$* | nach Bsp | Fp. |
|---|---|---|---|---|---|---|
| 88 | 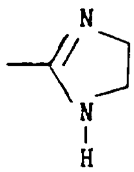 | $CH_3$ | A | 4 | 4 | 250-252°C |
| 89 | 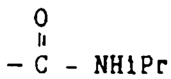 | $CH_3$ | A | 4 | 1 | 280°C |
| 90 | 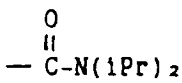 | $CH_3$ | A | 4 | 1 | 209°C |
| 91 | 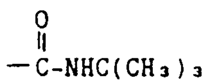 | $CH_3$ | A | 4 | 1 | 273°C |
| 92 | 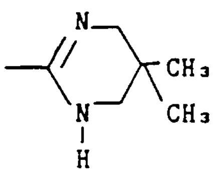 | $CH_3$ | A | 4 | 4 | 257°C |
| 93 | 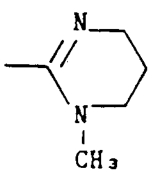 | $CH_3$ | A | 4 | 4 | 197°C |
| 94 | 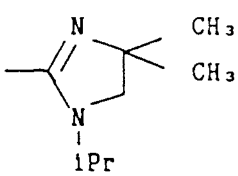 | $CH_3$ | A | 4 | 4 | 206°C |

| Bsp. | $R_2$* | $R_1$ | Type | Pos. $R_2$* | nach Bsp | Fp. |
|---|---|---|---|---|---|---|
| 95 | 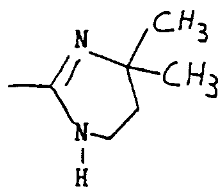 | $CH_3$ | A | 4 | 4 | 212-213°C |
| 96 | 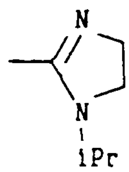 | $CH_3$ | A | 4 | 4 | 186°C |
| 97 | 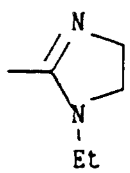 | $CH_3$ | A | 4 | 4 | 198-199°C |
| 98 | O H<br>‖ ǀ<br>$-C-N-(CH_2)_2-NH-Et$ | $CH_3$ | A | Neben-prod. bei 97 | | 166-167°C |
| 99 | 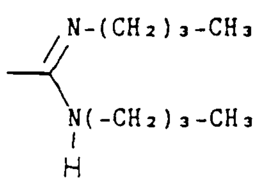 | $CH_3$ | A | 4 | 4 | amorphes Hydrat 80 - 85°C |
| 100 | 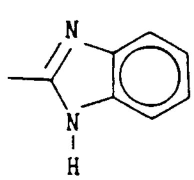 | $CH_3$ | A | 4 | 4 | 319°C |

| Bsp. | $R_2$* | $R_1$ | Type | Pos. $R_2$* | nach Bsp | Fp. |
|---|---|---|---|---|---|---|
| 101 | 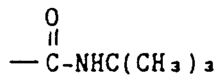 | $CH_3$ | A x=CH | 4 | | 278°C |
| 102 | 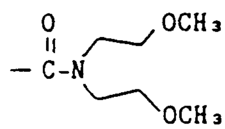 | $CH_3$ | A X=CH | 4 | 1 | amorph |
| 103 | 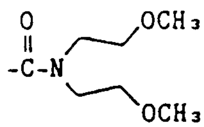 | $CH_3$ | A | 4 | 1 | amorph |
| 104 | 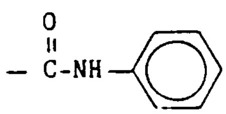 | $CH_3$ | A | 4 | | 270°C |
| 105 | $-CH_2-NH-Et$ | $CH_3$ | B | 3 | | Öl |
| 106 | 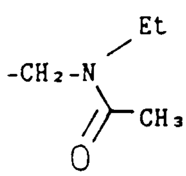 | $CH_3$ | B | 3 | | amorph |
| 107 | $-CH_2-NH-CH_2-CH_2-NMe_2 \times 2HCl$ | $CH_3$ | B | 3 | | amorph |
| 108 | 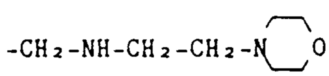 | $CH_3$ | B | 3 | | Öl |

| Bsp. | $R_2$* | $R_1$ | Type | Pos. $R_2$* | nach Bsp | Fp. |
|---|---|---|---|---|---|---|
| 109 | 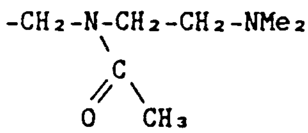 | $CH_3$ | B | 3 | | amorph |
| 110 | 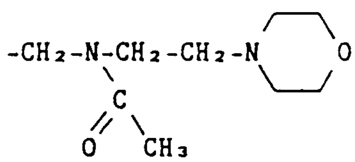 | $CH_3$ | B | 3 | | amorph |
| 111 | 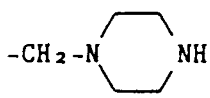 | $CH_3$ | B | 3 | | Öl |
| 112 | 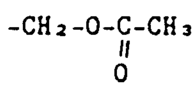 | $CH_3$ | B X=CH | 3 | | Öl |
| 113 | 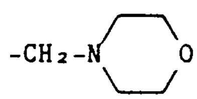 | $CH_3$ | B X=CH | 3 | | amorph |
| 114 | 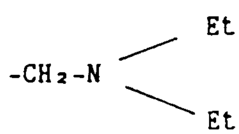 | $CH_3$ | B X=CH | 3 | | Öl |
| 115 | $-CH_2-NH-(CH_2)_2$ 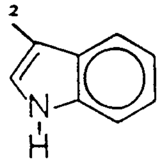 | $CH_3$ | B | 3 | | amorph |
| 116 | $-CH_2-OCH_3$ | $CH_3$ | B | 3 | | |

| Bsp. | $R_2$* | $R_1$ | Type | Pos. $R_2$* | nach Bsp | Fp. |
|---|---|---|---|---|---|---|
| 117 | $-CH_2-O-$ 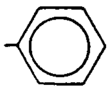 | $CH_3$ | B | 3 | | Öl |
| 118 | $-COOCH_3$  $R_3=C_6H_5$ | $CH_3$ | B | 3 | | 180°C |
| 119 | $-CH_2-OH$  $R_3=C_6H_5$ | $CH_3$ | B | 3 | | 235-236°C |
| 120 | $-CH_2-$ 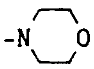 $R_3=C_6H_5$ | $CH_3$ | B | 3 | | 205-207°C |
| 121 | $-CH_2-O$ 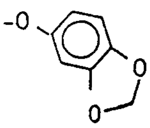 | $CH_3$ | B | 3 | | 206-207°C |
| 122 | $-CH_2-O$ 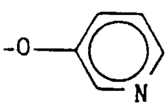 | $CH_3$ | B | 3 | | 200-205°C |
| 123 | $-CH_2-$ 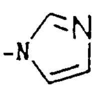 | $CH_3$ | B | 3 | | 222-225°C |
| 124 | $-CH_2-$ 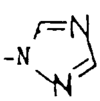 | $CH_3$ | B | 3 | | 205-210°C |
| 125 | $-CH_2-S-$ 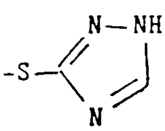 | $CH_3$ | B | 3 | | Öl |
| 126 | $-CH_2-Br$ | $CH_3$ | B | 3 | | Öl |
| 127 | $-CH_2-CN$ | $CH_3$ | B | 3 | | 227-230° |
| 128 | $-OH$ | $CH_3$ | A | 3 | 16 | 144-146°C |

| Bsp. | $R_2$* | $R_1$ | Type | Pos. $R_2$* | nach Bsp | Fp. |
|---|---|---|---|---|---|---|
| 129 | $-CH_2O-\underset{\underset{O}{\|}}{C}-CH_3$ | $CH_3$ | A | 3 | | 179-182°C |
| 130 | $-CH_2-$ 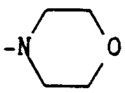 | $CH_3$ | A | 3 | | Öl |
| 131 | $-CH_2-OH$ | $CH_3$ | A | 3 | | amorph |
| 132 | $-CON(C_3H_7)_2$ | $CH_3$ | A | 4 | | 224-226° |
| 133 | $-CON(CH_2-CH=CH_2)_2$ | $CH_3$ | A X=CH | 4 | | Öl |
| 134 | $-CON(C_3H_7)_2$ | $CH_3$ | A X=CH | 4 | | Öl |
| 135 | $-CON(CH_2-CH=CH_2)_2$ | $CH_3$ | A | 4 | | Öl |
| 136 | -CO 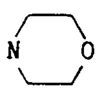 | $CH_2Cl$ | A | 4 | | 240° |
| 137 | -CO 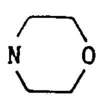 | $CH_2OH$ | A | 4 | | 242° |
| 138 | $-CO\ N(CH_2-CH=CH_2)_2$ | $CH_3$ | B | 3 | | amorph |

| Bsp. | $R_2$* | $R_1$ | Type | Pos. $R_2$* | nach Bsp | Fp. |
|---|---|---|---|---|---|---|
| 139 | $-CON[(CH_2)_7CH_3]_2$ | $CH_3$ | B | 3 | | Öl |
| 140 | $-CON(C_3H_7)_2$ | $CH_3$ | B | 3 | | Öl |
| 141 | $-CON(C_3H_7)_2$ | $CH_3$ | B X=CH | 3 | | Öl |
| 142 | $-CON(CH_2-CH=CH_2)_2$ | $CH_3$ | B X=CH | 3 | | Öl |
| 143 | -CO- 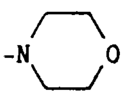 | $CH_3$ | B X=CH | 3 | | ab 95 zers |
| 144 | -CO- 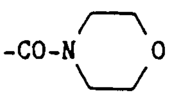 | Br | B X=CH | 3 | | ab 135 zers |
| 145 | -CO- 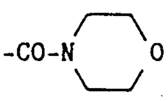 | H | B X= C-$CH_3$ | 3 | | ab 85 zers |
| 146 | -CO- 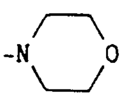 | $CH_3$ | B X=CH | 3 | | 130-131° |
| 147 | $-CO\ NH-(CH_2)_{15}-CH_3$ | $CH_3$ | B | 3 | | Öl |

| Bsp. | $R_2$* | $R_1$ | Type | Pos. $R_2$* | nach Bsp Fp. |
|---|---|---|---|---|---|
| 148 | $-CH_2-COOH$ | $CH_3$ | B | 3 | 233-234°C |
| 149 | $-CH_2-$ 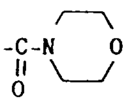 | $CH_3$ | B | 3 | amorph |
| 150 | $-CH_2-CH_2-OH$ | $CH_3$ | B | 3 | |
| 151 | $-CH_2-CH_2-O-SO_2CH_3$ | $CH_3$ | B | 3 | |
| 152 | $-CH_2-CH_2-$ 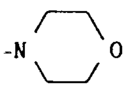 | $CH_3$ | B | 3 | |
| 153 | $-CH_2-CH_2-$ 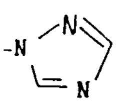 | $CH_3$ | B | 3 | |
| 154 | $-CH_2-COOMe$ | $CH_3$ | B | 3 | |
| 155 | $-CH_2-NH-n-C_{18}H_{37}$ | $CH_3$ | B | 3 | |
| 156 | $-CH_2-O-C(=O)-n-C_{17}H_{35}$ | $CH_3$ | B | 3 | Öl |
| 157 | $-CH_2-O-n-C_{18}H_{37}$ | $CH_3$ | B | 3 | |
| 158 | $CH_2-OH$ | $CH_3$ | B X=CH | 3 | helles Öl |
| 159 | $-CH_2-O-SO_2CH_3$ | $CH_3$ | B X=CH | 3 | gelbes Öl |
| 160 | 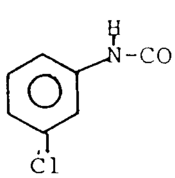 | CH3 | B | 3 | 285°C |

Tabelle II

Zwischenverbindungen der allgemeinen Formel

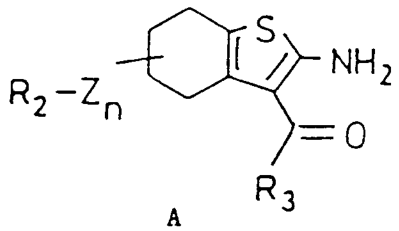

A

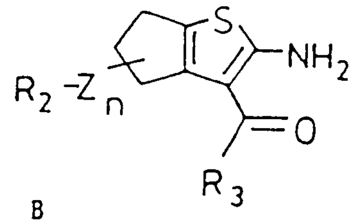

B

| Nr. | Zn | $R_2$ | $R_3$ | Typ | Pos. | Fp°C |
|---|---|---|---|---|---|---|
| 1. | - | $-COOCH_3$ | Cl | B | 5 | 121-122° |
| 2. | $-CH_2-$ | -OH | Cl | B | 5 | amorph |
| | | | | aus 1 mit | | $LiAlH_4$ |
| 3. | $-CH_2-$ | $-OSO_2-CH_3$ | Cl | B | 5 | Öl |
| 4. | $-CH_2-$ | 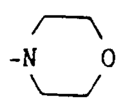 | Cl | B | 5 | 184-186° |
| 5. | - | $-COOCH_3$ | O | B | 5 | 133-135° |
| 6. | - | $-COOCH_3$ | Cl | A | 5 | 136-137° |
| 7. | - | $-COOCH_3$ | Cl | A | 6 | 167-168° |
| 8. | - | 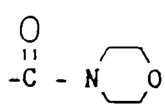 | Cl | A | 5 | |
| 9. | | 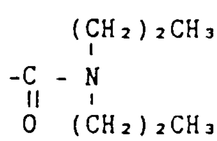 | Cl | B | 5 | |

In der nachfolgenden Tabelle sind die $^1$H-NMR-Spektren einiger Verbindungen aufgelistet.

Tabelle III: $^{1}$H-NMR-Spektren:

Beispiel 19

$^{1}$H-NMR ($CDCl_3$): δ 7.17 - 7.56 (m, 4H-Aryl); 4.94 (s, breit, $CH_2$-7-Ring); 3.98/d, J = 6Hz, 2H, $OCH_2$); 1.93-3.33 (m, 5H, 5-Ring); 2.70 (s, 3H, $CH_3$-Triazol); 2.02 (s, 3H, $CH_3C=O$).

Beispiel 20

$^{1}$H-NMR ($CDCl_3$): δ 7.18 - 7.62 (m, 4H, Aryl-H); 5.59, 4.22 (AB-System, $J_{AB}$ = 13Hz, 2H, $CH_2$-7-Ring); 3.98 (d, J = 5Hz, 2H, $CH_2$); 2.92 (S, 3H, $CH_3$-$SO_2$); 2.69 (s, 3H, $CH_3$-Triazol); 1.31 - 3.00 (m, 7H, $CH_2$, CH, 6-Ring)

Beispiel 22

$^{1}$H-NMR ($CDCl_3$): δ 7.62 - 7.91 (m, 4H, Aryl-phthalimid); 7.18 - 7.56 (m, 4H, Aryl-H); 4.92 (breit, 2H, $CH_2$-7-Ring); 4.13 (d, J = 6Hz, 2H, $NCH_2$); 2.69 (s, 3H, $CH_3$ Triazolring); 1.60 - 3.33 (m, 5H, $CH_2$, CH, 5-Ring).

Beispiel 23

1H-NMR ($CDCl_3$): δ 7.20 - 7.58 (m, 4H, Aryl-H); 4.92 (s, breit, 2H, $CH_2$-7-Ring); 2.69 (s, 3H, $CH_3$-Triazol-Ring); 1.71 (s, 2H, $NH_2$); 1.56 - 3.20 (m, 7H, $CH_2$, CH-5-Ring, N-$CH_2$).

Beispiel 24

$^{1}$H-NMR ($CDCl_3$): δ 7.22 - 7.56 (m, 4H, Aryl); 5.73 (t, J = 6Hz, 1H, NH); 4.91 (s, breit, 2H, $CH_2$-7-Ring; 3.22 (m, 2H, N-$CH_2$); 2.68 (s, 3H, $CH_3$ Triazol-Ring);1.94 (s, 3H, $CH_3$ (= O); 1.89 - 3.09 (m, 5H, $CH_2$, CH, 5-Ring).

Beispiel 27

$^{1}$H-NMR ($CDCl_3$): δ 7.18 - 7.55 (m, 4H, Aryl-H); 5.54/4.22 (2 s, breit, 2H, $CH_2$-7-Ring); 4.22 (qu, J = 7Hz, 2H, $OCH_2$); 3.91 (s, 2H, N-$CH_2C=C$); 3.43 (s, 2H, N-$CH_2C=O$); 2.78 (m, 2H, N$\underline{CH_2}CH_2$); 2.70 (s, 3H, $CH_3$ Triazol); 1.98 (m, 2H, N-$CH_2\underline{CH_2}$); 1.3O (t, J = 7Hz, $\underline{CH_3}CH_2$-).

Beispiel 28

$^{1}$H-NMR ($CDCl_3$); δ 7.O4 - 7.67 (m, 4H, Aryl); 6.87 (qu, J = < 1Hz, 1H, CH=); 5.38 und 4.O7 (AB-System, $J_{AB}$ = 13Hz, 2H, $CH_2$-7-Ring); 4.O6 (qu, J = 8Hz, 2H, $OCH_2$); 2.41 (d, J = < 1Hz, 3H, $CH_3$-C=); 1.48 - 3.O7 (m, 7H, $CH_2$, CH-6-Ring); 1.17 (t, J = 8Hz, $CH_2\underline{CH_3}$).

Beispiel 64

$^{1}$H-NMR ($CDCl_3$); δ 7.17 - 7.64 (m, 4H, Aryl-H); 6.87 (qu, J< 1Hz, 1H, HC=); 5.37/4.O9 (AB-System, $J_{AB}$ = 12Hz, 2H, $CH_2$-7-Ring); 3.37 (d, J = 6Hz, 2H, $CH_2$); 2.41 (d, J = < 1Hz, 3H, $CH_3$Imidazolring); 1.O4 - 2.86 (m, 7H, $CH_2$, CH, 6-Ring).

Beispiel 67

$^{1}$H-NMR ($CDCl_3$); δ 7.22 - 7.63 (m, 4H, Aryl-H); 4.97 (s, breit, 2H, $CH_2$-7-Ring); 4.12 (d, J = 6Hz, 2H, $OCH_2$); 2.98 (s, 3H, $CH_3$-$SO_2$-); 2.76 (s, 3H, $CH_3$-Triazol-Ring); 1.58 - 3.37 (m, 5H, $CH_2$, CH-5-Ring).

Beispiel 74

$^{1}$H-NMR ($CDCl_3$) δ 7.25 - 7.48 (4H, m. Aryl-H); 4.85 (2H, AB-System $J_{AB}$ = 12Hz, $CH_2$-7-Ring); 4.28 (3H, s, $0CH_3$); 3.64 (4H, m, $0CH_2$ Morpholinyl); 2.35 (4H, m, $NCH_2$ Morpholinyl); 1.43 - 3.28 (7H, m, Cyclopentenyl-H, N-$CH_2$).

Beispiel 77

$^1$H-NMR ($CDCl_3$); δ 7.19 - 7.60 (m, 4H, Aryl-H); 4.92 (s, breit, 2H, $CH_2$-7-Ring); 2.7o (s, 3H, $CH_3$ Triazol)-;2.47 (qu, J = 7Hz, 4H, N-$(CH_2)_2$), 1.47 - 3.18 (m, 7H, $CH_2$, CH-5-Ring, $NCH_2$); O.93 (t, J = 7Hz, 6H ($CH_3$-$CH_2)_2$-N).

Beispiel 78

$^1$H-NMR ($CDCl_3$) δ 7.20 - 7.54 (4H, m, Aryl-H); 4.92 (2H, s, breit, $CH_2$-7-Ring); 2.68 (3H, s, $CH_3C=N$); 2.15 (3H, s, $CH_3N$); 1.46 - 3.24 (8H, m, H-Cyclopentenyl, N-CH); 0.92 (6H, 2d, J = 7Hz, $CH_3$-Isopropyl).

Beispiel 86

$^1$H-NMR ($CDCl_3$): δ 7.23 - 7.62 (m, 4H, Aryl-H); 6.56 (t, J = 4Hz, $^1$H, NH); 5.62/4.31 (AB-System, breit, 2H, $CH_2$-7-Ring); 4.67 (s, 2H, $NCH_2$-Thiophen); 4.07 (d, J = 4Hz, 2H, $CH_2NH$); 3.51 (s, breit, 4H, $CH_2$ 6-Ring); 2.70 (s, 3H, $CH_3$ Triazol); 2.06 (s, 3H, $CH_3C=0$).

Beispiel 105

$^1$H-NMR ($CDCl_3$) δ 7.18 - 7.59 (4H, m, Aryl-H); 4.93 (2H, s, breit, $CH_2$-7-Ring); 2.69 (3H, s, $CH_3$-C=N);2.61 (2H, qu, J = 7Hz, $CH_3CH_2$-N); 1.44 - 3.29 (8H, m, H-Cyclopentenyl, NH); 1.05 (3H, t, J = 7Hz, $CH_3CH_2$-N).

Beispiel 106

$^1$H-NMR ($CDCl_3$) δ 7.22 - 7.56 (4H, m, Aryl-H); 4.93 (2H, s, breit, $CH_2$-7-Ring); 2.68 (3H, s, $CH_3C=N$); 2.06 (3H, s, $CH_3C=0$); 1.47 - 3.76 (9H, m, H-Cyclopentenyl, $NCH_2$); 1.13 (3H, t, J = 7Hz, $CH_3CH_2$).

Beispiel 107

$^1$H-NMR ($CDCl_3$) δ 7.05 - 7.61 (4H, m, Aryl H); 4.92 (2H, s, breit, $CH_2$-7-Ring); 2.69 (3H, s, $CH_3$-C=N);2.22) 6H, s, $(CH_3)_2N$); 1.53 - 3.35 (12H, m, Cyclopentenyl-H, $CH_2$-$CH_2$; NH).

Beispiel 108

$^1$H-NMR ($CDCl_3$) δ 7.22 - 7.58 (4H, m, Aryl-H); 4.93 (2H, s, breit, $CH_2$-7-Ring); 3.67(4H, m, $CH_20$-morpholinyl); 2.67 (3H, s, $CH_3C=N$); 2.41 (4H, m, N-$CH_2$ morpholinyl); 1.51 - 3.30 (12H, m, $NCH_2CH_2N$, Cyclopentenyl-H, NH).

Beispiel 109

$^1$H-NMR ($CDCl_3$ δ 7.18 - 7.60 (4H, m, Aryl-H); 4.94 (2H, s, breit, $CH_2$-7-Ring); 2.70 (3H, s, $CH_3C=N$); 2.25 (6H, s, $(CH_3)_2$-N); 2,12 (3H, s, $CH_3C=0$); 1.16 - 3.79 (11H, m, H-Cyclopentenyl, $NCH_2CH_2N$).

Beispiel 110

$^1$H-NMR ($CDCl_3$) δ 7.17 - 7.61 (4H, m, Aryl-H); 4.95 (2H, s, breit, $CH_2$-7-Ring); 3.71 (4H, m, $OCH_2$ morphinyl-H); 2.69 (3H, s, $CH_3C=N$); 2.45 (4H, m, $NCH_2$-morpholinyl); 2.10 (3H, s, $CH_3C=0$); 1.47 - 3.56 11H, m, cyclopentenyl-H, $NCH_2CH_2N$).

Beispiel 111

$^1$H-NMR ($CDCl_3$) δ 7.17 - 7.60 (4H, m, Aryl-H); 4.94 (2H, s, breit, $CH_2$-7-Ring); 2.69 (3H, s, $CH_3C=N$); 1.46 - 3.25 (16H, m, $CH_2$-Cyclopentenyl, $CH_2$-piperidine, NH).

Beispiel 112

$^1$H-NMR ($CDCl_3$) δ 7.10 - 7.59 (4H, m, Aryl-H); 6.89 (1H, qu, J = < 2Hz, CH=); 4.90 (2H, s, breit, $CH_2$-7-Ring); 3.98 (2H, d, J = 6Hz, $0CH_2$); 2.45 (3H, d, J = <2Hz, $CH_3$-C=C); 2.03 (3H, s, $CH_3$-C=0); 1.59 - 3.35

(5H, m, Cyclopentenyl-H).

Beispiel 113

$^1$H-NMR ($CDCl_3$) δ 7.23 - 7.61 (4H, m, Aryl-H); 6.90 (1H, qu, J = < 2Hz, CH=); 4.82 (2H, s, breit, 7-Ring-$CH_2$); 3.66 (4H, m, 0$CH_2$morpholinyl); 2.44 (3H, d, J = < 2Hz, $CH_3$-C=C); 2.37 (4H, m, N$CH_2$-morpholinyl); 1.46 - 3,26 (7H m, Cyclopentenyl H).

Beispiel 114

$^1$H-NMR ($CDCl_3$) δ 7.20 - 7.59 (4H, m, Aryl-H); 6.88 ($^1$H, qu, J = < 2 Hz, CH=); 4.78 (2H, s, breit, 7-Ring-$CH_2$); 2.47 (4H, 2qu, J = 7Hz, N-($CH_2CH_3$)$_2$); 2.45 (3H, d, J = <2Hz, $CH_3$C=C); 1.55 - 3.15 (7H, m, H-cyclopentenyl); 0.93 (6H, t, J = 7 Hz, N-($CH_2CH_3$)$_2$).

Beispiel 115

$^1$H-NMR ($CDCl_3$) δ 8.23 ($^1$H, s, NH-Indol); 6.97 -7.75 (9H m, Aryl; Indolyl-H); 4.90 (2H, s, breit, 7-Ring $CH_2$); 2.93 (4H, s, N$CH_2CH_2$N);2.68 (3H, s, $CH_3$C=N); 1.49 - 3.10 (8H, m, Cyclopentenyl-H,NH).

Beispiel 116

$^1$H-NMR ($CDCl_3$) δ 7.23 - 7.60 (4H, m, Aryl-H); 4.96 (2H, s, breit, $CH_2$-7-Ring); 3.31 (3H, s, $OCH_3$); 3.30 (2H, d, J = 6Hz, 0$CH_2$); 2.70 (3H, s, $CH_3$C=N); 1.56 - 3.39 (5H, m, Cyclopentenyl-H).

Beispiel 117

$^1$H-NMR ($CDCl_3$) δ 6.71 - 7.61 (9H, m, Aryl-H); 4.95 (2H, s, breit, $CH_2$-7-Ring); 3.90 (2H, d, J = 6Hz, 0$CH_2$); 2.72 (3H, s, $CH_3$C=N); 1.58 - 3.40 (5H, m, cyclopentenyl-H).

Beispiel 125

$^1$H-NMR DMSO-d6(δ 8.48 (1H, s, breit, CH=); 7.41 - 7.62 (4H, m, Aryl-H); 5.00 (2H, s, breit, $CH_2$-7-Ring); 3.16 - 3.26 (2H, m, $CH_2$-S); 2.63 (3H, s, $CH_3$-C=N), 1.40 - 3.16 (5H, m, H-Cyclopentenyl); NH nicht gezeigt.

Beispiel 126

$^1$H-NMR ($CDCl_3$) δ 7.22 - 7.62 (4H, m, Aryl-H); 4.93 (2H, s, breit, $CH_2$-7-Ring); 3.42 (2H, d, J = 10Hz, $CH_2$Br); 2.70 (3H, s, $CH_3$-C=N); 1.64 - 3.33 (5H, m, H-Cyclopentenyl)

Beispiel 127

$^1$H-NMR ($CDCl_3$) δ 7.34 - 7.63 (4H, m, Aryl-H); 4.92 (2H, s, breit, $CH_2$-7-Ring); 2.74 (3H, s, $CH_3$C=N); 2.47 (2H, d, J = 8Hz, $CH_2$C=N); 1.61 - 3.32 (5H, m, H-Cyclopentenyl).

Beispiel 130

$^1$H-NMR ($CDCl_3$): δ 7.10 - 7.61 (m, 4H-Aryl; 5.56/4.24 (AB-System, $J_{AB}$ = 12Hz, 2H, $CH_2$-7-Ring); 3.52 - 3.93 (m, 4H, -$OCH_2$ Morpholin); 2.68 (s, 3H, $CH_3$-Triazol-Ring); 2.25 - 2.55 (m, 4H, N-$CH_2$-Morpholin); 1.07 - 3.2O (m, 9H, $CH_2$, CH, 6-Ring, N-$CH_2$).

Beispiel 131

$^1$H-NMR ($CDCl_3$): δ 7.15 - 7.62 (m, 4H-Aryl); 5.56/4.17 (AB-System, $J_{AB}$ = 12Hz, 2H, $CH_2$-7-Ring); 3.59 (d, 2H, J = 6Hz, $CH_2$O); 2.66 (s, 3H, $CH_3$-Triazol-Ring); 1.06 - 3.12 (m, 8H, $CH_2$, CH 6-Ring, N-$CH_2$)

Beispiel 133

$^1$H-NMR ($CDCl_3$) δ 7.18 - 7.72 (4H, m, Aryl-H); 6.89 (1H, qu, J = <2Hz, CH=); 4.80 - 6.00 (6H, m, $CH_2$ = CH-); 5.39/4.10 (2H, AB-Syst. $J_{AB}$ = 12 Hz, $CH_2$-7-Ring); 3.73 - 4.01 (4H, m, $NCH_2$); 3.50 - 3.73 (1H, m, Cyclohexenyl-H); 2.41 (3H, d, J = < 2Hz, $CH_3C=C$); 1.58 - 200 (6H, m, $CH_2$-Cyclohexenyl).

Beispiel 134

$^1$H-NMR ($CDCl_3$) δ 7.11 - 7.64 (4H, m, Aryl-H); 6.90 (1H, qu, J = <2Hz, CH=); 5.42/4.12 (2H, AB-Syst., $J_{AB}$ = 12Hz, $CH_2$-7-Ring); 2.42 (3H, d, J = < 2Hz, $CH_3$-C=C); 1.08 - 3.48 (15H, m, Cyclohexenyl-H, N-$(CH_2CH_2-)_2$); 0.83 (6H, 2t, J = 7Hz, $(CH_2CH_2\underline{CH_3})_2$.

Beispiel 135

$^1$H-NMR ($CDl_3$) δ 7.20 - 7.60 (4H, m, Aryl-H); 4.80 - 5.97 (6H, m, $CH_2$ = CH-); 4.21/5.17 (2H, AB-Syst. $J_{AB}$ = 12Hz, $CH_2$-7-Ring); 3.54 - 4.01 (5H, m, $CH_2N$; CH-Cyclohexenyl); 2.67 (3HC=N); 1.56 - 3.04 (6H, m, $CH_2$-Cyclohexenyl).

Beispiel 138

$^1$H-NMR ($CDCl_3$) δ 7.21 - 7.56 (4H, m, Aryl-H); 4.67 - 6.09 (6H, m, $CH_2$=CH); 4.89 (2H, s, breit, $CH_2$-7-Ring); 1.89 - 4.13 (9H, m, $NCH_2$; cyclopentenyl-H); 2.68 (3H, s, $CH_3$-C=N).

Beispiel 139

$^1$H-NMR ($CDCl_3$) δ 7.33 - 7.55 (4H, m, Aryl-H); 4.92 (2H, s, breit, $CH_2$-7-Ring); 2.74 (3H, s, $CH_3C=N$); 1.04 - 3.81 (33H, m, Octylamin-$CH_2$, Cyclopentenyl-H); 0.89 (6H, 2t, J = 6Hz, $CH_3$-Cyclooctyl).

Beispiel 140

$^1$H-NMR ($CDCl_3$) δ 7.22 - 7.58 (4H, m, Aryl-H); 4.94 (2H, s, breit, $CH_2$-7-Ring); 2.69 (3H, s, $CH_3C=N$); 1.23 - 3.41 (13H, m, N-$CH_2CH_2$-$CH_3$); 0.82 (6H, t, J = 7Hz, N-$CH_2CH_2CH_3$).

Beispiel 141

$^1$H-NMR ($CDCl_3$) δ 7.22 - 7.56 (4H, m, Aryl-H); 6.91 (1H, qu, J = <2Hz, CH=); 4.80 (2H, s, braod, $CH_2$-Ring); 1.22 - 3.93 (13H, m, N-$CH_2CH_2$-$CH_3$, Cyclopentenyl-H); 2.43 (3H, d, J = <2Hz, $CH_3C=C$); 0.84 (6H, t, J = 7Hz, N-$CH_2CH_2CH_3$).

Beispiel 142

$^1$H-NMR ($CDCl_3$) δ 7.19 - 7.51 (4H, m, Aryl-H); 6.88 (1H, qu, J = <2Hz, CH=); 4.90 - 5.99 (6H, m, $CH_2$ = CH); 4.70 (2H, s, breit, $CH_2$-7-Ring); 1.81 - 4.06 (9H, m, $NCH_2$, Cyclopentenyl-H); 2.42 (3H, d, J = < 2Hz, $CH_3$-C=C).

Beispiel 143

$^1$H-NMR ($CDCl_3$) δ 7.28 - 7.54 (4H, m, Aryl-H); 7.28; 7.20 (2H, 2d, J = < 2Hz, CH = CH); 4.98; 4.82 (2H, AB System, $J_{AB}$ = 15Hz, $CH_2$-7-Ring);1.93 - 3.93 (13H, m, Cyclopentenyl-H, Morpholin-H).

Beispiel 144

$^1$H-NMR ($CDCl_3$) δ 7.20 - 7.53 (4H, m, Aryl-H); 5.22 (2H, s, breit, $CH_2$-7-Ring); 2.25 (3H, s, $CH_3$-C=C); 1.86 - 3.87 (13H, m, Cyclopentenyl-H, morpholin-H)

Beispiel 145

$^1$H-NMR ($CDCl_3$) δ 7.18 - 7.53 (4H, m, Aryl-H); 7.00 ($^1$H, qu, J = < 2Hz, CH=); 4.83 (2H, s, breit, $CH_2$-7-Ring); 1.91 - 3.87 (13H, m, Cyclopentenyl-H, Morpholin-H; 2.26 (3H, d, J = <2Hz, $CH_3$-C=C).

Beispiel 147

$^1$H-NMR ($CDCl_3$) δ 7.26 - 7.53 (4H, m, Aryl-H); 5.96 ($^1$H, t, J = 7Hz, NH); 4.86 (2H, s, breit, $CH_2$-7-Ring); 2.71 (3H, s, $CH_3$ C = N); 1.39 - 3.43 (7H, m, Cyclopentenyl-H, $NCH_2$); 1.26 (28H, s, Hexadecanyl); 0.88 (3H, t, J = 6Hz, 3H, $CH_3$-Hexadecanyl).

Nachfolgend werden Beispiele für einige pharmazeutische Zusammensetzungen unter Verwendung von Verbindungen der allgemeinen Formel I oder II als aktiver Bestandteil angegeben. Falls nicht ausdrücklich anders bezeichnet, handelt es sich bei den Teilen um Gewichtsteile.

Tabletten

1. Die Tablette enthält folgende Bestandteile:

| | |
|---|---|
| Wirkstoff gemäß Formel Ia/Ib | 0,020 Teile |
| Stearinsäure | 0,010 " |
| Dextrose | 1,890 " |
| gesamt | 1,920 Teile |

Herstellung:
Die Stoffe werden in bekannter Weise zusammengemischt und die Mischung zu Tabletten verpreßt, von denen jede 1,92 g wiegt und 20 mg Wirkstoff enthält.

2. Salbe
Die Salbe setzt sich aus folgenden Bestandteilen zusammen:

| | |
|---|---|
| Wirkstoff gemäß Formel Ia/Ib | 50 mg |
| Neribas Salbe (Handelsware Scherax) | ad 10 g |

Herstellung:
Der Wirkstoff wird mit 0,5 g Salbengrundlage verrieben und die restliche Grundlage in Teilmengen zu 1,0 g nach und nach innig zu einer Salbe vermischt. Man erhält eine 0,5 %ige Salbe. Die Verteilung des Wirkstoffes in der Grundlage wird optisch unter dem Mikroskop kontrolliert.

3. Creme
Zusammensetzung:
Wirkstoff gemäß Formel Ia/Ib 50 mg
Neribas Salbe (Handelsware Scherax) ad 10 g
Herstellung
Der Wirkstoff wird mit 0,5 g Cremegrundlage verrieben und die restliche Grundlage in Teilmengen zu 1,0 g nach und nach mit Pistill eingearbeitet. Man erhält eine 0,5%ige Creme. Die Verteilung des Wirkstoffes in der Grundlage wird optisch unter dem Mikroskop kontrolliert.

4. Ampullenlösung
Zusammensetzung:
Wirkstoff gemäß Formel Ia/Ib 1,0 mg
Natriumchlorid 45,0 mg
Aqua pro inj. ad 5,0 ml
Herstellung:
Der Wirkstoff wird bei Eigen-pH oder gegebenenfalls bei pH 5,5 bis 6,5 in Wasser gelöst und Natriumchlorid als Isotonanz zugegeben. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 1 mg, 5 mg und 10 mg Wirkstoff.

5. Suppositorien
Jedes Zäpfchen enthält:

| | |
|---|---|
| Wirkstoff gemäß Formel Ia/Ib | 1,0 Teile |
| Kakaobutter (Fp.36-37 °C) | 1200,0 Teile |
| Carnaubawachs | 5,0 Teile |

Herstellung

Kakaobutter und Carnaubawachs werden zusammengeschmolzen. Bei 45 °C gibt man den Wirkstoff hinzu und rührt, bis eine komplette Dispersion entstanden ist.

Die Mischung wird in Formen entsprechender Größe gegossen und die Zäpfchen zweckmäßig verpackt.

6. Inhalationslösungen

Zusammensetzung:

a)

| | |
|---|---|
| Wirkstoff gemäß Formel Ia/Ib | 500 mg |
| Na-EDTA | 50 mg |
| Benzalkoniumchlorid | 25 mg |
| Natriumchlorid | 880 mg |
| destilliertes Wasser ad | 100 ml |

Herstellung:

96 % der Wassermenge werden vorgelegt, darin nacheinander Na-EDTA, Benzalkoniumchlorid, Natriumchlorid und Wirkstoff klar gelöst und mit dem restlichen Wasser aufgefüllt. Die Lösung wird in 20 ml-Tropfflaschen abgefüllt. Eine Dosis (20 Tropfen, 1 ml) enthält 5 mg Wirkstoff.

b)

| | |
|---|---|
| Wirkstoff gemäß Formel Ia/Ib | 500 mg |
| Natriumchlorid | 820 mg |
| destilliertes Wasser ad | 100 ml |

Herstellung

96 % der Wassermenge werden vorgelegt, darin nacheinander der Wirkstoff und Natriumchlorid gelöst, mit dem restlichen Wasser aufgefüllt und die Lösung in Eindosenbehälter (4 ml) abgefüllt. Die Lösung enthält 20 mg Wirkstoff.

**Patentansprüche**

1. Verbindungen der allgemeinen Formel I

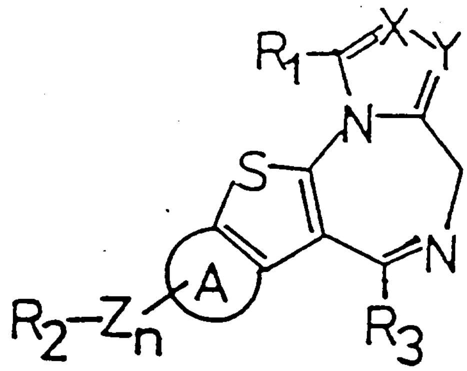

I a

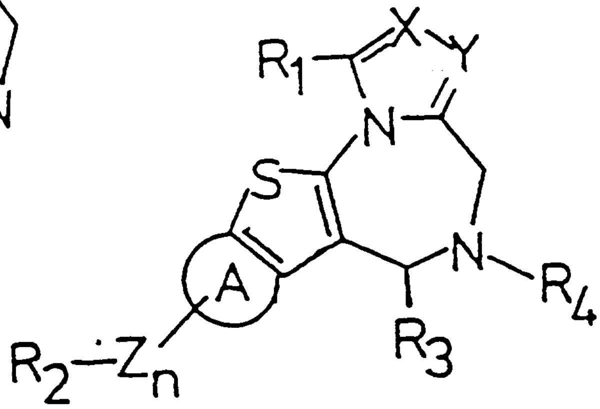

I b

worin

A ein ankondensierter einfach ungesättigter 5-, 6- oder 7- gliedriger Ring, wobei im Fall von n = 0 und $R_2$ = Wasserstoff ein Kohlenstoffatom durch C=0 ersetzt werden kann, oder A einen ankondensierten Ring der Formel

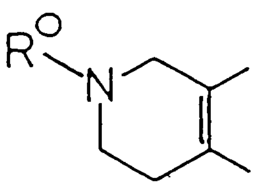

bedeutet,
wobei $R^0$ eine verzweigte oder unverzweigte Alkylgruppe mit 1 bis 18 Kohlenstoffatomen, eine Alkylcarbonyl- oder Alkylthiocarbonylgruppe mit 1 bis 18 Kohlenstoffatomen in der Alkylkette oder eine gegebenenfalls substituierte Phenylcarbonyl- oder gegebenenfalls substituierte Phenylthiocarbonylgruppe eine Alkoxycarbonylalkylgruppe mit bis zu 18, eine Aminocarbonylgruppe , eine Alkylcarbonylaminoalkylgruppe mit bis zu 18 Kohlenstoffatomen, eine Alkylcarbonylamioalkylcarbonylgruppe mit bis zu 18, oder eine Aminocarbonylalkylgruppe mit bis zu 18 Kohlenstoffatomen in der Alkylkette oder Wasserstoff, mit der Maßgabe, daß wenn $R^0$ Wasserstoff $R_1$ nicht gleichzeitig Methyl, $R_3$ nicht o-Chlorphenyl und X und Y nicht beide Stickstoff bedeuten;

Z eine verzweigte oder unverzweigte Alkylen- oder Alkenylengruppe mit n Kohlenstoffatomen;

n 0, 1, 2, 3, 4, 5 oder 6; im Fall einer Alkenylengruppe 0, 2, 3, 4, 5 oder 6;

X/Y unabhängig voneinander C-$R_1$, oder N, aber nicht beide C-$R_1$, oder Y die Gruppe C-COOR', wobei R' Alkyl oder Wasserstoff bedeutet und X Stickstoff bedeutet;

$R_1$ Wasserstoff, eine verzweigte oder unverzweigte Alkylgruppe mit 1 bis 4 Kohlenstoffatomen, die gegebenenfalls durch Hydroxy oder Halogen substituiert sein kann, eine Cyclopropyl- oder Cyclopentylgruppe, eine verzweigte oder unverzweigte Alkoxygruppe mit 1 bis 4 Kohlenstoffatomen, Halogen,

$R_2$ Hydroxy, Formyl, Carboxy, Cyano, verzweigtes oder unverzweigtes Alkyloxycarbonyl mit 1 bis 18, Kohlenstoffatomen, wobei die Alkylkette gegebenenfalls durch Hydroxy, Amino, Nitro oder Halogen substituiert sein kann, eine gegebenenfalls substituierte Phenyloxycarbonylgruppe;
einen Rest der allgemeinen Formel

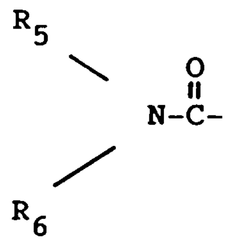

worin $R_5$ und $R_6$, die gleich oder verschieden sein können, Wasserstoff, Phenyl, substituiertes Phenyl, eine verzweigte oder unverzweigte Alkyl-, Alkenyl- oder Alkinylgruppe mit bis zu 18 Kohlenstoffatomen, die gegebenenfalls durch Halogen, Hydroxy, Nitro,
Amino , Alkoxy, oder im Fall von $R_6$ = Wasserstoff oder Alkyl, durch ein Säureamid der allgemeinen Formel

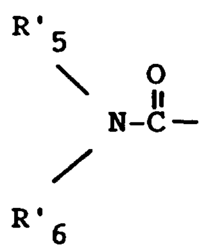

worin $R'_5$ und $R'_6$, dieselbe Bedeutung wie $R_5$ und $R_6$, jedoch mit Ausnahme eines Säureamids haben können, substituiert sein kann; $R_5$ oder $R_6$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoff verknüpfter 5- oder 6-gliedriger heterocyclischer Ring;
oder
$R_5$ und $R_6$ zusammen mit dem Stickstoffatom einen gesättigten oder ungesättigten gegebenenfalls ein- oder mehrfach durch verzweigte oder unverzweigte Alkylgruppen mit 1 bis 4 Kohlenstoffatomen substituierten 5- oder 6-Ring, der als weitere Heteroatome Stickstoff, Sauerstoff oder Schwefel enthalten kann, wobei jedes weitere Stickstoffatom durch eine verzweigte oder unverzweigte Alkylgruppe mit 1 bis 4 Kohlenstoffatomen, substituiert sein kann;

$R_2$ einen Rest der allgemeinen Formel

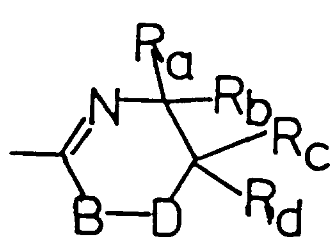

oder

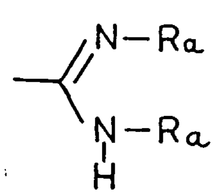

worin
B Sauerstoff, Schwefel, NH oder N-$C_1$-$C_6$-Alkyl,

D den Rest $(CReRf)_n$, wobei n 0 bis 3 sein kann, Ra Wasserstoff, Alkyl mit 1 bis 6 Kohlenstoffatomen, $C_1$ bis $C_4$ Alkoxycarbonyl oder Dialkylaminocarbonyl,
Rb, Rc, Rd, Re, Rf Wasserstoff, Alkyl mit 1 bis 6 Kohlenstoffatomen, oder Phenyl;

$R_2$ Wasserstoff, wenn A eine Carbonylfunktion oder Stickstoff als Ringglied enthält;

$R_2$

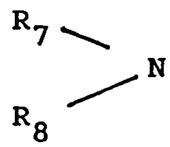

$R_7$ = Wasserstoff, $R_8$ = Wasserstoff,
Alkylcarbonyl oder Alkoxycarbonyl mit 1 bis 18, Aminocarbonyl, Alkylaminocarbonyl, Dialkylaminocarbonyl mit 1 bis 18 in der Alkylkette;

$R_2$ Wasserstoff (für n > O);

$R_2$ Halogen,

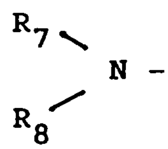

,

wobei $R_7$ und $R_8$, die gleich oder verschieden sein können, Wasserstoff, eine verzweigte oder unverzweigte Alkyl-, Alkenyl- oder Alkinylgruppe mit bis zu 18 Kohlenstoffatomen, die gegebenenfalls durch Halogen, Hydroxy oder einen C-verknüpften Heterocyclus substituiert sein kann, wobei die Kohlenstoffkette durch Stickstoff, Sauerstoff oder Schwefel unterbrochen sein kann, eine verzweigte oder unverzweigte Alkylcarbonylgruppe mit 1-6 Kohlenstoffatomen, gegebenenfalls substituiert durch Hydroxy oder Halogen, oder substituiert durch eine gegebenenfalls ein- oder zweifach durch eine verzweigte oder unverzweigte Alkylgruppe mit 1 bis 6 Kohlenstoffatomen substituierte Aminogruppe, wobei die Alkylgruppe durch Halogen oder Hydroxy substituiert sein kann,
eine gegebenenfalls substituierte Phenylcarbonylgruppe, eine gegebenenfalls substituierte Phenylsulfonyl, eine Alkylsulfonylgruppe mit 1 bis 4 Kohlenstoffatomen,
oder
$R_7$ und $R_8$ bilden zusammen mit dem Stickstoffatom einen gesättigten oder ungesättigten gegebenenfalls ein- oder mehrfach durch verzweigte oder unverzweigte Alkylgruppen mit 1 bis 4 Kohlenstoffatomen substituierten 5- oder 6-Ring, der als weitere Heteroatome Stickstoff, Sauerstoff oder Schwefel enthalten kann, wobei jedes weitere Stickstoffatom gegebenenfalls durch eine verzweigte oder unverzweigte Alkylgruppe mit 1 bis 4 Kohlenstoffatomen, substituiert sein kann;

$R_2$ Phenylsulfonyloxy, gegebenenfalls ein- oder mehrfach durch verzweigte oder unverzweigte Alkyl- und/oder Alkoxygruppen mit 1 bis 4 Kohlenstoffatomen substituiert;

$R_2$ eine verzweigte oder unverzweigte Alkylsulfonyloxygruppe mit 1 bis 4 Kohlenstoffatomen;

$R_2$ Phenylcarbonyloxy, gegebenenfalls ein oder mehrfach durch verzweigte oder unverzweigte Alkyl- und/oder Alkoxygruppen mit 1 bis 4 Kohlenstoffatomen substituiert;

$R_2$ eine verzweigte oder unverzweigte Alkylcarbonyloxygruppe mit 1 bis 18, Kohlenstoffatomen, wobei die Alkylkette durch Stickstoff, Sauerstoff oder Schwefel unterbrochen sein kann;

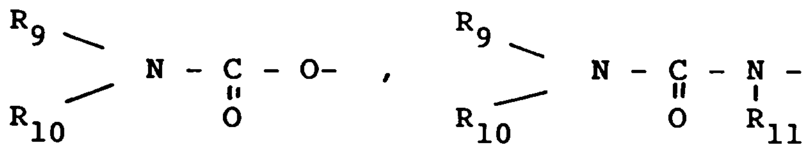

wobei $R_9$ Wasserstoff und $R_{10}$ eine Alkyl-, Alkenyl- oder Alkinylgruppe mit bis zu 4 Kohlenstoffatomen, gegebenenfalls durch Halogen substituiert, eine gegebenenfalls ein- oder mehrfach durch verzweigte oder unverzweigte Alkyl- und/oder Alkoxygruppen mit 1 bis 4 Kohlenstoffatomen substituierte Phenylgruppe,

$R_{11}$ Wasserstoff oder eine verzweigte oder unverzweigte Alkylgruppe mit 1 bis 4 Kohlenstoffatomen;

$R_2$ einen Imidorest; einen Benzimidazolylrest;

$R_2$ einen verzweigten oder unverzweigten Alkyloxy- bzw. Alkylthiorest mit 1 bis 18, Kohlenstoffatomen, einen gegebenenfalls substituierten Phenyloxy- oder Phenylthiorest, einen über Sauerstoff oder Schwefel verknüpften, gesättigten oder ungesättigten 5- oder 6-gliedrigen heterocyclischen Ring;

wobei als Substituenten für einen substituierten Phenylrest - sofern nicht gesondert definiert - $C_1$-$C_4$-Alkyl, $C_3$-$C_7$-Cycloalkyl, $C_1$-$C_4$-Alkoxy, Hydroxy oder Halogen angegeben wird,

$R_3$ Phenyl, wobei der Phenylring ein oder mehrfach, durch Methyl, Halogen, Nitro und/oder Trifluormethyl substituiert sein kann, oder Pyridyl, und

$R_4$ Wasserstoff, verzweigtes oder unverzweigtes Alkyl mit 1 bis 4 Kohlenstoffatomen oder Alkylcarbonyl mit 1 bis 4 Kohlenstoffatomen bedeuten können,

gegebenenfalls in Form ihrer Racemate, ihrer Enantiomere, ihrer Diastereomere und ihrer Gemische sowie gegebenenfalls ihre physiologisch unbedenklichen Säureadditionssalze.

2. Verbindungen der allgemeinen Formel Ia gemäß Anspruch 1, worin A ein ankondensierter einfach ungesättigter 5- oder 6-gliedriger Ring, oder A einen ankondensierten Ring der Formel

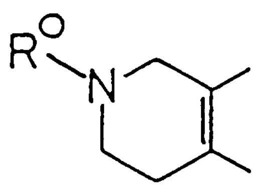

worin $R^\circ$ Acetylaminoacetyl, Acetyl, Thioacetyl, Ethoxycarbonylmethyl, Methoxycarbonylmethyl, Morpholinylcarbonylmethyl, Diethylaminocarbonylmethyl;

Z eine unverzweigte Alkylengruppe mit n Kohlenstoffatomen

n O, 1, 2, 3 oder 4;

X/Y beide N oder X = C-H oder C-$CH_3$ und Y = N,

$R_1$ Wasserstoff, Hydroxymethyl, Chlormethyl, Cyclopropyl, Ethyl, Methoxy, Ethoxy, Chlor, Brom, oder Methyl;

$R_2$ Hydroxy, Carboxy, Cyano, Brom, Alkyloxycarbonyl mit 1 bis 6 Kohlenstoffatomen,

$R_2$ einen Rest der allgemeinen Formel

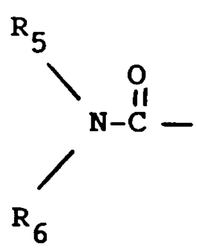

worin $R_5$ und $R_6$, die gleich oder verschieden sein können, Wasserstoff, eine verzweigte oder unverzweigte Alkylgruppe oder Alkenylgruppe mit bis zu 6, 8 oder 16 Kohlenstoffatomen, die gegebenenfalls durch Halogen, Hydroxy, Methoxy, Nitro, Amino, Alkylamino oder Dialkylamino mit 1 bis 4 Kohlenstoffatomen in der Alkylkette, oder im Fall von $R_6$ = Wasserstoff oder Alkyl durch Morpholinylcarbonyl oder Diethylaminocarbonyl substituiert sein kann,

im Fall von $R_5$ = Wasserstoff oder Methyl, $R_6$ ein Thiazolin- oder Thiazolrest, der gegebenenfalls durch eine verzweigte oder unverzweigte Alkylgruppe mit 1 bis 4 C-Atomen substituiert sein kann,

oder

$R_5$ und $R_6$ zusammen mit dem Stickstoffatomen einen Morpholino- oder Piperazinorest bilden, der gegebenenfalls ein- oder mehrfach durch Methyl substituiert sein kann;

$R_2$ ein C-verknüpfter $\Delta^2$-Imidazolin-, -Thiazolin-, -Ozazolin-, oder Tetrahydropyrimidin-Rest, der gegebenenfalls ein- oder mehrfach durch Alkyl mit 1 bis 4 Kohlenstoffatomen substituiert sein kann;

$R_2$ im Fall von n = O Wasserstoff, wenn A eine Carbonylfunktion oder Stickstoff als Ringglied enthält,

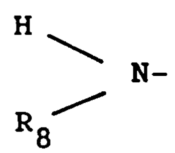

$R_8$ eine Alkyloxycarbonylgruppe mit 1 bis 4 Kohlenstoffatomen;

im Fall von n > O

$R_2$ eine Alkylcarbonyloxygruppe mit 1 bis 3 Kohlenstoffatomen;

$R_2$ eine Alkylsulfonyloxygruppe mit 1 bis 2 Kohlenstoffatomen;

$R_2$

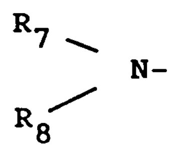

wobei $R_7$ und $R_8$, die gleich oder verschieden sein können, Wasserstoff, eine verzweigte oder unverzweigte Alkylgruppe mit 1 bis 6 Kohlenstoffatomen, gegebenenfalls durch Dialkylamino mit 1 bis 4 Kohlenstoffatomen,

Morpholino oder N-Alkylpiperazino oder ein Indolrest substituiert, eine Alkylcarbonylgruppe mit 1 bis 4 Kohlenstoffatomen,

oder

$R_7$ und $R_8$ zusammen mit dem Stickstoffatom einen Morpholino- oder Piperazinorest bilden, der gegebenenfalls ein- oder mehrfach durch Methyl substituiert sein kann, ein Triazolorest, ein Imidazolorest, ein Pyrazolorest, Pyrrolorest, ein Imidorest,

$R_2$ eine verzweigte oder unverzweigte Alkylsulfonyloxygruppe mit 1 bis 4 Kohlenstoffatomen, eine verzweigte oder unverzweigte Alkylcarbonyloxygruppe mit 1 bis 8 Kohlenstoffatomen;

$R_2$ Phenyloxy, 3,4-Methylendioxyphenoxy einen Pyridinyloxyrest, einen Alkyloxy- oder Alkylthiorest mit 1 bis 4 Kohlenstoffatomen;

$R_3$ Phenyl oder o-Chlorphenyl, bedeuten können, sowie gegebenenfalls ihre physiologisch unbedenklichen Säureadditionssalze und ihre optisch aktiven Verbindungen.

**3.** Verbindungen der allgemeinen Formel Ia gemäß Anspruch 1, worin A ein ankondensierter, in 3- oder 4-Stellung des Hetrazepins substituierter, einfach ungesättigter 5- oder 6-gliedriger Ring,

Z eine unverzweigte Alkylengruppe mit n Kohlenstoffatomen

n 0, 1 oder 2;

X/Y beide N, oder X C-H oder C-$CH_3$ und Y N,
$R_1$ Wasserstoff, Cyclopropyl, Methoxy, Brom, Methyl;
$R_2$ Hydroxy, Amino, Carboxy, Cyano, Methoxycarbonyl, Ethoxycarbonyl,
$R_2$ einen Rest der allgemeinen Formel

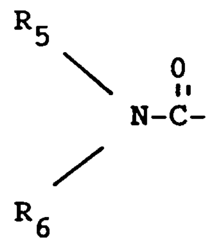

worin $R_5$ und $R_6$, die gleich oder verschieden sein können, Wasserstoff, Propenyl, Phenyl, eine verzweigte oder unverzweigte Alkylgruppe mit 1 bis 6, 8 oder 16 Kohlenstoffatomen, die gegebenenfalls durch Halogen, Hydroxy, Nitro, Amino, Ethylamino oder Diethylamino, Methoxy, oder im Fall von $R_6$ = Wasserstoff oder Alkyl durch Morpholinylcarbonyl oder Diethylaminocarbonyl substituiert sein kann, im Fall von $R_5$ = Wasserstoff oder Methyl, $R_6$ ein Thiazolin- oder Thiazolrest, der gegebenenfalls durch Methyl substituiert sein kann,
oder
$R_5$ und $R_6$ zusammen mit dem Stickstoffatom einen Morpholino- oder Piperazinorest bilden, der gegebenenfalls ein- oder mehrfach durch Methyl substituiert sein kann;

$R_2$ ein C-verknüpfter $\Delta^2$-Imidazolin-, -Thiazolin-Oxazolin-Rest, der gegebenenfalls ein- oder mehrfach durch Methyl, Ethyl und/oder iso-Propyl substituiert sein kann, ein Tetrahydro-Pyrimidinring, gegebenenfalls ein- oder mehrfach durch Methyl substituiert, ein Benzimidazolrest, ein Indolrest, oder Methoxycarbonylamino;

im Fall von n > 0

$R_2$ eine Acetoxygruppe, eine Methansulfonyloxygruppe,

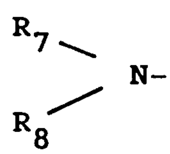

wobei $R_7$ und $R_8$, die gleich oder verschieden sein können, Wasserstoff, eine verzweigte oder unverzweigte Alkylgruppe mit 1 bis 4 Kohlenstoffatomen, die durch Diethylamino oder Morpholino substituiert sein kann, eine Acetylgruppe oder
$R_7$ und $R_8$ zusammen mit dem Stickstoffatom einen Morpholino- oder Piperazinorest bilden, der gegebenenfalls ein- oder mehrfach durch Methyl substituiert sein kann, ein Triazolorest, ein Imidazolorest, ein Phthalimid,

$R_2$ eine verzweigte oder unverzweigte Alkylsulfonyloxygruppe mit 1 bis 4 Kohlenstoffatomen, eine verzweigte oder unverzweigte Alkylcarbonyloxygruppe mit 1 bis 8 Kohlenstoffatomen;

$R_2$ ein Phenyloxyrest, ein Pyridyloxyrest, 3,4-Methylendioxyphenoxy, eine 1,2,4-Triazol-3-yl-thiogruppe, Methoxy,

$R_3$ Phenyl oder o-Chlorphenyl bedeuten können, sowie gegebenenfalls ihre physiologisch unbedenklichen Säureadditionssalze, gegebenenfalls ihre optisch aktiven Verbindungen.

**4.** Verbindungen nach einem der Ansprüche 1, 2 oder 3, dadurch gekennzeichnet, daß X und Y beide Stickstoff, $R_1$ Methyl und $R_3$ ortho-Chlorphenyl bedeuten.

**5.** 3-(Morpholin-4-yl-carbonyl)-5-(2-chlorphenyl)-10-methyl-3,4-dihydro-2H,7H-cyclopenta[4,5]thieno[3,2-f]-[1,2,4]triazolo[4,3-a][1,4]diazepin

**6.** 3-(Dipropylaminocarbonyl)-5-(2-chlorphenyl)-10-methyl-3,4-dihydro-2H, 7H-cyclopenta[4,5]thieno[3,2-f]-[1,2,4]triazolo[4,3-a][1,4]diazepin

**7.** Verfahren zur Herstellung von Verbindungen der allgemeinen Formel Ia nach einem der Ansprüche 1 bis 4, worin $R_1$, $R_3$, A, Z, und n wie zuvor definiert, $R_2$ Wasserstoff oder - COOR', mit R' = $C_1$ - $C_4$-Alkyl oder Wasserstoff, oder $R_2$ Alkylcarbonyloxy, einen Ether- oder Thioetherrest, ein Säureamid oder ein Amin, wie zuvor definiert,
dadurch gekennzeichnet, daß man eine entsprechende Verbindung der allgemeinen Formel

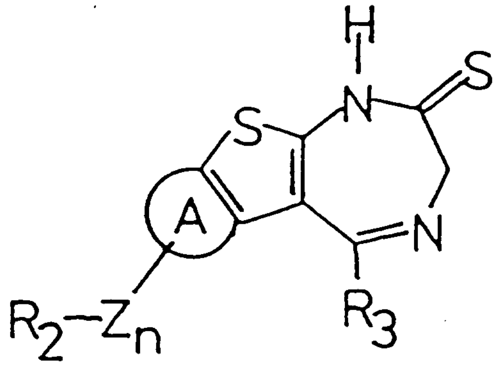

II

worin $R_2$, $R_3$, A, Z und n die zuvor genannte Bedeutung haben

A) für den Fall, daß X und Y Stickstoff bedeutet

a) mit einem Säurehydrazid der allgemeinen Formel

$R_1$-CONHNH$_2$

umsetzt,

b) oder aber mit Hydrazin in eine Verbindung der allgemeinen Formel

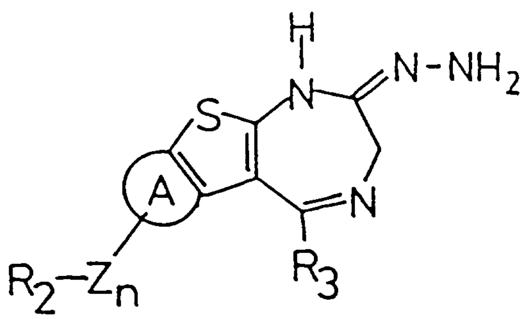

IV

überführt und anschließend mit einen Säurehalogenid der allgemeinen Formel

$R_1$-C0-Hal

oder mit einem Orthoester der allgemeinen Formel

$R_1$ - C (OR')$_3$

worin R' eine $C_1$-$C_4$ Alkylgruppe bedeutet, umsetzt, oder

B) für den Fall daß X C-H und Y Stickstoff

a) mit einem Aminoalkin der allgemeinen Formel

$R_{11}$ - C≡C - CH$_2$-NH$_2$

worin $R_{11}$ Wasserstoff oder eine $C_1$-$C_4$ Alkylgruppe bedeutet

oder aber

b) mit einem α-Aminoaldehyd-alkylacetal oder α-Aminoketon-alkylketal der allgemeinen Formel

$H_2NCH_2-CR_1(0R')_2$

worin $R_1$ Wasserstoff oder eine Alkylgruppe mit 1 bis 4 Kohlenstoffatomen und R' eine $C_1$-$C_4$ Alkylgruppe bedeutet, umsetzt

C) für den Fall, daß X Stickstoff und Y C-H man eine Verbindung der allgemeinen Formel

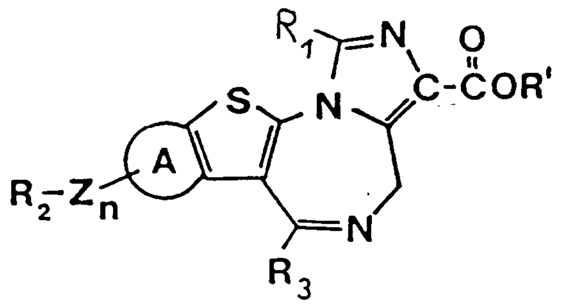

R' = $C_1$-$C_4$- Alkyl

decarboxyliert.

**8.** Verfahren zur Herstellung von Verbindungen der allgemeinen Formel

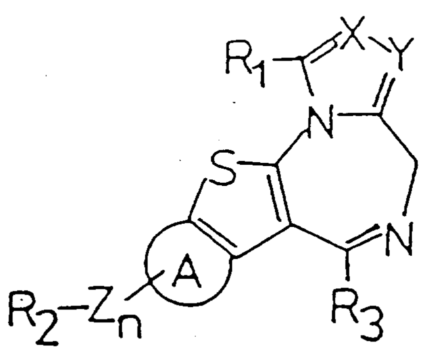

Ia

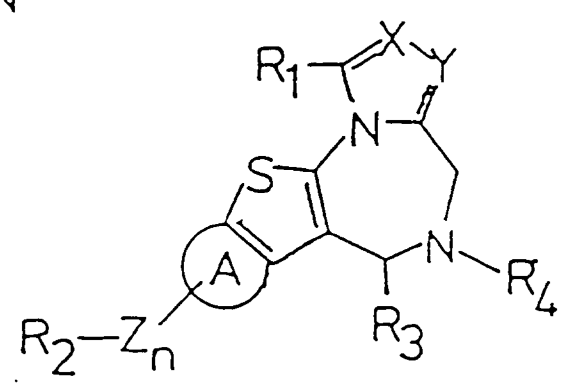

Ib

worin

A ein ankondensierter einfach ungesättigter 5-, 6- oder 7- gliedriger Ring, wobei im Fall von n = 0 und $R_2$ = Wasserstoff ein Kohlenstoffatom durch C=0 ersetzt werden kann, oder A einen ankondensierten Ring der Formel

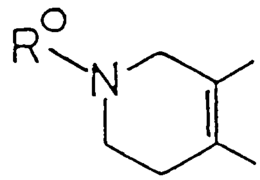

bedeutet,
wobei $R^0$ eine verzweigte oder unverzweigte Alkylgruppe mit 1 bis 18 Kohlenstoffatomen, eine Alkylcarbonyl- oder Alkylthiocarbonylgruppe mit 1 bis 18 Kohlenstoffatomen in der Alkylkette oder eine gegebenenfalls substituierte Phenylcarbonyl- oder gegebenenfalls substituierte Phenylthiocarbonylgruppe eine Alkoxycarbonylalkylgruppe mit bis zu 18, eine Aminocarbonylgruppe , eine Alkylcarbonylaminoalkylgruppe mit bis zu 18, eine Alkylcarbonylamioalkylcarbonylgruppe mit bis zu 18 oder eine Aminocarbonylalkylgruppe mit bis zu 18 Kohlenstoffatomen in der Alkylkette oder Wasserstoff, mit der Maßgabe, daß wenn $R^0$ Wasserstoff $R_1$ nicht gleichzeitig Methyl, $R_3$ nicht o-Chlorphenyl und X und Y nicht beide Stickstoff bedeuten;

Z eine verzweigte oder unverzweigte Alkylen- oder Alkenylengruppe mit n Kohlenstoffatomen;

n 0, 1, 2, 3, 4, 5 oder 6; im Fall einer Alkenylengruppe 0, 2, 3, 4, 5 oder 6;

X/Y unabhängig voneinander C-$R_1$, oder N, aber nicht beide C-$R_1$,
oder Y die Gruppe C-COOR', wobei R' Alkyl oder Wasserstoff bedeutet und X Stickstoff bedeutet;

$R_1$ Wasserstoff, eine verzweigte oder unverzweigte Alkylgruppe mit 1 bis 4 Kohlenstoffatomen, die gegebenenfalls durch Hydroxy oder Halogen substituiert sein kann, eine Cyclopropyl- oder Cyclopentylgruppe, eine verzweigte oder unverzweigte Alkoxygruppe mit 1 bis 4 Kohlenstoffatomen, Halogen,

$R_2$ Hydroxy, Formyl, Carboxy, Cyano, verzweigtes oder unverzweigtes Alkyloxycarbonyl mit 1 bis 18, Kohlenstoffatomen, wobei die Alkylkette gegebenenfalls durch Hydroxy, Amino, Nitro oder Halogen substituiert sein kann, eine gegebenenfalls substituierte Phenyloxycarbonylgruppe;
einen Rest der allgemeinen Formel

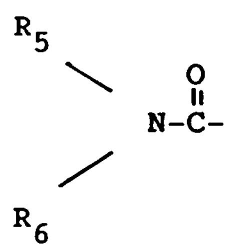

worin $R_5$ und $R_6$, die gleich oder verschieden sein können, Wasserstoff, Phenyl, substituiertes Phenyl, eine verzweigte oder unverzweigte Alkyl-, Alkenyl- oder Alkinylgruppe mit bis zu 18 Kohlenstoffatomen, die gegebenenfalls durch Halogen, Hydroxy, Nitro, Amino , Alkoxy, oder im Fall von
$R_6$ = Wasserstoff oder Alkyl, durch ein Säureamid der allgemeinen Formel

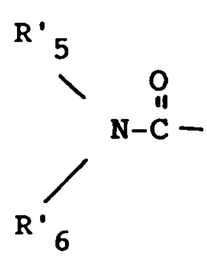

worin $R'_5$ und $R'_6$, dieselbe Bedeutung wie $R_5$ und $R_6$, jedoch mit Ausnahme eines Säureamids haben können, substituiert sein kann;

$R_5$ oder $R_6$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoff verknüpfter 5- oder 6-gliedriger heterocyclischer Ring;

oder

$R_5$ und $R_6$ zusammen mit dem Stickstoffatom einen gesättigten oder ungesättigten gegebenenfalls ein- oder mehrfach durch verzweigte oder unverzweigte Alkylgruppen mit 1 bis 4 Kohlenstoffatomen substituierten 5- oder 6-Ring, der als weitere Heteroatome Stickstoff, Sauerstoff oder Schwefel enthalten kann, wobei jedes weitere Stickstoffatom durch eine verzweigte oder unverzweigte Alkylgruppe mit 1 bis 4 Kohlenstoffatomen, substituiert sein kann;

$R_2$ einen Rest der allgemeinen Formel

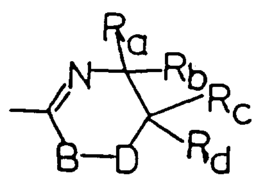

oder

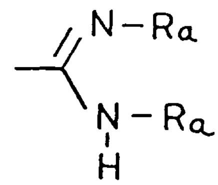

worin

B Sauerstoff, Schwefel, NH oder N-$C_1$-$C_6$-Alkyl,

D den Rest $(CReRf)_n$, wobei n 0 bis 3 sein kann, Ra Wasserstoff, Alkyl mit 1 bis 6 Kohlenstoffatomen, $C_1$ bis $C_4$ Alkoxycarbonyl oder Dialkylaminocarbonyl,

Rb, Rc, Rd, Re, Rf Wasserstoff, Alkyl mit 1 bis 6 Kohlenstoffatomen, oder Phenyl;

$R_2$ Wasserstoff, wenn A eine Carbonylfunktion oder Stickstoff als Ringglied enthält;

$R_2$

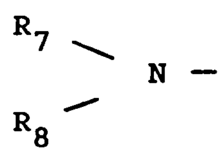

$R_7$ = Wasserstoff, $R_8$ = Wasserstoff, Alkylcarbonyl oder Alkoxycarbonyl mit 1 bis 18, Aminocarbonyl, Alkylaminocarbonyl, Dialkylaminocarbonyl mit 1 bis 18 in der Alkylkette;

$R_2$ Wasserstoff (für n > O);

$R_2$ Halogen,

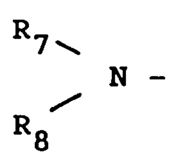

,

wobei $R_7$ und $R_8$, die gleich oder verschieden sein können, Wasserstoff, eine verzweigte oder unverzweigte Alkyl-, Alkenyl- oder Alkinylgruppe mit bis zu 18 Kohlenstoffatomen, die gegebenenfalls durch Halogen, Hydroxy oder einen C-verknüpften Heterocyclus substituiert sein kann, wobei die Kohlenstoffkette durch Stickstoff, Sauerstoff oder Schwefel unterbrochen sein kann, eine verzweigte oder unverzweigte Alkylcarbonylgruppe mit 1-6 Kohlenstoffatomen, gegebenenfalls substituiert durch Hydroxy oder Halogen, oder substituiert durch eine gegebenenfalls ein- oder zweifach durch eine verzweigte oder unverzweigte Alkylgruppe mit 1 bis 6 Kohlenstoffatomen substituierte Aminogruppe, wobei die Alkylgruppe durch

Halogen oder Hydroxy substituiert sein kann,
eine gegebenenfalls substituierte Phenylcarbonylgruppe, eine gegebenenfalls substituierte Phenylsulfonyl, eine Alkylsulfonylgruppe mit 1 bis 4 Kohlenstoffatomen,
oder
$R_7$ und $R_8$ bilden zusammen mit dem Stickstoffatom einen gesättigten oder ungesättigten gegebenenfalls ein- oder mehrfach durch verzweigte oder unverzweigte Alkylgruppen mit 1 bis 4 Kohlenstoffatomen substituierten 5- oder 6-Ring, der als weitere Heteroatome Stickstoff, Sauerstoff oder Schwefel enthalten kann, wobei jedes weitere Stickstoffatom gegebenenfalls durch eine verzweigte oder unverzweigte Alkylgruppe mit 1 bis 4 Kohlenstoffatomen, substituiert sein kann;

$R_2$ Phenylsulfonyloxy, gegebenenfalls ein- oder mehrfach durch verzweigte oder unverzweigte Alkyl- und/oder Alkoxygruppen mit 1 bis 4 Kohlenstoffatomen substituiert;

$R_2$ eine verzweigte oder unverzweigte Alkylsulfonyloxygruppe mit 1 bis 4 Kohlenstoffatomen;

$R_2$ Phenylcarbonyloxy, gegebenenfalls ein oder mehrfach durch verzweigte oder unverzweigte Alkyl- und/oder Alkoxygruppen mit 1 bis 4 Kohlenstoffatomen substituiert;

$R_2$ eine verzweigte oder unverzweigte Alkylcarbonyloxygruppe mit 1 bis 18, Kohlenstoffatomen, wobei die Alkylkette durch Stickstoff, Sauerstoff oder Schwefel unterbrochen sein kann;

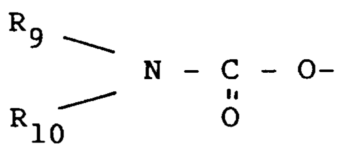 , 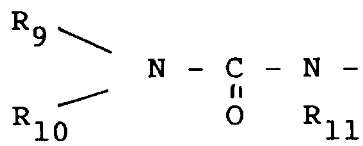

wobei $R_9$ Wasserstoff und $R_{10}$ eine Alkyl-, Alkenyl- oder Alkinylgruppe mit bis zu 4 Kohlenstoffatomen, gegebenenfalls durch Halogen substituiert, eine gegebenenfalls ein- oder mehrfach durch verzweigte oder unverzweigte Alkyl- und/oder Alkoxygruppen mit 1 bis 4 Kohlenstoffatomen substituierte Phenylgruppe,
$R_{11}$ Wasserstoff oder eine verzweigte oder unverzweigte Alkylgruppe mit 1 bis 4 Kohlenstoffatomen;

$R_2$ einen Imidorest; einen Benzimidazolylrest;

$R_2$ einen verzweigten oder unverzweigten Alkyloxy- bzw. Alkylthiorest mit 1 bis 18, Kohlenstoffatomen, einen gegebenenfalls substituierten Phenyloxy- oder Phenylthiorest, einen über Sauerstoff oder Schwefel verknüpften, gesättigten oder ungesättigten 5- oder 6-gliedrigen heterocyclischen Ring;
wobei als Substituenten für einen substituierten Phenylrest - sofern nicht gesondert definiert - $C_1$-$C_4$-Alkyl, $C_3$-$C_7$-Cycloalkyl, $C_1$-$C_4$-Alkoxy, Hydroxy oder Halogen angegeben wird,

$R_3$ Phenyl, wobei der Phenylring ein oder mehrfach, durch Methyl, Halogen, Nitro und/oder Trifluormethyl substituiert sein kann, oder Pyridyl,
und

$R_4$ Wasserstoff, verzweigtes oder unverzweigtes Alkyl mit 1 bis 4 Kohlenstoffatomen oder Alkylcarbonyl mit 1 bis 4 Kohlenstoffatomen
bedeuten können, gegebenenfalls in Form ihrer Racemate, ihrer Enantiomere, ihrer Diastereomere und ihrer Gemische sowie gegebenenfalls ihre physiologisch unbedenklichen Säureadditionssalze,
dadurch gekennzeichnet, daß man Verbindungen der allgemeinen Formel

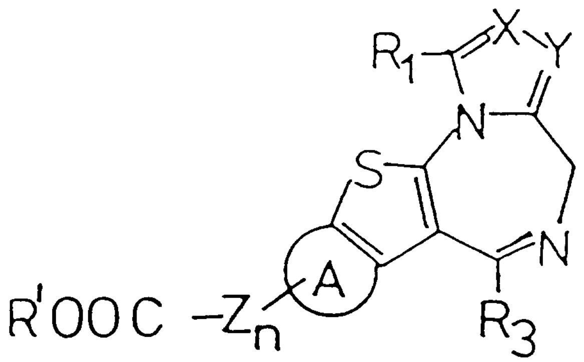

Ia1

worin $R_1$ = Wasserstoff, Alkyl, Cycloalkyl, A, Z, n, X, Y und $R_3$ wie zuvor definiert und R' eine $C_1$-$C_4$-Alkylgruppe ist,
wie folgt umsetzt:

1) Verbindungen der allgemeinen Formel Ia mit

$R_2$ = COOH

erhält man durch Verseifen von Verbindungen der Formel Ia1;

2) Verbindungen der allgemeinen Formel Ia mit

$R_2$ = $R_5R_6$ NCO-

erhält man durch Umsetzung von Ia mit

$R_2$ = C00H

mit einem Amin der Formel

$HNR_5R_6$

gegebenenfalls in Gegenwart von Sulfonyldimidazol, Carbonyldiimidazol oder Dicyclohexylcarbodiimid;

3) Verbindungen der allgemeinen Formel Ia mit

$R_2$ = OH

erhält man durch seletive Reduktion von Ia', z.B. mit Lithiumalanat oder Natriumborhydrid, bzw. für n = 0 durch Reduktion der entsprechenden Carbonylverbindung;

4) Verbindungen der allgemeinen Formel Ia mit

$R_2$ = $R_{10}$NH-COO- , $R_{10}$-CO-$NR_{11}$-

erhält man durch Reaktion des Alkohols oder Amins, z.B. hergestellt nach 3), mit einem Isocyanat der allgemeinen Formel

$R_{10}$ N = C = O;

5) Verbindungen der allgemeinen Formel Ia mit
$R_2$ = Alkyl-bzw. gegebenenfalls substituiertes Phenylcarbonyloxy
erhält man durch Reaktion des Alkohols, z.B. hergestellt nach 3), mit einem Säureäqivalent, z.B. einem Säurehalogenid, einer Carbonsäure der Formel

R-C00H,

worin R ein ein gegebenenfalls substituierter Phenylrest oder ein verzweigter oder ein unverzweigter Alkylrest mit 1 bis 8 Kohlenstoffatomen, wobei die Kohlenstoffkette durch Stickstoff, Sauerstoff oder Schwefel unterbrochen sein kann, bedeutet;
6) Verbindungen der allgemeinen Formel Ia mit
$R_2$ = Alkylsulfonyloxy oder Phenylsulfonyloxy erhält man
durch Reaktion des Alkohols z.B. hergestellt nach 3), mit einem Alkyl- oder Phenylsulfonylhalogenid unter Zusatz eines säurebindenden Mittels;
7) Verbindungen der allgemeinen Formel Ia mit

$R_2 = NR_7\ R_8$

oder ein Imidorest
erhält man durch Reaktion des Mesylats ($R_2 = CH_3SO_3$-), z. B. hergestellt nach 6), mit einem Amin der Formel

$HNR_7R_8$

oder einem Imid, gegebenenfalls in Form ihrer Alkalimetallsalze;
8) Verbindungen der allgemeinen Formel Ia mit

$R_2 = NH_2$

erhält man beispielsweise durch Spaltung des Phthalimids ($R_2$ = Phthalsäureimid) oder durch Curtius Abbau des entsprechenden Säureazids, bzw. durch Hydrolyse der entsprechenden Isocyanate, oder durch Reduktion des entsprechenden Azids;
9) Verbindungen der allgemeinen Formel Ia mit

$R_2 = NR_7\ R_8$

mit $R_7$ oder $R_8$ Alkyl- oder Phenylcarbonyl erhält man durch Reaktion des Amins, z.B. hergestellt nach 8), mit einem Carbonsäureäquivalent abgeleitet von einer Carbonsäure der Formel

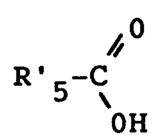

10) Verbindungen der allgemeinen Formel Ia mit

$R_2$ = Formyl

erhält man durch Oxidation des Alkohols, z.B, hergestellt nach 3), unter Verkürzung der Kette von n auf n-1;
11) Verbindungen der allgemeinen Formel Ia worin
$R_2$ ein Rest der allgemeinen Formel

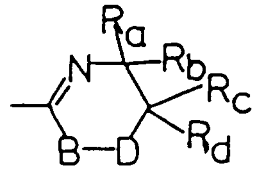

bedeutet,

erhält man durch Reaktion einer Verbindung der allgemeinen Formel Ia mit

$R_2$ = COOH

mit einem bisfunktionalisiertem Amin der allgemeinen Formel

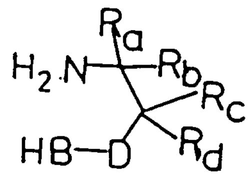

worin $R_a$, $R_b$, $R_c$, $R_d$, D und B die zuvor genannte Bedeutung aufweist, in Gegenwart von Triphenylphospin, $CCl_4$ und einer Base;
11a) Verbindungen der allgemeinen Formel Ia, worin $R_2$ ein gegebenenfalls substituierter Thiazolinrest ist, erhält man aus den entsprechenden Oxazolinen, z.B. hergestellt nach 11) durch Schwefelung, z.B. mit Phosphorpentasulfid oder Lawesson-Reagenz$^R$.
12) Verbindungen der allgemeinen Formel IA, worin
$R_2$ ein Amidin der allgemeinen Formel

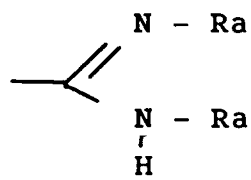

bedeute,
erhält man analog 11) durch Umsetzung mit einem primären Amin der Formel $H_2NRa$;
13) Verbindungen der allgemeinen Formel Ia mit

$R_2$ = CN

erhält man durch Reaktion von Verbindungen der allgemeinen Formel Ia, worin

$R_2$ = $CONH_2$

bedeutet, mit Phosphoroxychlorid;
14) Verbindungen der allgemeinen Formel Ia
worin $R_2$ = ein gegebenenfalls durch verzweigte oder unverzweigte Alkylgruppen substituiertes 2-Imidazolin ist,
erhält man durch Reaktion von Verbindungen der allgemeinen Formel Ia mit

$R_2$ = CN

a) mit ethanolischer HCl,
b) Umsetzen des entstandenen Imidoethylesters mit einen gegebenfalls durch verzweigte oder unverzweigte Alkylgruppen substituiertem Ethylendiamin,
c) gegebenfalls erfolgt die Alkylierung der freien N-H Funktion mit bekannten Alkylierungsmitteln;
15) Verbindungen der allgemeinen Formel Ia,
worin $R_2$ = Alkyl- oder Phenyloxycarbonyl bedeutet, erhält man durch Umsetzung von einem Säureäquivalent von I ($R_2$ = COOH) mit den entsprechenden Alkoholen;
16) Verbindungen der allgemeinen Formel Ia mit

mit $R_2$ = Halogen,

erhält man durch Reaktion des Tosylats

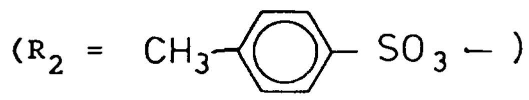

mit einem Halogenierungsmittel;
17) Verbindungen der allgemeinen Formel I. worin $R_2$ einen Ether, bzw. Thioether, bedeutet.
erhält man aus den entsprechenden Mesylaten, z.B. hergestellt nach 6) ($R_2 = CH_3SO_3$), durch Umsetzung mit den entsprechenden Alkoholen oder Mercaptanen als Solvens oder ihrer Alkalimetallsalze in einem inerten organischen Lösungsmittel;
18) Verbindungen der allgemeinen Formel Ia mit A gleich

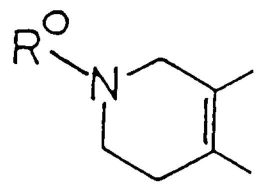

und R° gleich Alkyl, substituiertes Alkyl, Alkylcarbonyl, substituiertes Alkylcarbonyl oder Arylcarbonyl erhält man durch Alkylierung bzw. Acylierung von Verbindungen der allgemeinen Formel I mit R° = Wasserstoff,
die entsprechenden Thiocarbonylverbindungen durch Umsetzung der Carbonylverbindung mit einem Schwefelreagenz, z.B. Phosphorpentasulfid;
anschließend gewünschtenfalls die erhaltenen Verbindungen Ia

mit $R_1$ = Wasserstoff

in Gegenwart einer Base mit Chlor oder Brom zu einer Verbindung der allgemeinen Formel Ia mit $R_1$ = Chlor oder Brom umsetzt;
anschließend gewünschtenfalls die Halogenverbindung in eine Verbindung der allgemeinen Formel Ia mit $R_1$ = Alkoxy mit 1 bis 4 Kohlenstoffatomen durch Reaktion mit dem entsprechenden Alkoholat überführt;
anschließend gewünschtenfalls eine Verbindung der allgemeinen Formel Ia selektiv reduziert und gewünschtenfalls die erhaltene Verbindung Ib worin $R_4$ Wasserstoff bedeutet alkyliert oder acyliert und gegebenfalls in ihre pharmakologisch unbedenklichen Säureadditionssalze überführt und anschließend gegebenenfalls in ihre optisch aktiven Verbindungen trennt.

**9.** Verbindungen der allgemeinen Formel

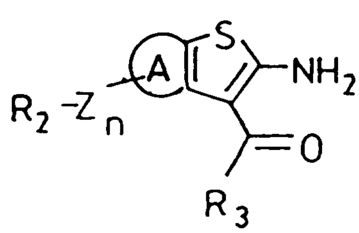

worin

A ein ankondensierter einfach ungesättigter 5-, 6- oder 7-gliedriger carbocyclischer Ring,
Z eine verzweigte oder unverzweigte Alkylen- oder Alkenylengruppe;
n 0, 1, 2, 3, 4, 5, oder 6; im Fall einer Alkenylengruppe 0, 2, 3, 4, 5 oder 6;
$R_2$ COOR*, mit R* = Wasserstoff oder $C_1$-$C_4$-Alkyl;
oder
$R_2$ $R_5R_6N$-CO-
$R_2$ -$NR_7R_8$
oder
$R_2$ Wasserstoff (für n größer 0), Hydroxy, Alkylcarbonyloxy, einen Ether oder Thioetherrest, Alkyl oder Phenylsulfonyloxy wie in einem der vorhergehenden Ansprüche definiert sind,
und
$R_3$ Phenyl, gegebenenfalls ein- oder mehrfach substituiert durch Halogen, Methyl, Nitro und/oder Trifluormethyl bedeuten können.

**10.** Verbindungen der allgemeinen Formel

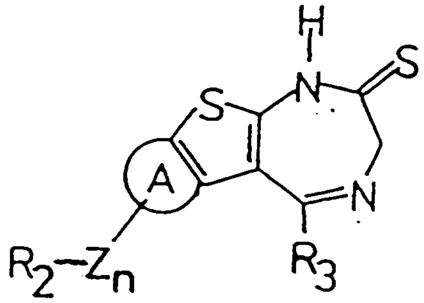

worin A, Z, n und $R_3$ wie in einem der vorhergehenden Ansprüche definiert und
$R_2$ Wasserstoff (für n größer 0), Hydroxy,
$R_2$ COOR*, mit R* = Wasserstoff oder $C_1$-$C_4$-Alkyl;
oder
$R_2$ $R_5R_6N$-CO-, worin $R_5$ und $R_6$ wie zuvor definiert sind;
$R_2$ -$NR_7R_8$, worin $R_7$ und $R_8$ wie zuvor definiert sind;
oder
$R_2$ einen Alkylcarbonyloxy, einen Ether - oder Thioetherrest, ein Amin, Alkyl- oder Phenylsulfonyloxyrest wie in einem der vorhergehenden Ansprüche definiert, bedeuten können.

**11.** Pharmazeutische Präparate, enthaltend als Wirkstoffe Verbindungen der allgemeinen Formel Ia oder Ib gemäß Anspruch 1 in Kombination mit üblichen Hilfs- und/oder Trägerstoffen.

**12.** Verfahren zur Herstellung von pharmazeutischen Präparaten nach Anspruch 11, dadurch gekennzeichnet, daß man Verbindungen der allgemeinen Formel Ia oder Ib mit üblichen galenischen Hilfs- und/oder Trägerstoffen zu üblichen pharmazeutischen Anwendungsformen verarbeitet.

**13.** Verbindungen der allgemeinen Formel Ia oder Ib gemäß Anspruch 1 oder 2 zur Verwendung für die Herstellung von Arzneimitteln mit PAF-antagonistischer Wirkung.

**14.** Verwendung von Verbindungen der allgemeinen Formel Ia wie in Anspruch 1, 2, 3, 4, 5 oder 6 definiert zur Herstellung eines Arzneimittels zur Behandlung von Asthma bronchiale.

## Claims

**1.** Compounds of general formula I

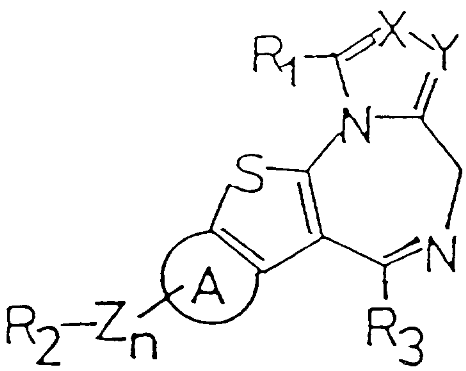

I a

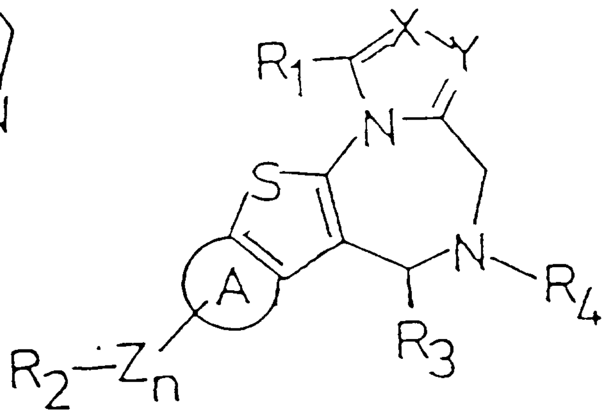

I b

wherein

A represents a fused mono-unsaturated 5-, 6-, or 7-membered ring, whilst if n = 0 and $R_2$ = hydrogen, a carbon atom may be replaced by C=0,
or A is a fused ring of formula

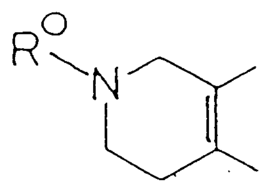

wherein R° is a branched or unbranched alkyl group having 1 to 18 carbon atoms, an alkylcarbonyl or alkylthiocarbonyl group having 1 to 18 carbon atoms in the alkyl chain or an optionally substituted phenylcarbonyl or
optionally substituted phenylthiocarbonyl group, an alkoxycarbonylalkyl group having up to 18, an aminocarbonyl group, an alkylcarbonylaminoalkyl group having up to 18 carbon atoms, an alkylcarbonylaminoalkylcarbonyl group having up to 18, or an aminocarbonylalkyl group having up to 18 carbon atoms in the alkyl chain or hydrogen, with the proviso that if R° is hydrogen, $R_1$ is not simultaneously methyl, $R_3$ is not o-chlorophenyl and X and Y are not both simultaneously nitrogen;

Z represents a branched or unbranched alkylene or alkenylene group having n carbon atoms;

n represents 0, 1, 2, 3, 4, 5 or 6; in the case of an alkenylene group, it represents 0, 2, 3, 4, 5 or 6;

X/Y independently of each other represent C-$R_1$ or N but cannot both represent C-$R_1$,
or Y represents the group C-COOR', wherein R' represents alkyl or hydrogen and X represents nitrogen;

$R_1$ represents hydrogen, a branched or unbranched alkyl group having 1 to 4 carbon atoms which may optionally be substituted by hydroxy or halogen, a cyclopropyl or cyclopentyl group, a branched or unbranched alkoxy group having 1 to 4 carbon atoms, halogen,

$R_2$ hydroxy, formyl, carboxy, cyano, branched or unbranched alkoxycarbonyl having 1 to 18 carbon atoms, whilst the alkyl chain may optionally be substituted by hydroxy, amino, nitro

or halogen, an optionally substituted phenyloxycarbonyl group; a group of general formula

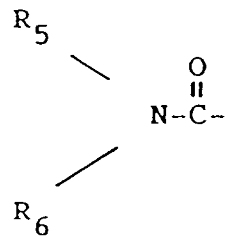

wherein $R_5$ and $R_6$ which may be identical or different represent hydrogen, phenyl, substituted phenyl, a branched or unbranched alkyl, alkenyl or alkynyl group with up to 18 carbon atoms which may optionally be substituted by halogen, hydroxy, nitro, amino, alkoxy or, if $R_6$ = hydrogen or alkyl, by an acid amide of general formula

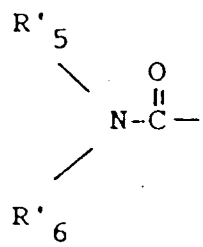

wherein $R'_5$ and $R'_6$ have the same meaning as $R_5$ and $R_6$ with the exception of an acid amide;

$R_5$ or $R_6$ represent a saturated or unsaturated 5- or 6-membered heterocyclic ring linked by a carbon, optionally mono or polysubstituted by branched or unbranched alkyl with 1 to 4 carbon atoms;

or

$R_5$ and $R_6$ together with the nitrogen atom represent a saturated or unsaturated 5- or 6-membered ring optionally mono- or polysubstituted by branched or unbranched alkyl groups with 1 to 4 carbon atoms, which may contain, as further heteroatoms, nitrogen, oxygen or sulphur whilst each additional nitrogen atom may be substituted by a branched or unbranched alkyl group with 1 to 4 carbon atoms;

$R_2$ represents a group of general formula

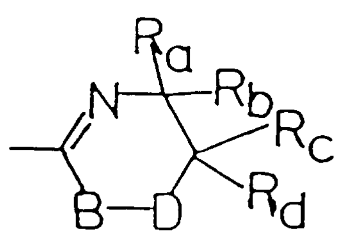

or

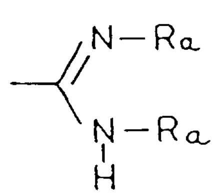

wherein

B represents oxygen, sulphur, NH or N-$C_{1-6}$-alkyl,

D represents the group $(CReRf)_n$, wherein n may be 0 to 3,

Ra represents hydrogen, alkyl with 1 to 6 carbon atoms, $C_{1-4}$-alkoxycarbonyl or dialkylaminocarbonyl,

Rb, Rc, Rd, Re, Rf represent hydrogen, alkyl with 1 to 6 carbon atoms, or phenyl;

$R_2$ represents hydrogen if A contains a carbonyl function or nitrogen as a ring member;

$R_2$

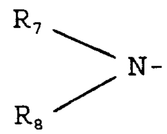

$R_7$ = hydrogen, $R_8$ = hydrogen,
alkylcarbonyl or alkoxycarbonyl with 1 to 18, aminocarbonyl, alkylaminocarbonyl, dialkylaminocarbonyl with 1 to 18 in the alkyl chain;

$R_2$ represents hydrogen (for n > 0);

$R_2$ represent halogen,

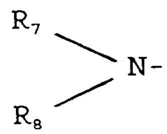

wherein $R_7$ and $R_8$ which may be identical or different, represent hydrogen, a branched or unbranched alkyl, alkenyl or alkynyl group with up to 18 carbon atoms which may optionally be substituted by halogen, hydroxy or C-linked heterocyclic group, whilst the carbon chain may be interrupted by nitrogen, oxygen or sulphur, a branched or unbranched alkylcarbonyl group with 1 to 6 carbon atoms, optionally substituted by hydroxy or halogen, or substituted by an amino group optionally mono- or bisubstituted by a branched or unbranched alkyl group with 1 to 6 carbon atoms, whilst the alkyl group may be substituted by halogen or hydroxy,

an optionally substituted phenylcarbonyl group, an optionally substituted phenylsulphonyl, an alkylsulphonyl group with 1 to 4 carbon atoms,

or

$R_7$ and $R_8$ together with the nitrogen atom form a saturated or unsaturated 5- or 6-membered ring optionally mono- or polysubstituted by branched or unbranched alkyl groups with 1 to 4 carbon atoms, which may contain, as further heteroatoms, nitrogen, oxygen or sulphur, whilst each additional nitrogen atom may optionally be substituted by a branched or unbranched alkyl group with 1 to 4 carbon atoms;

$R_2$ represents phenylsulphonyloxy, optionally mono- or polysubstituted by branched or unbranched alkyl and/or alkoxy groups with 1 to 4 carbon atoms;

$R_2$ represents a branched or unbranched alkylsulphonyloxy group having 1 to 4 carbon atoms;

$R_2$ represents phenylcarbonyloxy, optionally mono- or polysubstituted by branched or unbranched alkyl- and/or alkoxy groups having 1 to 4 carbon atoms;

$R_2$ represents a branched or unbranched alkylcarbonyloxy group with 1 to 18 carbon atoms, whilst the alkyl chain may be interrupted by nitrogen, oxygen or sulphur;

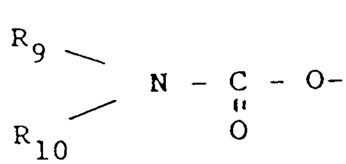 , 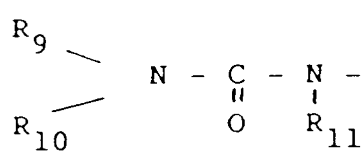

wherein $R_9$ represents hydrogen and $R_{10}$ is an alkyl-, alkenyl- or alkynyl group with up to 4 carbon atoms, optionally substituted by halogen, a phenyl group optionally mono- or polysubstituted by branched or unbranched alkyl and/or alkoxy groups with 1 to 4 carbon atoms,

$R_{11}$ represents hydrogen or a branched or unbranched alkyl group with 1 to 4 carbon atoms;

$R_2$ represents an imido group; a benzimidazolyl group;

$R_2$ represents a branched or unbranched alkyloxy or alkylthio group with 1 to 18 carbon atoms, an optionally substituted phenyloxy or phenylthio group, a saturated or unsaturated 5- or 6-membered heterocyclic ring linked via oxygen or sulphur;
the substituents for a substituted phenyl group being $C_{1-4}$-alkyl, $C_{3-7}$-cycloalkyl, $C_{1-4}$-alkoxy, hydroxy or halogen unless defined otherwise,

$R_3$ represents phenyl, whilst the phenyl ring may be mono- or polysubstituted by methyl, halogen, nitro and/or trifluoromethyl, or pyridyl, and

$R_4$ may represent hydrogen, branched or unbranched alkyl with 1 to 4 carbon atoms or alkylcarbonyl with 1 to 4 carbon atoms,

optionally in the form of their racemates, enantiomers, diastereomers and mixtures thereof; and optionally the physiologically harmless acid addition salts thereof.

**2.** Compounds of general formula Ia according to claim 1, wherein A represents a fused mono-unsaturated 5- or 6-membered ring, or A represents a fused ring of formula

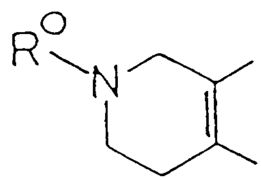

wherein R° represents acetylaminoacetyl, acetyl, thioacetyl, ethoxycarbonylmethyl, methoxycarbonylmethyl, morpholinylcarbonylmethyl, diethylaminocarbonylmethyl;

Z represents an unbranched alkylene group with n carbon atoms

n represents 0, 1, 2, 3 or 4;

X/Y both represent N or X = C-H or C-$CH_3$ and Y = N,

$R_1$ represents hydrogen, hydroxymethyl, chloromethyl, cyclopropyl, ethyl, methoxy, ethoxy, chlorine, bromine or methyl;

$R_2$ represents hydroxy, carboxy, cyano, bromine, alkyloxycarbonyl with 1 to 6 carbon atoms,

$R_2$ represents a group of general formula

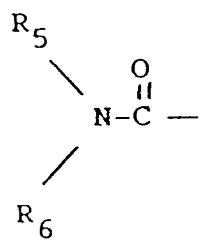

wherein $R_5$ and $R_6$ which may be identical or different represent hydrogen, a branched or unbranched alkyl group or alkenyl group with up to 6, 8 or 16 carbon atoms, which may optionally be substituted by halogen, hydroxy, methoxy, nitro, amino, alkylamino or dialkylamino having 1 to 4 carbon atoms in the alkyl chain, or if $R_6$ = hydrogen or alkyl by morpholinylcarbonyl or diethylaminocarbonyl,

if $R_5$ = hydrogen or methyl, $R_6$ represents a thiazoline or thiazole group which may optionally be substituted by a branched or unbranched alkyl group with 1 to 4 carbon atoms, or

$R_5$ and $R_6$ form, together with the nitrogen atom a morpholino- or piperazino group which may optionally be mono- or polysubstituted by methyl;

$R_2$ represents a C-linked $\Delta^2$-imidazoline-, -thiazoline-, -oxazoline-, or tetrahydropyrimidine group which may optionally be mono- or polysubstituted by alkyl with 1 to 4 carbon atoms;

$R_2$ represents, when n = O, hydrogen if A contains a carbonyl function or nitrogen as a ring member,

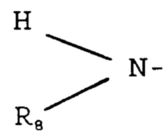

$R_8$ represents an alkyloxycarbonyl group with 1 to 4 carbon atoms;
in the case of n > O
$R_2$ represents an alkylcarbonyloxy group with 1 to 3 carbon atoms;
$R_2$ represents an alkylsulphonyloxy group with 1 to 2 carbon atoms;
$R_2$

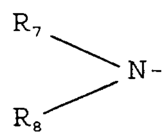

wherein $R_7$ and $R_8$, which may be identical or different, represent hydrogen, a branched or unbranched alkyl group with 1 to 6 carbon atoms, optionally substituted by dialkylamino with 1 to 4 carbon atoms,
morpholino or N-alkylpiperazino or an indole group, an alkylcarbonyl group with 1 to 4 carbon atoms, or $R_7$ and $R_8$ form together with the nitrogen atom a morpholino or piperazino group which may optionally be mono- or polysubstituted by methyl, a triazolo group, an imidazolo group, a pyrazolo group, pyrrolo group, an imido group,
$R_2$ represents a branched or unbranched alkylsulphonyloxy group with 1 to 4 carbon atoms, a branched or unbranched alkylcarbonyloxy group with 1 to 8 carbon atoms;
$R_2$ represents phenyloxy, 3,4-methylenedioxyphenoxy, a pyridinyloxy group, an alkyloxy- or alkylthio group with 1 to 4 carbon atoms;
$R_3$ may represent phenyl or o-chlorophenyl, and optionally the physiologically harmless acid addition salts and optically active compounds thereof.

**3.** Compounds of general formula Ia according to claim 1, wherein A represents a fused mono-unsaturated 5-, 6-membered ring substituted in the 3- or 4-position of the hetrazepine,
Z represents an unbranched alkylene group with n carbon atoms
n represents 0, 1 or 2;
X/Y both represent N or X represents C-H or C-$CH_3$ and Y represents N,
$R_1$ represents hydrogen, cyclopropyl, methoxy, bromine, methyl;
$R_2$ represents hydroxy, amino, carboxy, cyano, methoxycarbonyl, ethoxycarbonyl,
$R_2$ represents a group of general formula

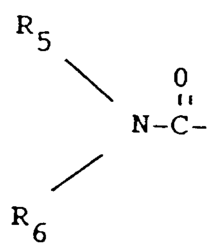

wherein $R_5$ and $R_6$, which may be identical or different, represent hydrogen, propenyl, phenyl, a branched or unbranched alkyl group with 1 to 6, 8 or 16 carbon atoms which may optionally be substituted by halogen, hydroxy, nitro, amino, ethylamino or diethylamino, methoxy or,
in the case of $R_6$ = hydrogen or alkyl, by morpholinylcarbonyl or diethylaminocarbonyl, in the case of $R_5$ = hydrogen or methyl, $R_6$ represents a thiazoline or thiazole group which

may optionally be substituted by methyl
or
$R_5$ and $R_6$ form together with the nitrogen atom a morpholino- or piperazino group which may optionally be mono- or polysubstituted by methyl;

$R_2$ represents a C-linked $\Delta^2$-imidazoline, -thiazoline, -oxazoline group which may optionally be mono- or polysubstituted by methyl, ethyl and/or isopropyl, a tetrahydro-pyrimidine ring, optionally mono- or polysubstituted by methyl,
a benzimidazole group, an indole group, or methoxycarbonylamino;

in the case of n > O

$R_2$ represents an acetoxy group, a methanesulphonyloxy group

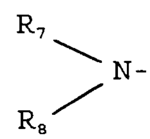

wherein $R_7$ and $R_8$, which may be identical or different, represent hydrogen, a branched or unbranched alkyl group with 1 to 4 carbon atoms which may be substituted by diethylamino or morpholino,
an acetyl group, or
$R_7$ and $R_8$ represent together with the nitrogen atom a morpholino- or piperazino group which may optionally be mono- or polysubstituted by methyl, a triazolo group, an imidazolo group, a phthalimide,

$R_2$ represents a branched or unbranched alkylsulphonyloxy group with 1 to 4 carbon atoms, a branched or unbranched alkylcarbonyloxy group with 1 to 8 carbon atoms;

$R_2$ represents a phenyloxy group, a pyridyloxy group, 3,4-methylenedioxyphenoxy, a 1,2,4-triazol-3-yl-thio group, methoxy,

$R_3$ may represent phenyl or o-chlorophenyl, and optionally the physiologically harmless acid addition salts and optionally the optically active compounds thereof.

**4.** Compounds according to one of claims 1, 2 or 3, characterised in that X and Y both represent nitrogen, $R_1$ represents methyl and $R_3$ represents ortho-chlorophenyl.

**5.** 3-(Morpholin-4-yl-carbonyl)-5-(2-chlorophenyl)-10-methyl-3,4-dihydro-2H,7H-cyclopenta[4,5]thieno[3,2-f]-[1,2,4]triazolo[4,3-a][1,4]diazepine.

**6.** 3-(Dipropylaminocarbonyl)-5-(2-chlorophenyl)-10-methyl-3,4-dihydro-2H,7H-cyclopenta[4,5]thieno[3,2-f]-[1,2,4]triazolo[4,3-a][1,4]diazepine.

**7.** A process for preparing compounds of general formula Ia as claimed in one of claims 1 to 4, wherein $R_1$, $R_3$, A, Z and n are as hereinbefore defined, $R_2$ represents hydrogen or -COOR', wherein R' = $C_{1-4}$-alkyl or hydrogen, or $R_2$ represents alkylcarbonyloxy, an ether or thioether group, an acid amide or an amine, characterised in that a corresponding compound of general formula

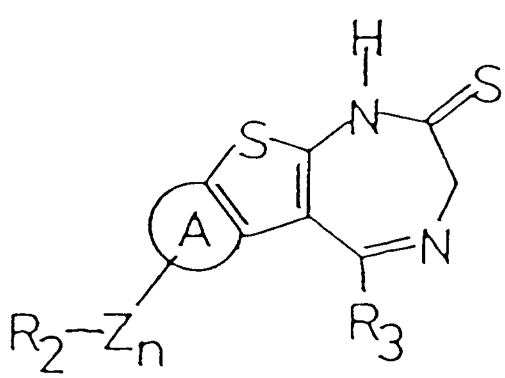

II

wherein $R_2$, $R_3$, A, Z and n are defined as hereinbefore

A) when X and Y represent nitrogen

a) is reacted with an acid hydrazide of general formula

$R_1$-CONHNH$_2$

b) or is converted with hydrazine into a compound of general formula

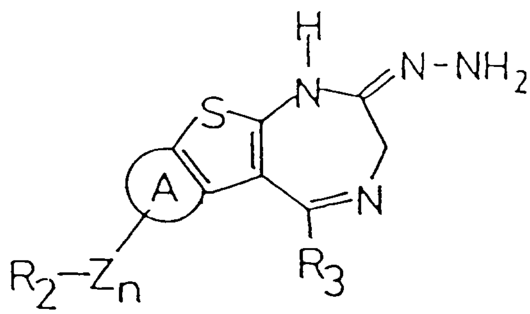

IV

and subsequently reacted with an acid halide of general formula

$R_1$-CO-Hal

or with an orthoester of general formula

$R_1$ - C (OR')$_3$

wherein R' represents a $C_{1-4}$-alkyl group, or

B) if X represents C-H and Y represents nitrogen

a) it is reacted with an aminoalkyne of general formula

$R_{11}$ - C ≡ C - CH$_2$-NH$_2$

wherein $R_{11}$ represents hydrogen or a $C_{1-4}$-alkyl group or

b) is reacted with an α-aminoaldehyde-alkylacetal or α-aminoketone-alkylketal of general formula

$H_2NCH_2$-CR$_1$(OR')$_2$

wherein $R_1$ represents hydrogen or an alkyl group with 1 to 4 carbon atoms and R' represents a $C_{1-4}$-alkyl group,

C) if X represents nitrogen and Y represents C-H a compound of general formula

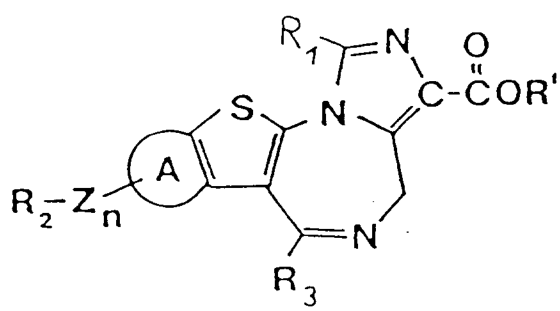

R' = $C_{1-4}$-alkyl

is decarboxylated.

**8.** A process for preparing compounds of general formula

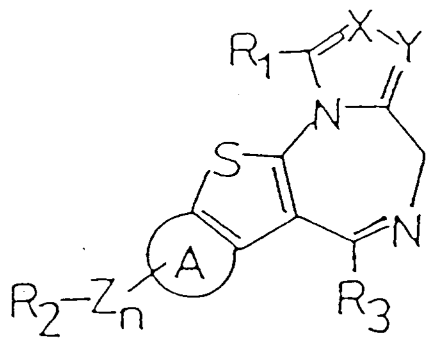

I a

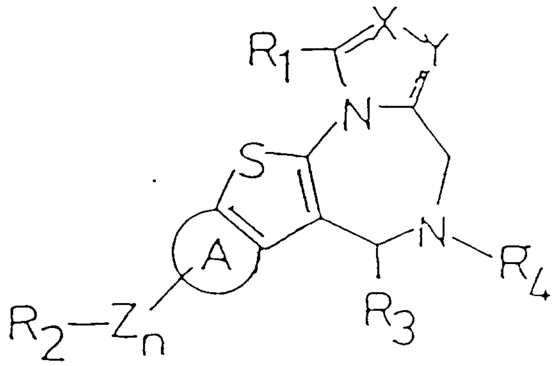

I b

wherein

A represents a fused mono-unsaturated 5-, 6- or 7-membered ring whilst in the case of n = O and $R_2$ = hydrogen a carbon atom may be replaced by C=O, or A represents a fused ring of formula

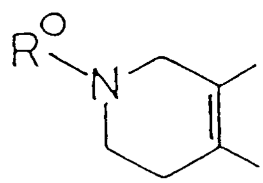

wherein

$R^0$ represents a branched or unbranched alkyl group with 1 to 18 carbon atoms, an alkylcarbonyl- or alkylthiocarbonyl group with 1 to 18 carbon atoms in the alkyl chain or an optionally substituted phenylcarbonyl- or optionally substituted phenylthiocarbonyl group, an alkoxycarbonylalkyl group with up to 18, an aminocarbonyl group, an alkylcarbonylaminoalkylcarbonyl group with up to 18 or an aminocarbonylalkyl group with up to 18 carbon atoms in the alkyl chain or hydrogen, with the proviso that if $R^0$ represents hydrogen $R_1$ cannot simultaneously represent methyl, $R_3$ cannot simultaneously represent o-chlorophenyl and X and Y cannot both represent nitrogen;

Z represents branched or unbranched alkylene- or alkenylene group with n carbon atoms;

n represents 0, 1, 2, 3, 4, 5 or 6; in the case of an alkenylene group it represents 0, 2, 3, 4, 5 or 6;

X/Y independently of each other represents C-$R_1$ or N but cannot both represent C-$R_1$, or Y represents the group C-COOR', wherein R' represents alkyl or hydrogen and X represents nitrogen;

$R_1$ represents hydrogen, a branched or unbranched alkyl group with 1 to 4 carbon atoms which may optionally be substituted by hydroxy or halogen, a cyclopropyl or cyclopentyl group, a branched or unbranched alkoxy group with 1 to 4 carbon atoms, halogen,

$R_2$ represents hydroxy, formyl, carboxy, cyano, branched or unbranched alkyloxycarbonyl with 1 to 18 carbon atoms, whilst the alkyl chain may optionally be substituted by hydroxy,

amino, nitro or halogen, an optionally substituted phenyloxycarbonyl group;
a group of general formula

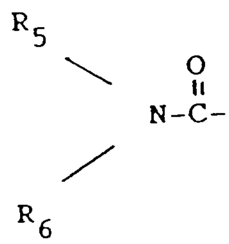

wherein $R_5$ and $R_6$, which may be identical or different, represent hydrogen, phenyl, substituted phenyl, a branched or unbranched alkyl-, alkenyl- or alkynyl group with up to 18 carbon atoms which may optionally be substituted by halogen, hydroxy, nitro, amino, alkoxy, or if $R_6$ = hydrogen or alkyl, by an acid amide of general formula

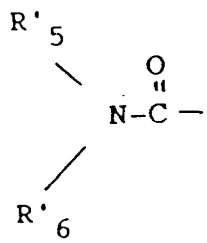

wherein $R'_5$ and $R'_6$ have the same meanings as $R_5$ and $R_6$ with the exception of an acid amide;
$R_5$ or $R_6$ represent a saturated or unsaturated 5- or 6-membered heterocyclic ring linked via a carbon, optionally mono- or polysubstituted by branched or unbranched alkyl with 1 to 4 carbon atoms;
or
$R_5$ and $R_6$ together with the nitrogen atom represent a saturated or unsaturated 5- or 6-membered ring optionally mono- or polysubstituted by branched or unbranched alkyl groups with 1 to 4 carbon atoms, which may contain, as further heteroatoms, nitrogen, oxygen or sulphur whilst each additional nitrogen atom may be substituted by a branched or unbranched alkyl group with 1 to 4 carbon atoms;

$R_2$ represents a group of general formula

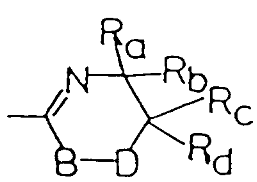

or

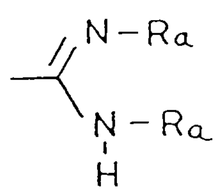

wherein
B represents oxygen, sulphur, NH or N-$C_{1-6}$-alkyl,
D represents the group $(CReRf)_n$ wherein n may be 0 to 3,
Ra represents hydrogen, alkyl with 1 to 6 carbon atoms, $C_{1-4}$-alkoxycarbonyl or dialkylaminocarbonyl,
Rb, Rc, Rd, Re and Rf represent hydrogen, alkyl with 1 to 6 carbon atoms, or phenyl;

$R_2$ represents hydrogen if A contains a carbonyl function or nitrogen as a ring member;

$R_2$ represents

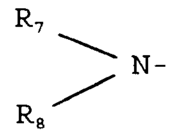

$R_7$ = hydrogen, $R_8$ = hydrogen, alkylcarbonyl or alkoxycarbonyl with 1 to 18, aminocarbonyl, alkylaminocarbonyl, dialkylaminocarbonyl with 1 to 18 in the alkyl chain;

$R_2$ represents hydrogen (for n > O);

$R_2$ represents halogen

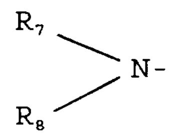

wherein $R_7$ and $R_8$, which may be identical or different, represent hydrogen, a branched or unbranched alkyl-, alkenyl- or alkynyl group with 1 to 18 carbon atoms which may optionally be substituted by halogen, hydroxy or a C-linked heterocyclic group, whilst the carbon chain may be interrupted by nitrogen, oxygen or sulphur, a branched or unbranched alkylcarbonyl group with 1 to 6 carbon atoms, optionally substituted by hydroxy or halogen, or substituted by an amino group optionally mono- or disubstituted by a branched or unbranched alkyl group with 1 to 6 carbon atoms, whilst the alkyl group may be substituted by halogen or hydroxy, an optionally substituted phenylcarbonyl group,

an optionally substituted phenylsulphonyl, an alkylsulphonyl group with 1 to 4 carbon atoms, or

$R_7$ and $R_8$ together with the nitrogen atom form a saturated or unsaturated 5- or 6-membered ring optionally mono- or polysubstituted by branched or unbranched alkyl groups with 1 to 4 carbon atoms, which may contain as further heteroatoms nitrogen, oxygen or sulphur, whilst each additional nitrogen atom may optionally be substituted by a branched or unbranched alkyl group with 1 to 4 carbon atoms;

$R_2$ represents phenylsulphonyloxy, optionally mono- or polysubstituted by branched or unbranched alkyl and/or alkoxy groups with 1 to 4 carbon atoms;

$R_2$ represents a branched or unbranched alkylsulphonyloxy group with 1 to 4 carbon atoms;

$R_2$ represents phenylcarbonyloxy, optionally mono- or polysubstituted by branched or unbranched alkyl- and/or alkoxy groups with 1 to 4 carbon atoms;

$R_2$ represents a branched or unbranched alkylcarbonyloxy group with 1 to 18 carbon atoms, whilst the alkyl chain may be interrupted by nitrogen, oxygen or sulphur;

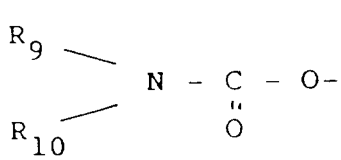 , 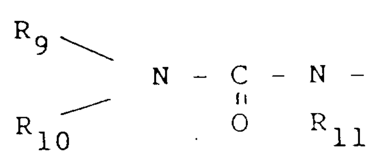

wherein $R_9$ represents hydrogen and $R_{10}$ an alkyl-, alkenyl- or alkynyl group with up to 4 carbon atoms, optionally substituted by halogen, a phenyl group optionally mono- or polysubstituted by branched or unbranched alkyl and/or alkoxy groups with 1 to 4 carbon atoms,

$R_{11}$ represents hydrogen or a branched or unbranched alkyl group with 1 to 4 carbon atoms;

$R_2$ represents an imido group, a benzimidazolyl group;

$R_2$ represents a branched or unbranched alkyloxy or alkylthio group with 1 to 18 carbon atoms, an optionally substituted phenyloxy or phenylthio group, a saturated or unsaturated 5- or 6-membered heterocyclic ring linked via oxygen or sulphur;

whilst, unless defined separately, $C_{1-4}$-alkyl, $C_{3-7}$-cycloalkyl, $C_{1-4}$-alkoxy, hydroxy or

halogen are given as substituents for a substituted phenyl group,

$R_3$ represents phenyl, wherein the phenyl ring may be mono- or polysubstituted by methyl, halogen, nitro and/or trifluoromethyl, or pyridyl,

and

$R_4$ may represent hydrogen, branched or unbranched alkyl with 1 to 4 carbon atoms or alkylcarbonyl with 1 to 4 carbon atoms,

optionally in the form of their racemates, enantiomers, diastereomers and mixtures thereof and optionally the physiologically harmless acid addition salts thereof,

characterised in that compounds of general formula

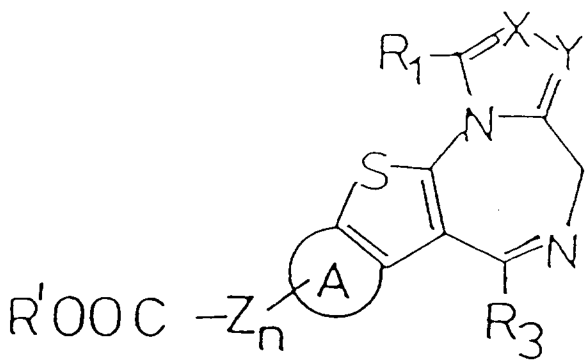

Ia1

wherein $R_1$ = hydrogen, alkyl, cycloalkyl, A, Z, n, X, Y and $R_3$ are defined as hereinbefore and R' represents a $C_{1-4}$-alkyl group, are reacted as follows:

1) compounds of general formula Ia wherein

$R_2$ = COOH

are obtained by saponification of compounds of formula Ia1;

2) compounds of general formula Ia wherein

$R_2 = R_5R_6$ NCO-

are obtained by reacting Ia with

$R_2$ = COOH

with an amine of formula

$HNR_5R_6$

optionally in the presence of sulphonyldimidazole, carbonyldiimidazole or dicyclohexylcarbodiimide;

3) compounds of general formula Ia with

$R_2$ = OH

are obtained by selective reduction of Ia', e.g. with lithium alanate or sodium borohydride, or, in the case of n = O, by reduction of the corresponding carbonyl compound;

4) compounds of general formula Ia wherein

$R_2 = R_{10}NH\text{-}COO\text{-}, R_{10}\text{-}CO\text{-}NR_{11}\text{-}$

are obtained by reacting the alcohol or amine, e.g. prepared as in 3), with an isocyanate of general formula

$R_{10}$ N=C=O;

5) compounds of general formula Ia wherein $R_2$ = alkyl or optionally substituted phenylcarbonyloxy are obtained by reacting the alcohol, e.g. prepared as in 3), with an acid equivalent, e.g. an acid halide, or a carboxylic acid of formula

R-COOH,

wherein R is an optionally substituted phenyl group or a branched or unbranched alkyl group with 1 to 8 carbon atoms, whilst the carbon chain may be interrupted by nitrogen, oxygen or sulphur;
6) compounds of general formula Ia wherein $R_2$ = alkylsulphonyloxy or phenylsulphonyloxy are obtained by reacting the alcohol, e.g. prepared as in 3) with an alkyl or phenylsulphonylhalide with the addition of an acid binding agent;
7) compounds of general formula Ia wherein

$R_2$ = $NR_7$ $R_8$

or an imido group
are obtained by reacting the mesylate ($R_2$ = $CH_3SO_3$-), e.g. prepared as in 6), with an amine of formula

$HNR_7R_8$

or an imide, optionally in the form of the alkali metal salts thereof;
8) compounds of general formula Ia wherein

$R_2$ = $NH_2$

may be obtained for example by cleaving phthalimide ($R_2$ = phthalic imide) or by Curtius degradation of the corresponding acid azide or by hydrolysis of the corresponding isocyanates, or by reduction of the corresponding azide;
9) compounds of general formula Ia wherein

$R_2$ = $NR_7$ $R_8$

wherein $R_7$ or $R_8$ is alkyl- or phenylcarbonyl are obtained by reacting the amine, e.g. prepared as in 8), with a carboxylic acid equivalent derived from a carboxylic acid of formula

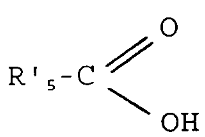

10) compounds of general formula Ia wherein

$R_2$ = formyl

are obtained by oxidation of the alcohol, e.g. as prepared in 3), shortening the chain from n to n-1;

11) compounds of general formula Ia wherein $R_2$ represents a group of general formula

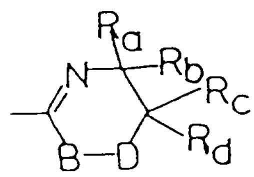

are obtained by reacting a compound of general formula Ia wherein

$R_2$ = COOH

with a bifunctionalised amine of general formula

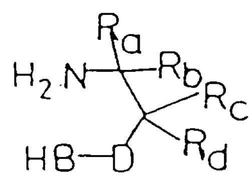

wherein $R_a$, $R_b$, $R_c$, $R_d$, D and B are defined as hereinbefore,
in the presence of triphenylphosphine, $CCl_4$ and a base;
11a) compounds of general formula Ia wherein $R_2$ is an optionally substituted thiazoline group, are obtained from the corresponding oxazolines, e.g. prepared as in 11) by sulphurisation, e.g. with phosphorus pentasulphide or Lawesson's reagent®.
12) compounds of general formula IA wherein $R_2$ represents an amidine of general formula

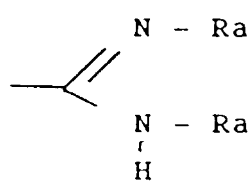

are obtained analogously to 11) by reacting with a primary amine of formula $H_2NRa$;
13) compounds of general formula Ia wherein

$R_2$ = CN

are obtained by reacting compounds of general formula Ia wherein

$R_2$ = $CONH_2$

with phosphorusoxychloride;
14) compounds of general formula Ia
wherein $R_2$ = a 2-imidazoline optionally substituted by branched or unbranched alkyl groups, is obtained by reacting compounds of general formula Ia with

$R_2$ = CN

a) with ethanolic HCl,

b) by reacting the resulting imidoethylester with an ethylenediamine optionally substituted by branched or unbranched alkyl groups,

c) optionally the free N-H function is alkylated with known alkylating agents;

15) compounds of general formula Ia wherein $R_2$ = alkyl- or phenyloxycarbonyl are obtained by reacting an acid equivalent of I ($R_2$ = COOH) with the corresponding alcohols;

16) compounds of general formula Ia wherein

$R_2$ = halogen

are obtained by reacting the tosylate

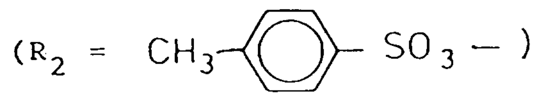

with a halogenating agent;

17) compounds of general formula I wherein $R_2$ represents an ether or thioether are obtained from the corresponding mesylates, e.g. prepared as in 6) ($R_2$ = $CH_3SO_3$), by reacting with the corresponding alcohols or mercaptanes as solvent or the alkali metal salts thereof in an inert organic solvent;

18) compounds of general formula Ia wherein A represents

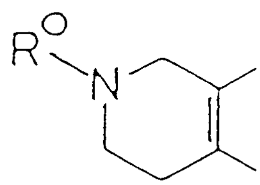

and R° represents alkyl, substituted alkyl, alkylcarbonyl, substituted alkylcarbonyl or arylcarbonyl are obtained by alkylation or acylation of compounds of general formula I wherein R° = hydrogen,

the corresponding thiocarbonyl compounds are obtained by reacting the carbonyl compound with the sulphur reagent, e.g. phosphorus pentasulphide;

and subsequently, if desired, the resulting compounds Ia wherein $R_1$ = hydrogen are reacted in the presence of a base with chlorine or bromine to yield a compound of general formula Ia wherein $R_1$ = chlorine or bromine;

and subsequently, if desired, the halogen compound is converted into a compound of general formula Ia wherein $R_1$ = alkoxy with 1 to 4 carbon atoms by reacting with the corresponding alcoholate;

subsequently if desired a compound of general formula Ia is selectively reduced and if desired the resulting compound Ib wherein $R_4$ represents hydrogen is alkylated or acylated and optionally converted into the pharmacologically acceptable acid addition salts thereof and subsequently, if desired, resolved into its optically active compounds.

**9.** Compounds of general formula

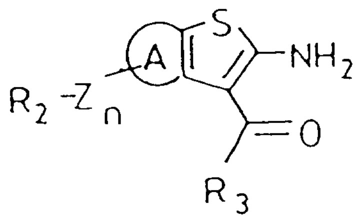

wherein

- A represents a fused mono-unsaturated 5-, 6- or 7-membered carbocyclic ring,
- Z represents a branched or unbranched alkylene or alkenylene group;
- n represents 0, 1, 2, 3, 4, 5 or 6; if it is an alkenylene group it represents 0, 2, 3, 4, 5 or 6;
- $R_2$ represents COOR*, wherein R* = hydrogen or $C_{1-4}$-alkyl; or
- $R_2$ represents $R_5R_6N$-CO-
- $R_2$ represents $-NR_7R_8$

  or
- $R_2$ represents hydrogen (if n > O), hydroxy, alkylcarbonyloxy, an ether or thioether group, alkyl or phenylsulphonyloxy as defined in one of the preceding claims, and
- $R_3$ may represent phenyl, optionally mono- or polysubstituted by halogen, methyl, nitro and/or trifluoromethyl.

**10.** Compounds of general formula

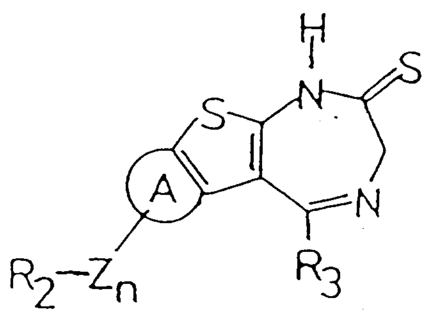

wherein A, Z, n and $R_3$ are defined as in one of the preceding claims and

- $R_2$ represents hydrogen (if n > O), hydroxy,
- $R_2$ represents COOR* wherein R* = hydrogen or $C_{1-4}$-alkyl, or
- $R_2$ represents $R_5R_6N$-CO- wherein $R_5$ and $R_6$ are as hereinbefore defined;
- $R_2$ represents $-NR_7R_8$ wherein $R_7$ and $R_8$ are as hereinbefore defined;

  or
- $R_2$ represents an alkylcarbonyloxy, an ether or thioether group, an amine, alkyl- or phenylsulphonyloxy group as defined in one of the preceding claims.

**11.** Pharmaceutical preparations containing as active substances compounds of general formula Ia or Ib as claimed in claim 1, combined with conventional excipients and/or carriers.

**12.** Process for preparing pharmaceutical preparations as claimed in claim 11, characterised in that compounds of general formula Ia or Ib are processed with conventional galenic excipients and/or carriers to produce pharmaceutical preparations.

**13.** Compounds of general formula Ia or Ib as claimed in claim 1 or 2 for use in the preparation of pharmaceutical compositions with a PAF-antagonistic activity.

**14.** Use of compounds of general formula Ia as claimed in claim 1, 2, 3, 4, 5 or 6 for preparing a pharmaceutical composition for treating bronchial asthma.

## Revendications

**1.** Composés de formule générale I

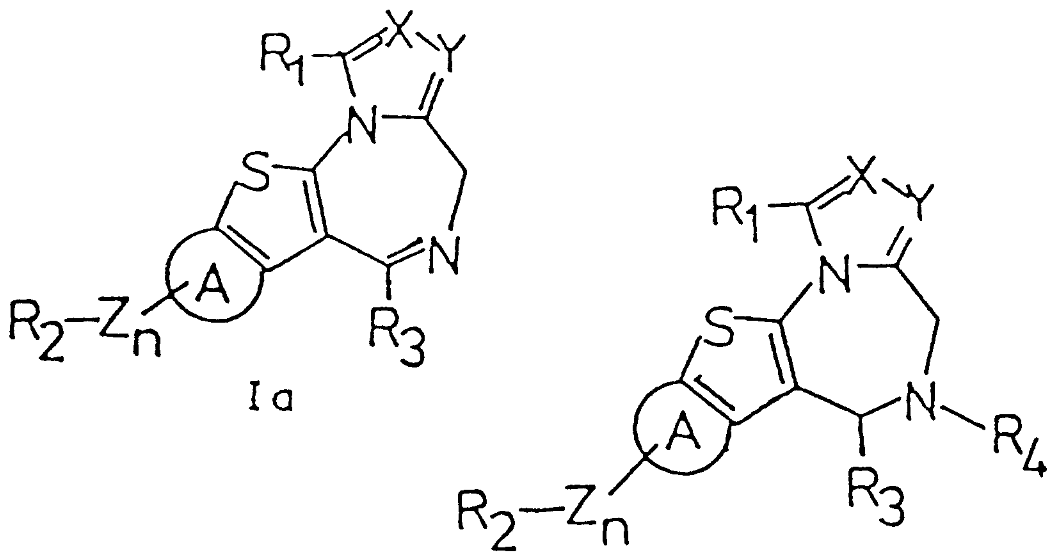

Ia

Ib

dans laquelle
A représente un cycle condensé mono-insaturé à 5, 6 ou 7 sommets, dans lequel, lorsque n = 0 et $R_2$ représente l'hydrogène, un atome de carbone peut être remplacé par C=O, ou bien A représente un cycle condensé de formule

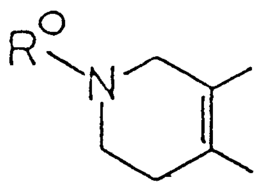

dans laquelle $R^o$ peut représenter un groupe alkyle ramifié ou non ramifié de 1 à 18 atomes de carbone, un groupe alkylcarbonyle ou alkylthiocarbonyle de 1 à 18 atomes de carbone dans la chaîne alkyle ou un groupe phénylcarbonyle éventuellement substitué ou phénylthiocarbonyle éventuellement substitué, un groupe alcoxycarbonylalkyle ayant jusqu'à 18 atomes de carbone, un groupe aminocarbonyle, un groupe alkylcarbonylaminoalkyle ayant jusqu'à 18 atomes de carbone, un groupe alkylcarbonylaminoalkylcarbonyle ayant jusqu'à 18 atomes de carbone ou un groupe aminocarbonylalkyle ayant jusqu'à 18 atomes de carbone dans la chaîne alkyle ou l'hydrogène, avec la condition que lorsque $R^o$ représente l'hydrogène $R_1$ ne représente pas en même temps un groupe méthyle, $R_3$ ne représente pas un groupe o-chlorophényle et X et Y ne représentent pas l'un et l'autre un atome d'azote;
Z peut représenter un groupe alkylène ou alcénylène ramifié ou non ramifié ayant n atomes de carbone;
n peut représenter 0, 1, 2, 3, 4, 5 ou 6; dans le cas d'un groupe alcénylène 0, 2, 3, 4, 5 ou 6;
X/Y peuvent représenter indépendamment l'un de l'autre C-$R_1$ ou N mais pas tous les deux C-$R_1$,
ou bien Y représente le groupe C-COOR' dans lequel R' représente un groupe alkyle ou un atome d'hydrogène et X représente un atome d'azote;
$R_1$ peut représenter un atome d'hydrogène, un groupe alkyle ramifié ou non ramifié de 1 à 4 atomes de carbone, qui peut éventuellement être substitué par hydroxyle ou halogène, un groupe cyclopropyle

ou cyclopentyle, un groupe alcoxy ramifié ou non ramifié de 1 à 4 atomes de carbone, un halogène,
$R_2$ peut représenter un groupe hydroxyle, formyle, carboxyle, cyano, alkyloxycarbonyle ramifié ou non ramifié de 1 à 18 atomes de carbone, dans lequel la chaîne alkyle peut éventuellement être substituée par hydroxyle, amino, nitro ou halogène, un groupe phényloxycarbonyle éventuellement substitué;
un reste de formule générale

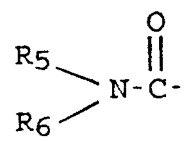

dans laquelle $R_5$ et $R_6$, qui peuvent être identiques ou différents, peuvent représenter l'hydrogène, un groupe phényle, phényle substitué, un groupe alkyle, alcényle ou alcynyle ramifié ou non ramifié ayant jusqu'à 18 atomes de carbone, qui peut éventuellement être substitué par halogène, hydroxyle, nitro, amino, alcoxy, ou dans le cas où $R_6$ = hydrogène ou alkyle, par un amide d'acide de formule générale

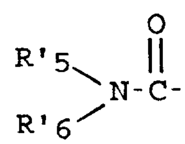

dans laquelle $R'_5$ et $R'_6$ peuvent avoir la même signification que $R_5$ et $R_6$, mais à l'exception d'un amide d'acide;
$R_5$ ou $R_6$ peut représenter un hétérocycle saturé ou insaturé à 5 ou 6 sommets, éventuellement substitué une ou plusieurs fois par un groupe alkyle ramifié ou non ramifié de 1 à 4 atomes de carbone, relié par l'intermédiaire d'un atome de carbone;
ou bien
$R_5$ et $R_6$ peuvent former avec l'atome d'azote un cycle saturé ou insaturé à 5 ou 6 sommets, éventuellement substitué une ou plusieurs fois par des groupes alkyle ramifiés ou non ramifiés de 1 à 4 atomes de carbone, qui peut contenir comme hétéroatomes supplémentaires l'azote, l'oxygène ou le soufre, chaque atome d'azote supplémentaire pouvant être substitué par un groupe alkyle ramifié ou non ramifié de 1 à 4 atomes de carbone;
$R_2$ peut représenter un reste de formule générale

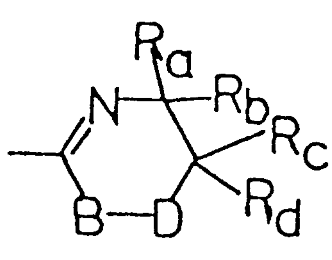

ou

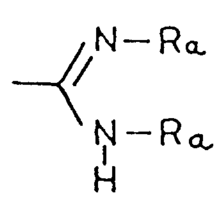

dans laquelle
B peut représenter un atome d'oxygène, de soufre, NH ou un groupe N-alkyle en $C_1$-$C_6$,
D peut représenter le reste $(CR_eR_f)_n$ où n peut être compris entre 0 et 3,
$R_a$ peut représenter l'hydrogène, un groupe alkyle de 1 à 6 atomes de carbone, alcoxycarbonyle en $C_1$ à $C_4$ ou dialkylaminocarbonyle,
$R_b$, $R_c$, $R_d$, $R_e$, $R_f$ peuvent représenter l'hydrogène, un groupe alkyle de 1 à 6 atomes de carbone, ou un groupe phényle;
$R_2$ peut représenter l'hydrogène, lorsque A contient une fonction carbonyle ou l'azote comme chaînon du cycle;
$R_2$ peut représenter

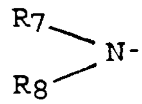

$R_7$ = hydrogène, $R_8$ = hydrogène, un groupe alkylcarbonyle ou alcoxycarbonyle de 1 à 18 atomes de carbone, aminocarbonyle, alkylaminocarbonyle, dialkylaminocarbonyle de 1 à 18 atomes de carbone dans la chaîne alkyle;
$R_2$ peut représenter l'hydrogène (pour n > 0);
$R_2$ peut représenter un halogène,

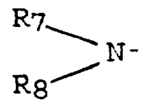

où $R_7$ et $R_8$, qui peuvent être identiques ou différents, peuvent représenter l'hydrogène, un groupe alkyle, alcényle ou alcynyle ramifié ou non ramifié ayant jusqu'à 18 atomes de carbone, qui peut éventuellement être substitué par halogène, hydroxyle ou un hétérocycle relié par un atome de carbone, la chaîne carbonée pouvant être interrompue par l'azote, l'oxygène ou le soufre, un groupe alkylcarbonyle ramifié ou non ramifié de 1 à 6 atomes de carbone, éventuellement substitué par hydroxyle ou halogène, ou substitué par un groupe amino éventuellement substitué une ou deux fois par un groupe alkyle ramifié ou non ramifié de 1 à 6 atomes de carbone, le groupe alkyle pouvant être substitué par halogène ou hydroxyle, un groupe phénylcarbonyle éventuellement substitué, un groupe phénylsulfonyle éventuellement substitué, un groupe alkylsulfonyle de 1 à 4 atomes de carbone,
ou bien
$R_7$ et $R_8$ forment avec l'atome d'azote un cycle saturé ou insaturé à 5 ou 6 sommets, éventuellement substitué une ou plusieurs fois par des groupes alkyle ramifiés ou non ramifiés de 1 à 4 atomes de carbone, qui peut contenir comme hétéroatomes supplémentaires l'azote, l'oxygène ou le soufre, chaque atome d'azote supplémentaire pouvant éventuellement être substitué par un groupe alkyle ramifié ou non ramifié de 1 à 4 atomes de carbone;
$R_2$ peut représenter un groupe phénylsulfonyloxy, éventuellement substitué une ou plusieurs fois par des groupes alkyle et/ou alcoxy ramifiés ou non ramifiés de 1 à 4 atomes de carbone;
$R_2$ peut représenter un groupe alkylsulfonyloxy ramifié ou non ramifié de 1 à 4 atomes de carbone;
$R_2$ peut représenter un groupe phénylcarbonyloxy, éventuellement substitué une ou plusieurs fois par des groupes alkyle et/ou alcoxy ramifiés ou non ramifiés de 1 à 4 atomes de carbone;
$R_2$ peut représenter un groupe alkylcarbonyloxy ramifié ou non ramifié de 1 à 18 atomes de carbone, la chaîne alkyle pouvant être interrompue par l'azote, l'oxygène ou le soufre;

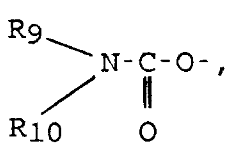

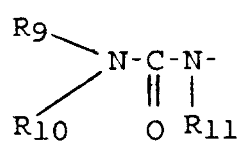

où $R_9$ peut représenter l'hydrogène et $R_{10}$ peut représenter un groupe alkyle, alcényle ou alcynyle de 1 à 4 atomes de carbone, éventuellement substitué par halogène, un groupe phényle éventuellement substitué une ou plusieurs fois par des groupes alkyle et/ou alcoxy ramifiés ou non ramifiés de 1 à 4 atomes de carbone,
$R_{11}$ peut représenter l'hydrogène ou un groupe alkyle ramifié ou non ramifié de 1 à 4 atomes de carbone;
$R_2$ peut représenter un reste imido, un reste benzimidazolyle;
$R_2$ peut représenter un reste alkyloxy ou alkylthio ramifié ou non ramifié de 1 à 18 atomes de carbone, un reste phényloxy ou phénylthio éventuellement substitué, un hétérocycle saturé ou insaturé à 5 ou 6 sommets, relié par l'oxygène ou le soufre;
les substituants indiqués pour un reste phényle substitué, sauf définition particulière, étant alkyle en $C_1$-

$C_4$, cycloalkyle en $C_3$-$C_7$, alcoxy en $C_1$-$C_4$, hydroxyle ou halogène,
$R_3$ peut représenter un groupe phényle dans lequel le cycle phényle peut être substitué une ou plusieurs fois par méthyle, halogène, nitro et/ou trifluorométhyle, ou un groupe pyridyle,
et
$R_4$ peut représenter l'hydrogène, un groupe alkyle ramifié ou non ramifié de 1 à 4 atomes de carbone ou un groupe alkylcarbonyle de 1 à 4 atomes de carbone,
éventuellement sous forme de leurs racémates, de leurs énantiomères, de leurs diastéréoisomères et de leurs mélanges et éventuellement de leurs sels d'addition d'acide acceptables du point de vue physiologique.

**2.** Composés de formule générale Ia selon la revendication 1, où A peut représenter un cycle condensé mono-insaturé à 5 ou 6 sommets, ou bien A peut représenter un cycle condensé de formule

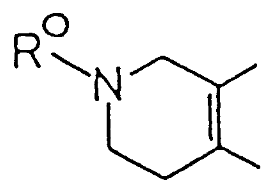

dans laquelle R° peut représenter un groupe acétylaminoacétyle, acétyle, thioacétyle, éthoxycarbonylméthyle, méthoxycarbonylméthyle, morpholinylcarbonylméthyle, diéthylaminocarbonylméthyle;
Z peut représenter un groupe alkylène non ramifié ayant n atomes de carbone;
n peut représenter 0, 1, 2, 3 ou 4;
X/Y peuvent représenter tous les deux N ou X = C-H ou C-$CH_3$ et Y = N,
$R_1$ peut représenter l'hydrogène, un groupe hydroxyméthyle, chlorométhyle, cyclopropyle, éthyle, méthoxy, éthoxy, le chlore, le brome ou un groupe méthyle;
$R_2$ peut représenter un groupe hydroxyle, carboxyle, cyano, le brome, un groupe alkyloxycarbonyle de 1 à 6 atomes de carbone,
$R_2$ peut représenter un reste de formule générale

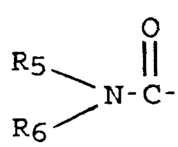

dans laquelle $R_5$ et $R_6$, qui peuvent être identiques ou différents, peuvent représenter l'hydrogène, un groupe alkyle ou alcényle ramifié ou non ramifié ayant jusqu'à 6, 8 ou 16 atomes de carbone, qui peut éventuellement être substitué par halogène, hydroxyle, méthoxy, nitro, amino, alkylamino ou dialkylamino de 1 à 4 atomes de carbone dans la chaîne alkyle, ou dans le cas où $R_6$ = hydrogène ou alkyle, par un groupe morpholinylcarbonyle ou diéthylaminocarbonyle,
lorsque $R_5$ = hydrogène ou méthyle, $R_6$ peut représenter un reste thiazoline ou thiazole qui peut éventuellement être substitué par un groupe alkyle ramifié ou non ramifié de 1 à 4 atomes de carbone, ou bien
$R_5$ et $R_6$ forment avec l'atome d'azote un reste morpholino ou pipérazino qui peut éventuellement être substitué une ou plusieurs fois par un groupe méthyle;
$R_2$ peut représenter un reste $\Delta^2$-imidazoline, -thiazoline, -oxazoline ou tétrahydropyrimidine relié par un atome de carbone, qui peut éventuellement être substitué une ou plusieurs fois par alkyle de 1 à 4 atomes de carbone;
$R_2$ peut représenter l'hydrogène, dans le cas où n=0, lorsque A contient une fonction carbonyle ou contient l'azote comme chaînon du cycle

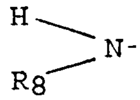

$R_8$ peut représenter un groupe alkyloxycarbonyle de 1 à 4 atomes de carbone;
dans le cas où n > 0
$R_2$ peut représenter un groupe alkylcarbonyloxy de 1 à 3 atomes de carbone;
$R_2$ peut représenter un groupe alkylsulfonyloxy de 1 à 2 atomes de carbone;
$R_2$ peut représenter

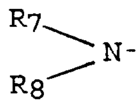

où $R_7$ et $R_8$, qui peuvent être identiques ou différents, peuvent représenter l'hydrogène, un groupe alkyle ramifié ou non ramifié de 1 à 6 atomes de carbone, éventuellement substitué par dialkylamino de 1 à 4 atomes de carbone, morpholino ou N-alkylpipérazino ou un reste indole, un groupe alkylcarbonyle de 1 à 4 atomes de carbone,
ou bien
$R_7$ et $R_8$ peuvent former avec l'atome d'azote un reste morpholino ou pipérazino qui peut éventuellement être substitué une ou plusieurs fois par méthyle, un reste triazolo, un reste imidazolo, un reste pyrazolo, un reste pyrrolo, un reste imido,
$R_2$ peut représenter un groupe alkylsulfonyloxy ramifié ou non ramifié de 1 à 4 atomes de carbone, un groupe alkylcarbonyloxy ramifié ou non ramifié de 1 à 8 atomes de carbone,
$R_2$ peut représenter un reste phényloxy, 3,4-méthylènedioxyphénoxy, un reste pyridinyloxy, un reste alkyloxy ou alkylthio de 1 à 4 atomes de carbone;
$R_3$ peut représenter un groupe phényle ou o-chlorophényle, et éventuellement leurs sels d'addition d'acide acceptables du point de vue physiologique et leurs composés optiquement actifs.

**3.** Composés de formule générale Ia selon la revendication 1, où A peut représenter un cycle condensé mono-insaturé à 5 ou 6 sommets, substitué en position 3 ou 4 de l'hétrazépine,
Z peut représenter un groupe alkylène non ramifié ayant n atomes de carbone;
n peut représenter 0, 1 ou 2;
X/Y peuvent représenter tous les deux N ou X = C-H ou $C-CH_3$ et Y = N,
$R_1$ peut représenter l'hydrogène, un groupe cyclopropyle, méthoxy, le brome, un groupe méthyle;
$R_2$ peut représenter un groupe hydroxyle, amino, carboxyle, cyano, méthoxycarbonyle, éthoxycarbonyle,
$R_2$ peut représenter un reste de formule générale

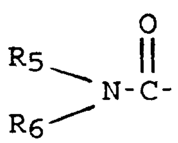

dans laquelle $R_5$ et $R_6$, qui peuvent être identiques ou différents, peuvent représenter l'hydrogène, un groupe propényle, phényle, un groupe alkyle ramifié ou non ramifié ayant 1 à 6, 8 ou 16 atomes de carbone, qui peut éventuellement être substitué par halogène, hydroxyle, nitro, amino, éthylamino ou diéthylamino, méthoxy, ou dans le cas où $R_6$ = hydrogène ou alkyle, par un groupe morpholinylcarbonyle ou diéthylaminocarbonyle,
lorsque $R_5$ = hydrogène ou méthyle, $R_6$ peut représenter un reste thiazoline ou thiazole qui peut éventuellement être substitué par méthyle,
ou bien
$R_5$ et $R_6$ forment avec l'atome d'azote un reste morpholino ou pipérazino qui peut éventuellement être

substitué une ou plusieurs fois par méthyle;
$R_2$ peut représenter un reste $\Delta^2$-imidazoline, -thiazoline, -oxazoline relié par un atome de carbone, qui peut éventuellement être substitué une ou plusieurs fois par méthyle, éthyle et/ou isopropyle, un cycle tétrahydropyrimidine, éventuellement substitué une ou plusieurs fois par méthyle, un reste benzimidazole, un reste indole, ou méthoxycarbonylamino;
dans le cas où n > 0
$R_2$ peut représenter un groupe acétoxy, un groupe méthanesulfonyloxy,

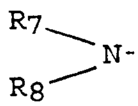

où $R_7$ et $R_8$, qui peuvent être identiques ou différents, peuvent représenter l'hydrogène, un groupe alkyle ramifié ou non ramifié de 1 à 4 atomes de carbone, qui peut être substitué par diéthylamino ou morpholino, un groupe acétyle ou bien
$R_7$ et $R_8$ peuvent former avec l'atome d'azote un reste morpholino ou pipérazino qui peut éventuellement être substitué une ou plusieurs fois par méthyle, un reste triazolo, un reste imidazolo, un reste phtalimide,
$R_2$ peut représenter un groupe alkylsulfonyloxy ramifié ou non ramifié de 1 à 4 atomes de carbone, un groupe alkylcarbonyloxy ramifié ou non ramifié de 1 à 8 atomes de carbone,
$R_2$ peut représenter un reste phényloxy, un reste pyridyloxy, 3,4-méthylènedioxyphénoxy, un groupe 1,2,4-triazol-3-ylthio, méthoxy;
$R_3$ peut représenter un groupe phényle ou o-chlorophényle, et éventuellement leurs sels d'addition d'acide acceptables du point de vue physiologique et éventuellement leurs composés optiquement actifs.

**4.** Composés selon l'une des revendications 1, 2 ou 3, caractérisés en ce que X et Y représentent tous les deux l'azote, $R_1$ représente un groupe méthyle et $R_3$ représente un groupe ortho-chlorophényle.

**5.** 3-(morpholin-4-yl-carbonyl)-5-(2-chlorophényl)-10-méthyl-3,4-dihydro-2H,7H-cyclopenta[4,5]thiéno[3,2-f]-[1,2,4]triazolo[4,3-a][1,4]diazépine.

**6.** 3-(dipropylaminocarbonyl)-5-(2-chlorophényl)-10-méthyl-3,4-dihydro-2H,7H-cyclopenta[4,5]thiéno[3,2-f]-[1,2,4]triazolo[4,3-a][1,4]diazépine.

**7.** Procédé de préparation de composés de formule générale Ia selon l'une des revendications 1 à 4, où $R_1$, $R_3$, A, Z et n sont tels que définis précédemment, $R_2$ représente l'hydrogène ou -COOR', avec R' = alkyle en $C_1$-$C_4$ ou l'hydrogène, ou bien $R_2$ représente un groupe alkylcarbonyloxy, un reste éther ou thioéther, un amide d'acide ou une amine, comme défini précédemment,
caractérisé en ce que l'on fait réagir un composé correspondant de formule générale

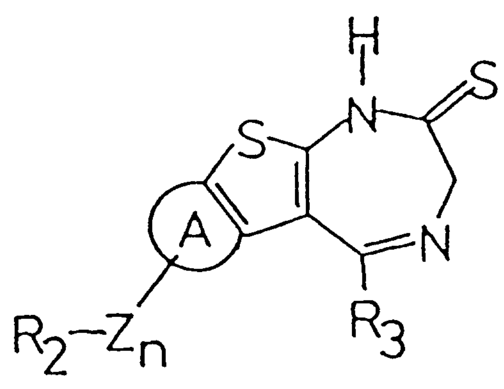

II

dans laquelle $R_2$, $R_3$, A, Z et n ont la signification citée précédemment
A) lorsque X et Y représentent l'azote

a) avec un hydrazide d'acide de formule générale

$R_1$-CONHNH$_2$

b) ou bien avec l'hydrazine pour former un composé de formule générale

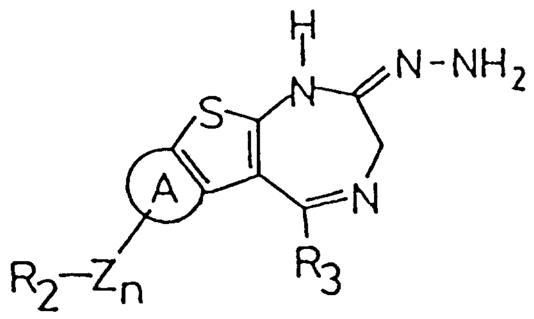

IV

puis on fait réagir avec un halogénure d'acide de formule générale

$R_1$-CO-Hal

ou avec un orthoester de formule générale

$R_1$-C(OR')$_3$

dans laquelle R' représente un groupe alkyle en $C_1$-$C_4$, ou bien

B) lorsque X représente C-H et Y l'azote

a) avec un aminoalcyne de formule générale

$R_{11}$-C≡C-CH$_2$-NH$_2$

dans laquelle $R_{11}$ représente l'hydrogène ou un groupe alkyle en $C_1$-$C_4$, ou bien

b) avec un alkylacétal d'α-aminoaldéhyde ou un alkylcétal d'α-aminocétone de formule générale

$H_2NCH_2$-CR$_1$(OR')$_2$

dans laquelle $R_1$ représente l'hydrogène ou un groupe alkyle de 1 à 4 atomes de carbone et R' représente un groupe alkyle en $C_1$-$C_4$

C) lorsque X représente l'azote et Y représente C-H on décarboxyle un composé de formule générale

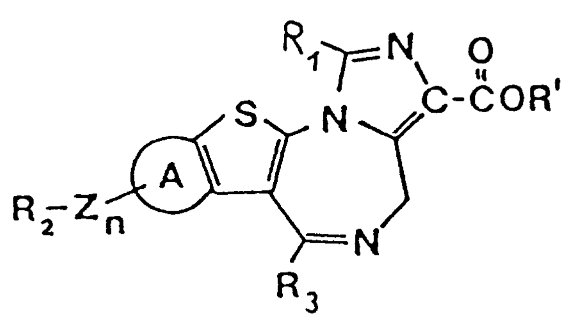

R' = alkyle en $C_1$-$C_4$

**8.** Procédé de préparation de composés de formule générale

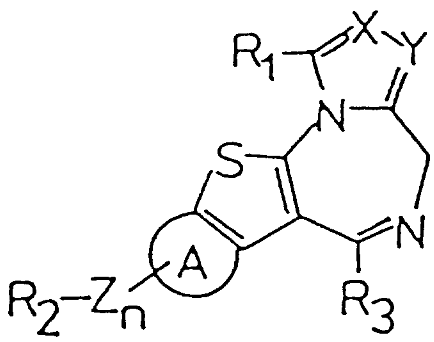

I a

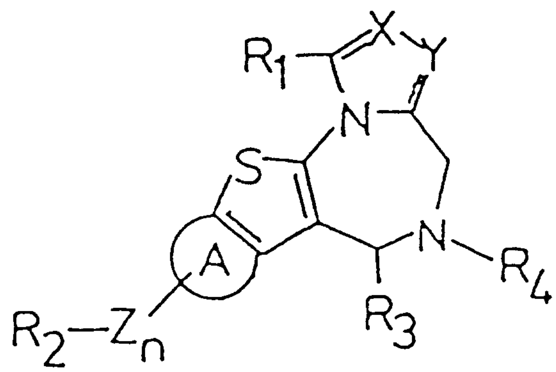

I b

dans laquelle
A représente un cycle condensé mono-insaturé à 5, 6 ou 7 sommets, dans lequel, lorsque n = 0 et $R_2$ représente l'hydrogène, un atome de carbone peut être remplacé par C=O, ou bien A représente un cycle condensé de formule

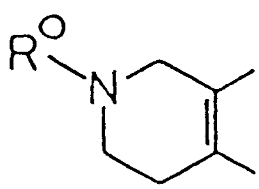

dans laquelle $R^o$ représente un groupe alkyle ramifié ou non ramifié de 1 à 18 atomes de carbone, un groupe alkylcarbonyle ou alkylthiocarbonyle de 1 à 18 atomes de carbone dans la chaîne alkyle ou un groupe phénylcarbonyle éventuellement substitué ou un groupe phénylthiocarbonyle éventuellement substitué, un groupe alcoxycarbonylalkyle ayant jusqu'à 18 atomes de carbone, un groupe aminocarbonyle, un groupe alkylcarbonylaminoalkyle ayant jusqu'à 18 atomes de carbone, un groupe alkylcarbonylaminoalkylcarbonyle ayant jusqu'à 18 atomes de carbone ou un groupe aminocarbonylalkyle ayant jusqu'à 18 atomes de carbone dans la chaîne alkyle ou l'hydrogène, avec la condition que lorsque $R^o$ représente l'hydrogène $R_1$ ne représente pas en même temps un groupe méthyle, $R_3$ ne représente pas un groupe o-chlorophényle et X et Y ne représentent pas l'un et l'autre un atome d'azote;
Z peut représenter un groupe alkylène ou alcénylène ramifié ou non ramifié ayant n atomes de carbone;
n peut représenter 0, 1, 2, 3, 4, 5 ou 6; dans le cas d'un groupe alcénylène 0, 2, 3, 4, 5 ou 6;
X/Y peuvent représenter indépendamment l'un de l'autre C-$R_1$ ou N mais pas tous les deux C-$R_1$,
ou bien Y représente le groupe C-COOR' dans lequel R' représente un groupe alkyle ou un atome d'hydrogène et X représente un atome d'azote,
$R_1$ peut représenter un atome d'hydrogène, un groupe alkyle ramifié ou non ramifié de 1 à 4 atomes de carbone, qui peut éventuellement être substitué par hydroxyle ou halogène, un groupe cyclopropyle ou cyclopentyle, un groupe alcoxy ramifié ou non ramifié de 1 à 4 atomes de carbone, un halogène,
$R_2$ peut représenter un groupe hydroxyle, formyle, carboxyle, cyano, alkyloxycarbonyle ramifié ou non ramifié de 1 à 18 atomes de carbone, dans lequel la chaîne alkyle peut éventuellement être substituée par hydroxyle, amino, nitro ou halogène, un groupe phényloxycarbonyle éventuellement substitué;
un reste de formule générale

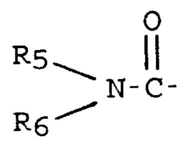

dans laquelle $R_5$ et $R_6$, qui peuvent être identiques ou différents, peuvent représenter l'hydrogène, un groupe phényle, phényle substitué, un groupe alkyle, alcényle ou alcynyle ramifié ou non ramifié ayant jusqu'à 18 atomes de carbone, qui peut éventuellement être substitué par halogène, hydroxyle, nitro, amino, alcoxy, ou dans le cas où $R_6$ = hydrogène ou alkyle, par un amide d'acide de formule générale

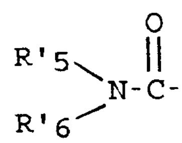

dans laquelle $R'_5$ et $R'_6$ peuvent avoir la même signification que $R_5$ et $R_6$, mais à l'exception d'un amide d'acide;

$R_5$ ou $R_6$ peut représenter un hétérocycle saturé ou insaturé à 5 ou 6 sommets, éventuellement substitué une ou plusieurs fois par un groupe alkyle ramifié ou non ramifié de 1 à 4 atomes de carbone, relié par l'intermédiaire d'un atome de carbone;

ou bien

$R_5$ et $R_6$ forment avec l'atome d'azote un cycle saturé ou insaturé à 5 ou 6 sommets, éventuellement substitué une ou plusieurs fois par des groupes alkyle ramifiés ou non ramifiés de 1 à 4 atomes de carbone, qui peut contenir comme hétéroatomes supplémentaires l'azote, l'oxygène ou le soufre, chaque atome d'azote supplémentaire pouvant être substitué par un groupe alkyle ramifié ou non ramifié de 1 à 4 atomes de carbone;

$R_2$ peut représenter un reste de formule générale

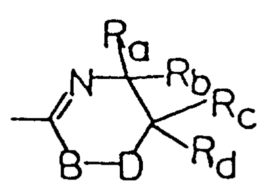

ou

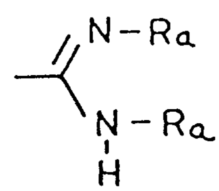

dans laquelle

B peut représenter un atome d'oxygène, de soufre, NH ou un groupe N-alkyle en $C_1$-$C_6$,

D peut représenter le reste $(CR_eR_f)_n$ où n peut être compris entre 0 et 3,

$R_a$ peut représenter l'hydrogène, un groupe alkyle de 1 à 6 atomes de carbone, alcoxycarbonyle en $C_1$ à $C_4$ ou dialkylaminocarbonyle,

$R_b$, $R_c$, $R_d$, $R_e$, $R_f$ peuvent représenter l'hydrogène, un groupe alkyle de 1 à 6 atomes de carbone, ou un groupe phényle;

$R_2$ peut représenter l'hydrogène, lorsque A contient une fonction carbonyle ou l'azote comme chaînon du cycle;

$R_2$ peut représenter

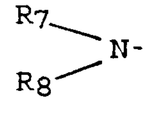

$R_7$ = hydrogène, $R_8$ = hydrogène, un groupe alkylcarbonyle ou alcoxycarbonyle de 1 à 18 atomes de carbone, aminocarbonyle, alkylaminocarbonyle, dialkylaminocarbonyle de 1 à 18 atomes de carbone dans la chaîne alkyle;
$R_2$ peut représenter l'hydrogène (pour n > 0);
$R_2$ peut représenter un halogène,

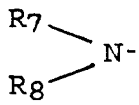

où $R_7$ et $R_8$, qui peuvent être identiques ou différents, peuvent représenter l'hydrogène, un groupe alkyle, alcényle ou alcynyle ramifié ou non ramifié ayant jusqu'à 18 atomes de carbone, qui peut éventuellement être substitué par halogène, hydroxyle ou un hétérocycle relié par un atome de carbone, la chaîne carbonée pouvant être interrompue par l'azote, l'oxygène ou le soufre, un groupe alkylcarbonyle ramifié ou non ramifié de 1 à 6 atomes de carbone, éventuellement substitué par hydroxyle ou halogène, ou substitué par un groupe amino éventuellement substitué une ou deux fois par un groupe alkyle ramifié ou non ramifié de 1 à 6 atomes de carbone, le groupe alkyle pouvant être substitué par halogène ou hydroxyle, un groupe phénylcarbonyle éventuellement substitué, un groupe phénylsulfonyle éventuellement substitué, un groupe alkylsulfonyle de 1 à 4 atomes de carbone,
ou bien
$R_7$ et $R_8$ forment avec l'atome d'azote un cycle saturé ou insaturé à 5 ou 6 sommets, éventuellement substitué une ou plusieurs fois par des groupes alkyle ramifiés ou non ramifiés de 1 à 4 atomes de carbone, qui peut contenir comme hétéroatomes supplémentaires l'azote, l'oxygène ou le soufre, chaque atome d'azote supplémentaire pouvant éventuellement être substitué par un groupe alkyle ramifié ou non ramifié de 1 à 4 atomes de carbone;
$R_2$ peut représenter un groupe phénylsulfonyloxy, éventuellement substitué une ou plusieurs fois par des groupes alkyle et/ou alcoxy ramifiés ou non ramifiés de 1 à 4 atomes de carbone;
$R_2$ peut représenter un groupe alkylsulfonyloxy ramifié ou non ramifié de 1 à 4 atomes de carbone;
$R_2$ peut représenter un groupe phénylcarbonyloxy, éventuellement substitué une ou plusieurs fois par des groupes alkyle et/ou alcoxy ramifiés ou non ramifiés de 1 à 4 atomes de carbone;
$R_2$ peut représenter un groupe alkylcarbonyloxy ramifié ou non ramifié de 1 à 18 atomes de carbone, la chaîne alkyle pouvant être interrompue par l'azote, l'oxygène ou le soufre;

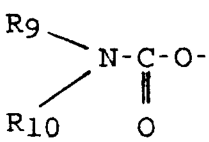,

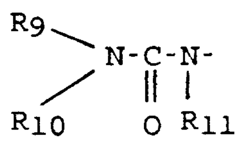

où $R_9$ peut représenter l'hydrogène et $R_{10}$ peut représenter un groupe alkyle, alcényle ou alcynyle de 1 à 4 atomes de carbone, éventuellement substitué par halogène, un groupe phényle éventuellement substitué une ou plusieurs fois par des groupes alkyle et/ou alcoxy ramifiés ou non ramifiés de 1 à 4 atomes de carbone,
$R_{11}$ peut représenter l'hydrogène ou un groupe alkyle ramifié ou non ramifié de 1 à 4 atomes de carbone;
$R_2$ peut représenter un reste imido, un reste benzimidazolyle;
$R_2$ peut représenter un reste alkyloxy ou alkylthio ramifié ou non ramifié de 1 à 18 atomes de carbone, un reste phényloxy ou phénylthio éventuellement substitué, un hétérocycle saturé ou insaturé à 5 ou 6 sommets, relié par l'oxygène ou le soufre;
les substituants indiqués pour un reste phényle substitué, sauf définition particulière, étant alkyle en $C_1$-$C_4$, cycloalkyle en $C_3$-$C_7$, alcoxy en $C_1$-$C_4$, hydroxyle ou halogène,
$R_3$ peut représenter un groupe phényle dans lequel le cycle phényle peut être substitué une ou plusieurs fois par méthyle, halogène, nitro et/ou trifluorométhyle, ou un groupe pyridyle,
et

$R_4$ peut représenter l'hydrogène, un groupe alkyle ramifié ou non ramifié de 1 à 4 atomes de carbone ou un groupe alkylcarbonyle de 1 à 4 atomes de carbone, éventuellement sous forme de leurs racémates, de leurs énantiomères, de leurs diastéréoisomères et de leurs mélanges et éventuellement de leurs sels d'addition d'acide acceptables du point de vue physiologique,
caractérisé en ce que l'on fait réagir des composés de formule générale

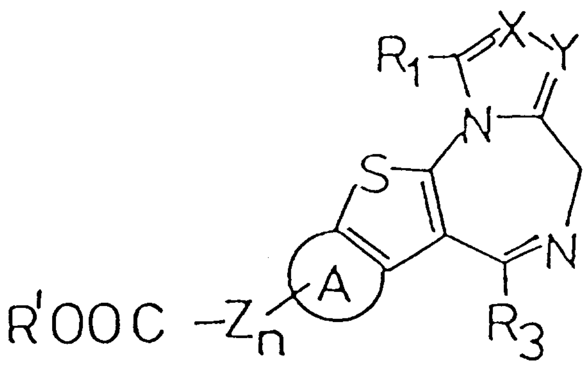

Ia1

dans laquelle $R_1$ = hydrogène, un groupe alkyle, cycloalkyle, A, Z, n, X, Y et $R_3$ sont tels que définis précédemment et R' représente un groupe alkyle en $C_1$-$C_4$,
de la manière suivante:

1) on obtient les composés de formule générale Ia où

$R_2$ = COOH

par saponification de composés de formule générale Ia1;

2) on obtient les composés de formule générale Ia avec

$R_2$ = $R_5R_6$NCO-

par réaction de Ia où

$R_2$ = COOH

avec une amine de formule

$HNR_5R_6$

éventuellement en présence de sulfonyldiimidazole, de carbonyldiimidazole ou de dicyclohexylcarbodiimide;

3) on obtient les composés de formule générale Ia où

$R_2$ = OH

par réduction sélective de Ia', par exemple avec l'alanate de lithium ou le borohydrure de sodium, ou pour n = 0 par réduction du composé carbonylé correspondant;

4) on obtient les composés de formule générale Ia où

$R_2$ = $R_{10}$NH-COO-, $R_{10}$-CO-$NR_{11}$-

par réaction de l'alcool ou de l'amine, préparé par exemple selon 3), avec un isocyanate de formule générale

$R_{10}$-N=C=O;

5) on obtient les composés de formule générale Ia avec $R_2$ = alkyle ou phénylcarbonyloxy éventuellement substitué par réaction de l'alcool, préparé par exemple selon 3), avec un équivalent d'acide, par exemple un halogénure d'acide, un acide carboxylique de formule

R-COOH

dans laquelle R représente un reste phényle éventuellement substitué ou un reste alkyle ramifié ou non ramifié de 1 à 8 atomes de carbone, la chaîne carbonée pouvant être interrompue par l'azote, l'oxygène ou le soufre;
6) on obtient les composés de formule générale Ia avec $R_2$ = alkylsulfonyloxy ou phénylsulfonyloxy par réaction de l'alcool, préparé par exemple selon 3), avec un halogénure d'alkylsulfonyle ou de phénylsulfonyle avec addition d'un agent fixant les acides;
7) on obtient les composés de formule générale Ia avec

$R_2 = NR_7R_8$

ou un reste imido
par réaction du mésylate ($R_2 = CH_3SO_3$-), préparé par exemple selon 6), avec une amine de formule

$HNR_7R_8$

ou avec un imide, éventuellement sous forme de leurs sels de métal alcalin;
8) on obtient les composés de formule générale Ia où

$R_2 = NH_2$

par exemple par clivage du phtalimide ($R_2$ = imide d'acide phtalique) ou par dégradation de Curtius de l'azide d'acide correspondant, ou par hydrolyse des isocyanates correspondants, ou par réduction de l'azide correspondant;
9) on obtient les composés de formule Ia où

$R_2 = NR_7R_8$

où $R_7$ ou $R_8$ représente un groupe alkylcarbonyle ou phénylcarbonyle, par réaction de l'amine, préparée par exemple selon 8), avec un équivalent d'acide carboxylique dérivé d'un acide carboxylique de formule

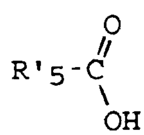

10) on obtient les composés de formule générale Ia avec

$R_2$ = formyle

par oxydation de l'alcool, préparé par exemple selon 3), avec raccourcissement de la chaîne de n à n-1;
11) on obtient les composés de formule générale Ia dans laquelle
$R_2$ représente un reste de formule générale

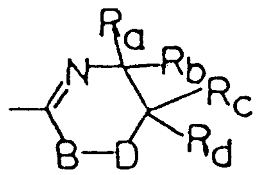

par réaction d'un composé de formule générale Ia où

$R_2$ = COOH

avec une amine bisfonctionnalisée de formule générale

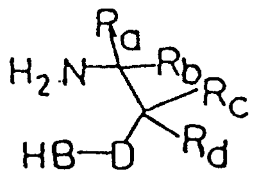

dans laquelle $R_a$, $R_b$, $R_c$, $R_d$, D et B ont la signification donnée précédemment,
en présence de triphénylphosphine, de $CCl_4$ et d'une base;
11a) on obtient les composés de formule générale Ia dans laquelle $R_2$ représente un reste thiazoline éventuellement substitué à partir des oxazolines correspondantes, préparées par exemple selon 11), par sulfuration, par exemple avec le pentasulfure de phosphore ou le réactif de Lawesson®;
12) on obtient les composés de formule générale Ia dans laquelle
$R_2$ représente une amidine de formule générale

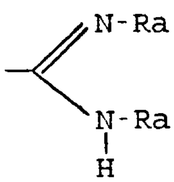

de manière analogue à 11) par réaction avec une amine primaire de formule $H_2NR_a$;
13) on obtient les composés de formule générale Ia où

$R_2$ = CN

par réaction de composés de formule générale Ia dans laquelle

$R_2$ = $CONH_2$

avec l'oxychlorure de phosphore;
14) on obtient les composés de formule générale Ia dans laquelle $R_2$ représente une 2-imidazoline éventuellement substituée par des groupes alkyle ramifiés ou non ramifiés par réaction de composés de formule générale Ia où

$R_2$ = CN

a) avec HCl éthanolique,
b) réaction de l'imidoéthylester formé avec une éthylènediamine éventuellement substituée par des groupes alkyle ramifiés ou non ramifiés,
c) puis éventuellement alkylation de la fonction N-H libre avec des agents alkylants connus;

15) on obtient les composés de formule générale Ia dans laquelle $R_2$ = alkyl- ou phényloxycarbonyle par réaction d'un équivalent d'acide de I ($R_2$ = COOH) avec les alcools correspondants;
16) on obtient les composés de formule générale Ia avec $R_2$ = halogène par réaction du tosylate

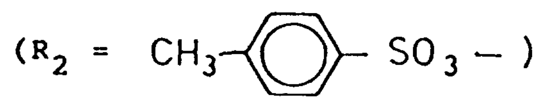

avec un agent halogénant;
17) on obtient les composés de formule générale I dans laquelle $R_2$ représente un éther ou un thioéther à partir des mésylates correspondants, préparés par exemple selon 6) ($R_2$ = $CH_3SO_3$) par réaction avec les alcools ou mercaptans correspondants comme solvant ou leurs sels de métaux alcalins dans un solvant organique inerte;
18) on obtient les composés de formule générale Ia où A représente

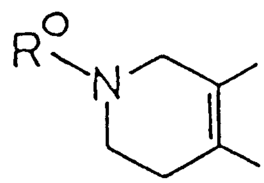

et $R^o$ représente un groupe alkyle, alkyle substitué, alkylcarbonyle, alkylcarbonyle substitué ou arylcarbonyle par alkylation ou acylation de composés de formule générale I avec $R^o$ = hydrogène, les composés thiocarbonylés correspondants par réaction du composé carbonylé avec un réactif soufré, par exemple le pentasulfure de phosphore;
puis, si on le souhaite, on fait réagir les composés Ia obtenus où $R_1$ = hydrogène en présence d'une base avec le chlore ou le brome pour former un composé de formule générale Ia où $R_1$ = chlore ou brome;
puis, si on le souhaite, on convertit le composé halogéné en un composé de formule générale Ia où $R_1$ = alcoxy de 1 à 4 atomes de carbone par réaction avec l'alcoolate correspondant;
puis, si on le souhaite, on réduit sélectivement un composé de formule générale Ia et, si on le souhaite, on alkyle ou acyle le composé Ib obtenu dans lequel $R_4$ représente l'hydrogène et on le convertit éventuellement en ses sels d'addition d'acide acceptables du point de vue pharmacologique puis on le résout éventuellement en ses composés optiquement actifs.

**9.** Composés de formule générale

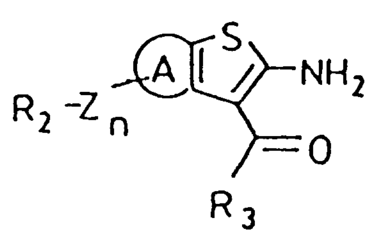

dans laquelle
A peut représenter un cycle carbocyclique condensé monoinsaturé à 5, 6 ou 7 sommets,
Z peut représenter un groupe alkylène ou alcénylène ramifié ou non ramifié;
n peut représenter 0, 1, 2, 3, 4, 5 ou 6; dans le cas d'un groupe alcénylène 0, 2, 3, 4, 5 ou 6;
$R_2$ peut représenter COOR* avec R* = hydrogène ou alkyle en $C_1$-$C_4$;
ou bien
$R_2$ peut représenter $R_5R_6$N-CO-
$R_2$ peut représenter $-NR_7R_8$

ou bien

$R_2$ peut représenter l'hydrogène (pour n supérieur à 0), un groupe hydroxyle, alkylcarbonyloxy, un reste éther ou thioéther, un groupe alkyle ou phénylsulfonyloxy tels qu'ils sont définis dans l'une des revendications précédentes,

et

$R_3$ peut représenter un groupe phényle, éventuellement substitué une ou plusieurs fois par halogène, méthyle, nitro et/ou trifluorométhyle.

**10.** Composés de formule générale

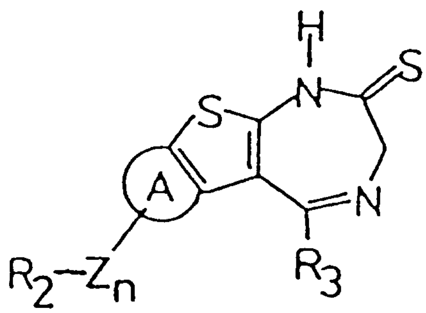

dans laquelle A, Z, n et $R_3$ sont tels que définis dans l'une des revendications précédentes et

$R_2$ peut représenter l'hydrogène (pour n supérieur à 0), hydroxyle,

$R_2$ peut représenter COOR* avec R* = hydrogène ou alkyle en $C_1$-$C_4$;

ou bien

$R_2$ peut représenter $R_5R_6N$-CO- où $R_5$ et $R_6$ sont définis comme précédemment;

$R_2$ peut représenter $-NR_7R_8$ où $R_7$ et $R_8$ sont définis comme précédemment;

ou bien

$R_2$ peut représenter un groupe alkylcarbonyloxy, un reste éther ou thioéther, un reste amine, alkyl- ou phénylsulfonyloxy tels qu'ils sont définis dans l'une des revendications précédentes.

**11.** Préparations pharmaceutiques contenant comme principes actifs des composés de formule générale Ia ou Ib selon la revendication 1 en combinaison avec des adjuvants et/ou véhicules habituels.

**12.** Procédé d'obtention de préparations pharmaceutiques selon la revendication 11, caractérisé en ce que l'on met des composés de formule générale Ia ou Ib sous forme de formes d'utilisation pharmaceutiques habituelles avec des adjuvants et/ou véhicules galéniques habituels.

**13.** Composés de formule générale Ia ou Ib selon la revendication 1 ou 2 destinés à être utilisés pour la préparation de médicaments à action antagoniste du PAF.

**14.** Utilisation de composés de formule générale Ia tels que définis dans la revendication 1, 2, 3, 4, 5 ou 6 pour la préparation d'un médicament pour le traitement de l'asthme bronchique.